# EUROPEAN PATENT SPECIFICATION

(11) **EP 4 196 572 B1**
(45) Date of publication and mention of the grant of the patent: **29.07.2026**
(21) Application number: 21766408.5
(22) Date of filing: 13.08.2021
(51) Int. Cl.: A61K 40/11, A61K 40/15, A61K 40/22, A61K 40/36, A61K 40/41, A61K 40/46, A61P 31/12, A61P 35/00, C12N 5/0783

(54) **IMMUNOSUPPRESSANT-RESISTANT T-CELLS FOR ADOPTIVE IMMUNOTHERAPY**
GEGEN IMMUNSUPPRESSIVUM RESISTENTE T-ZELLEN FÜR ADOPTIVE IMMUNTHERAPIE
LYMPHOCYTES T RÉSISTANTS AUX IMMUNOSUPPRESSEURS POUR L'IMMUNOTHÉRAPIE ADOPTIVE

(30) Priority: 14.08.2020 EP 20191216; 21.08.2020 EP 20192262
(43) Date of publication of application: 21.06.2023
(73) Proprietor: Charité - Universitätsmedizin Berlin, 10117 Berlin (DE)
(72) Inventor: AMINI, Leila, 13353 Berlin (DE); SCHMÜCK-HENNERESSE, Michael, 10247 Berlin (DE); REINKE, Petra, 10117 Berlin (DE); VOLK, Hans-Dieter, 10117 Berlin (DE); WAGNER, Dimitrios Laurin, 09128 Chemnitz (DE)
(74) Representative: Moré, Solveig Helga
(86) International application number: PCT/EP2021/072651
(87) International publication number: WO 2022/034233

(56) References cited:
- WO-A1-2014/191128
- HANS-DIETER VOLK: "Immunological Challenges of Regenerative Therapies - What, Why, How ?", 1 January 2020 (2020-01-01), XP055765365, Retrieved from the Internet <URL:https://www.bihealth.org/fileadmin/bih_lectures/immunology_and_advanced_therapies_bih_31-01-20-2_volk.pdf> [retrieved on 20210115]
- ANONYMOUS: "ReSHAPE Kick-off Meeting - Next-generation Treg development platform", 7 February 2019 (2019-02-07), XP055765373, Retrieved from the Internet <URL:http://www.reshape-h2020.eu/documents/meetings/kom2019/AGENDA_RESHAPE_KO_Meeting_2019.pdf> [retrieved on 20210115]
- PETRA REINKE: "Meeting of the Executive Project Management Team (EPMT)30 Introduction RESHAPE -Reshaping undesired Inflammation in challenged Tissue Homeostasis by Next-Generation regulatory T cell (Treg) Approaches -from Advanced Technology Developments to First-in-Human Trials First Annual Meeting 23 rd -24 th J", 23 January 2020 (2020-01-23), pages 0 - 12, XP055765370, Retrieved from the Internet <URL:http://www.reshape-h2020.eu/documents/meetings/fam2020/RESHAPE_01_2020_AGENDA_P.pdf> [retrieved on 20210115]
- B. DE ANGELIS ET AL: "Generation of Epstein-Barr virus-specific cytotoxic T lymphocytes resistant to the immunosuppressive drug tacrolimus (FK506)", BLOOD, vol. 114, no. 23, 26 November 2009 (2009-11-26), US, pages 4784 - 4791, XP055570458, ISSN: 0006-4971, DOI: 10.1182/blood-2009-07-230482
- KOICHI ARAKI ET AL: "Pathogenic virus-specific T cells cause disease during treatment with the calcineurin inhibitor FK506: implications for transplantation", THE JOURNAL OF EXPERIMENTAL MEDICINE, vol. 207, no. 11, 25 October 2010 (2010-10-25), US, pages 2355 - 2367, XP055765761, ISSN: 0022-1007, DOI: 10.1084/jem.20100124
- LEILA AMINI ET AL: "CRISPR-Cas9-Edited Tacrolimus-Resistant Antiviral T Cells for Advanced Adoptive Immunotherapy in Transplant Recipients", MOLECULAR THERAPY, 1 September 2020 (2020-09-01), XP055738895, ISSN: 1525-0016, DOI: 10.1016/j.ymthe.2020.09.011

## Description

The present invention relates to the field of cell therapy, in particular, to adoptive T cell or NK cell therapy. It provides a concept of targeted reshaping a pathogenic immune imbalance by a combination of immunosuppression, in particular calcineurin inhibitor alone or in combination with corticosteroids, with a highly specialized human cell product with a specific immunophilin knockout alone or in combination with steroid receptor knockout. The invention provides a guide RNA targeting a specific sequence in FK506-binding protein 12 (FKBP12). It also provides a T cell or NK cell, wherein the immunophilin FKBP12 is knocked out by targeting it for CRISPR/Cas mediated gene editing with a guide RNA targeting a specific sequence, or a cell subset or population of such cells. Additionally, also via CRISPR/Cas mediated gene editing with a specific guide RNA, the immunophilin cyclophilin A may be knocked out. The gene encoding cyclophilin A is commonly designated PPIA, peptidylprolyl isomerase A. The cell is resistant to the action of the immunosuppressant Tacrolimus and, optionally cyclosporine A. This immunophilin knockout can be combined with knockout of the nuclear receptor subfamily 3 group C member 1 (NR3C1; the gene encoding for the glucocorticoid receptor). The cell is then resistant to the immunosuppressants Tacrolimus as well as, optionally, the immunosuppressive corticosteroids (cortisol, prednisolone, methylprednisolon, dexamethasone) and/or cyclosporine A. It is obtainable by gene editing, in particular, Clustered Regularly Interspaced Short Palindromic Repeats (CRISPR)-Cas-based knockout of the immunophilin, e.g., by CRISPR-Cas9, with a guide RNA targeting a specific sequence. The invention thus provides a vector-free CRISPR-Cas-ribonucleoprotein-based good manufacturing practice (GMP)-compliant protocol, which efficiently targets and prevents the production of functional adaptor protein FKBP12 required for the immunosuppressive function of Tacrolimus, and, optionally, of cyclophilin A required for the immunosuppressive function of cyclosporine A and, optionally, of the glucocorticoid receptor required for the lymphotoxic function of glucocorticoids. The cell can be, e.g., a virus-specific cell to restore protection to viral infections, e.g., specific for an antigen from any of CMV, EBV, BKV, HPV, ADV, influenza virus, or SARS-CoV-2. It could be also a tumor antigen-specific effector cell, targeting cancer cells. It may also be a regulatory T cell, a specialized cell type to prevent overwhelming immunity/inflammation. The invention further provides a method for preparing the human immunophilin knockout T cell or NK cell of the invention, pharmaceutical compositions or kits comprising said cells, pharmaceutical compositions or kits for use in in treatment or prevention of an infection with a virus or another pathogen or for use in treatment of cancer, or for use in balancing an unwanted immune response, e.g., in the context of a condition selected from the group comprising autoimmunity, allergy, a transplantation and bystander activation.

Viral diseases are often associated with unwanted immune responses, e.g. allogenic organ rejection in solid organ transplant (SOT) recipients or acute T cell immunopathology caused by bystander T-cell activation during acute viral infections. Viral infections, such as cytomegalovirus (CMV), Epstein-Barr-Virus and BK-Virus, remain a major problem for morbidity and mortality in chronically immunocompromised patients. An exemplary case is cytomegalovirus (CMV) disease, a life-threatening complication occurring due to the immunosuppressive medication required for prevention of organ rejection in solid organ transplant (SOT) recipients. CMV disease causes direct and indirect morbidities including rejection of the transplanted organ¹ or chronic allograft nephropathy in kidney transplantation (KTx)². Classical antiviral medication is used as prophylaxis, pre-emptive or rescue therapy, though careful dosing is crucial to prevent toxicities and resistance³. Nevertheless, high frequencies of late-onset CMV disease have been observed with increased mortality among CMV-infected KTx recipients compared to uninfected recipients⁴. The T-cell-mediated anti-CMV response has proven to be a suitable predictive and stratification marker for CMV disease outcome^{5,6}. Thus, regeneration of the endogenous T-cell response by adoptive anti-viral T-cell therapy may diminish CMV-associated morbidities and mortality after transplantation.

Antiviral T-cell therapy is an established method to reconstitute effective immunity in hematopoietic stem cell transplant (HSCT) recipients ⁷⁻¹². The inventors have previously confirmed that antiviral T-cell products (TCPs) can be successfully generated from immunosuppressed solid organ transplant recipients^{13,14}. Treatment of CMV -disease using adoptive T-cell transfer to solid organ transplant recipients has been reported in a few studies¹⁵⁻¹⁷. However, despite the initial efficient reduction of symptoms and viral load^{18,19}, limited persistence and longevity of the TCP represent major challenges for successful adoptive cell therapy for CMV-disease. Similarly, relapses in Epstein-Barr-Virus load have been documented in TCP-treated SOT patients²⁰.

One reason for limited long-term efficacy of antiviral T-cell therapy may be the ongoing immunosuppression required to prevent organ rejection in solid organ transplant recipients. Conventional immunosuppression for KTx (and other solid organ transplant patients) may comprise at least one of three classes of immunosuppressants: Calcineurin inhibitors (CNIs, *e.g.* Tacrolimus [FK506] and Cyclosporine A [CsA]) potently reduce T-cell activation, maturation and cytokine secretion²¹. Glucocorticoids blunt cytokine production by T-cells²² as well as provoking lymphocyte apoptosis²³. The proliferation inhibitor Mycophenolic acid (MPA), which also inhibits proinflammatory cytokine production²⁴, induces lymphocyte apoptosis and decreases homing of T-cells²⁵. Preformed allo-antigen-reactive effector T-cells are particularly difficult to control in transplant recipients.

Calcineurin inhibitors are currently the most effective treatment for the sustained suppression of these allo-antigen-reactive cells. Therefore, solutions are required to maintain control of allograft reactive T-cells whilst overcoming suppression of protective antiviral T-cell responses. To circumvent the effects of Tacrolimus on adoptively transferred virus-specific TCPs, Tacrolimus-insensitive forms of calcineurin^{26,27} have been introduced into cells using retroviruses. Another method to induce Tacrolimus insensitivity is the use of a retrovirally integrated siRNA expression cassette to knockdown the immunophilin FKBP12²⁸, the adaptor protein needed for the immunosuppressive function of Tacrolimus in T-cells²⁹ or Koichi Araki et al., J. Exp. Med. 207 (11), 2010: 2355-2367. However, retroviruses integrate into the genome randomly, therefore posing a possible safety risk due to the potential for gene disruption or dysregulation. Furthermore, the mutated proteins introduced are putatively immunogenic. Moreover, RNA interference is often incomplete, thus partial susceptibility to Tacrolimus may be retained. Neither of these approaches has yet reached clinical application.

WO 2014/191128 A1 describes CRISPR Cas9 edited T cells for adoptive immunotherapy and contemplates receptors for immunosuppressive agents as targets of inactivation. A presentation by Hans-Dieter Volk: "Immunological Challenges of Regenerative Therapies - What, Why, How?", 1 January 2020, mentions the ReSHAPE Horizon EU project for engineering Tacrolimus-resistant FKBP12/-/- regulatory T cells for adoptive therapy using CRISPR/Cas9.

Severe or chronic viral diseases also cause T-cell immunopathology due to bystander activation, e.g., of highly differentiated memory T-cells independent of their T-cell receptor specificity, for example via IL-15, especially in the elderly population possessing highly differentiated T-cells.

In light of this, the inventors have addressed the problem of providing an improved T cell or NK cell that may be used in a cell product for adoptive transfer to a patient, e.g., in a patient wherein an undesired immune response is inhibited by a calcineurin inhibitor. The aim is to allow specific T cells to react to their targets adequately, at the same time as the patient receives immunosuppressant chemotherapy to limit a pathological immune response.

The present invention provides a guide RNA targeting a sequence of SEQ ID NO: 1 for CRISPR/Cas mediated gene editing, and a human immunophilin knockout cell that is a T cell or NK cell, wherein the immunophilin is FKBP12, obtainable by gene editing with said guide RNA, as described in the claims.

Cyclophilin A is also designated peptidylprolyl isomerase A or PPIA. The gene is commonly designated PPIA. The terms are used interchangeably herein.

In the context of the invention, a knockout cell does not comprise a functional allele of the knocked out FKBP12 gene. This renders the cell resistant to an immunosuppressant capable of interacting with the FKBP12. In particular, as the immunophilin is FKBP-12, the cell is resistant to Tacrolimus.

The invention thus provides a GMP-compliant, vector-free, gene-editing approach targeting the FKBP12 gene with a guide RNA targeting a sequence of SEQ ID NO: 1 for CRISPR/Cas mediated gene editing to make CMV-specific Tacrolimus--resistant T-cells. Efficient knockout, or gene editing, is achieved by transfection of ribonucleoprotein complexes (RNP) comprising CRISPR-associated protein (Cas), e.g., Cas9 and respective single sgRNA (sgRNA)³⁰. Alternatively, other CRISPR-associated proteins, such as Cas12a, or related technologies such as base editor proteins or prime editing technology may be used instead of the Cas9 protein. The inventors implemented a RNP nucleofection step, e.g., in their existing clinical protocol for the generation of antiviral TCPs¹⁹. For safety, the protocol comprises an initial sorting step, e.g., to solely expand T-cells of a certain specificity (e.g. virus-specific T-cells), activated upon exposure to the respective (viral) antigens²⁷. Functionally, FKBP12^{.o.} antiviral T-cells showed superior cytokine production and activation in the presence of the calcineurin inhibitor Tacrolimus compared to unedited antiviral T-cells³¹. For FKBP-12^{k.o.}, these properties were inhibited in the presence of an alternative calcineurin inhibitor, CsA³¹, thereby demonstrating the specificity of pathway inhibition and providing an optional safety switch to control potential undesired effects of gene-edited FKBP12^{k.o.} T-cells in patients. By combining multiple highly refined technologies, the inventors have developed a unique approach for the efficient and safe generation of clinical-grade Tacrolimus -resistant TCPs or NK cell products suitable for adoptive T-cell therapy or adoptive NK cell therapy.

The invention can be used in two scenarios to reshape unwanted immune imbalance:
i) patients suffering from strong unwanted immune response (e.g. alloimmunity in organ or hematopoetic stem cell transplantation, or uncontrolled hyperimmunity as in severe atopic eczema) who routinely receive calcineurin inhibitor tacrolimus which effectively suppresses the unwanted immune response but also the protective anti-viral T-cell response. Consequently, patients are at high risk of infectious complications and virus-related tumors, frequently associated with need for reducing immunosuppression resulting in relapse of the underlying disease. The use of specific adoptive antiviral T/NK cell therapy can improve the pathogen control, but its efficacy is limited due to sensitivity of those cells to the calcineurin inhibitor as well. The invention provides Tacrolimus resistant (optionally, in combination with steroid-resistant) anti-viral cells that can effectively protect even in presence of basal immunosuppression, thus overcoming the need for weaning of the calcineurin inhibitors Tacrolimus in those patients. Moreover, unwanted immune reactivity is limited by self-regulation *via* regulatory T cells. Unfortunately, they are also sensitive to calcineurin inhibitors, meaning that both bad and good players are suppressed, preventing endogenous reshaping of unwanted immune responses. The invention provides a tool, Tacrolimus -resistant (optionally, in combination with steroid-resistant) Treg, that can reshape unwanted immune response in presence of immunosuppression with Tacrolimus to allow longlasting weaning of the latter.
ii) The pathogenicity and mortality associated with viral infections in non-immunosuppressed patients, such as influenza, SARS-CoV2, hepatitis B/C etc., is often more related to unspecific immune overactivation (bystander activation) than to the pathogen itself. Although immunosuppression with calcineurin inhibitor Tacrolimus (alone or in combination with steroids) or the application of regulatory T cells would be useful, it is commonly not used because of the undesired inhibition of protective antiviral response resulting in an unfavourable risk/benefit analysis. The invention provides a concept to combine useful immunosuppression in particular by the calcineurin inhibitor Tacrolimus alone or in combination with steroids by adoptive transfer of Tacrolimus -resistant (optionally, in combination with steroid resistant) protective anti-viral cells and/or Tacrolimus -resistant (optionally, in combination with steroid resistance) regulatory T cells to reshape unwanted bystander reactions without affecting protective immunity.

In a preferred embodiment, the cell is a T cell. Adoptive T cell therapy is more commonly used than NK cell therapy, for example, adoptive T cell therapy may be used in treatment of cancer or an infection, e.g., a virus infection. The T cell may be a CD4+ T cell. However, in particular in the context of virus-specific T cells, the T cell may be a CD8+ T cell.

In the context of the invention, "a" is to be understood as "at least one", unless explicitly mentioned otherwise. Thus, the method also relates to human immunophilin knockout cells that are T cells or NK cells, wherein the immunophilin is FKBP12. These cells may also, in a preferred embodiment, comprise a mixture of CD4+ and CD8+ T cells. Preferably, both CD4+ and CD8+ T cells are specific for the same antigen, or the same antigens. The antigen or antigens may be derived from one or multiple pathogens, e.g., from one virus, or from multiple viruses or a tumorantigen.

The cell may be a T cell specific for a virus, another pathogen or a cancer antigen. For example, the T cell may be a virus-specific T cell, wherein the virus is selected from the group comprising CMV, EBV, BKV, HPV, ADV, influenza virus, parvovirus, rubella virus, hepatitis viruses (HBV, HCV, HAV, HDV, HEV), coxsackie virus, respiratory syncytial virus (RSV), coronaviruses, such as but not only: MERS, SARS-CoV1 and SARS-CoV-2. The T cells may also be multi-virus specific T cells, e.g., T cells activated and selected by addition of antigens from multiple viruses.

Alternatively, the cell is a T cell specific for a cancer antigen, e.g., a tumor-specific antigen, a differentiation antigen, a viral antigen, a cancer-testis antigen. Typical cancer antigens are CD19, CD38, MAGE-A1, CTAG1B, AFP, CEA, CA-125, ETA, tyrosinase, or NY-ESO-1.

In another embodiment of the invention, the cell is a regulatory T cell, preferably, a CD4+CD25+ regulatory T cell, optionally, a CD4+CD25+ CD127⁻ regulatory T cell. It may be a regulatory T cell product consisting of polyclonal repertoire or specific for a defined antigen, e.g., a certain allogenic MHC molecule, autoantigen, or a virus such as MERS, SARS-CoV1 and SARS-CoV-2.

Alternatively, the cell may also be an NKT cell, an NK cell or a γδ T cell. NK cells are particularly useful for cancer indications (solid, haematological), in viral infection, such as CMV or EBV, bacterial infections, in macrophage activation syndrome or similar. NKT cells may be used for cancer indications (solid, haematological), in viral infection, such as CMV or EBV, in macrophage activation syndrome or similar. γδ T cells may be used for cancer indications (solid, haematological), in viral infection, such as CMV or EBV, bacterial infections.

The cell of the invention, e.g., the T cell of the invention, may comprise at least one, e.g., two or three further genetic modifications.

For example, the cell may comprise a) a knockout of both immunophilin genes with specific guide RNAs as defined in claim 1 and, optionally, in claim 6, so that the genes encoding FKBP12 and cyclophilin A are knocked out. Preferably, the glucocorticoid receptor gene is functional. In this case, steroid therapy provides a safety switch in case there are any issues with the transgenic T cells of the invention, e.g., any pathogenicity caused by these cells.

Alternatively, the cell may comprise b) a knockout of the glucocorticoid receptor gene, and a knockout of the immunophilin gene encoding FKBP12, whereas the second immunophilin gene encoding Cyclophilin A is functional. An immunosuppressant capable of interacting with the second immunophilin can thus be used as a safety switch in case there are any issues with the transgenic T cells of the invention, e.g., any pathogenicity caused by these cells. If there is more than one genetic modification in the cell relating to resistance to immunosuppressants, this embodiment is preferred, as the safety switch is robust and can easily be used in the clinic.

In a further embodiment, the cell may comprise c) a knockout of both immunophilin genes encoding FKBP12 as defined in the claims and Cyclophilin A, so that the genes encoding FKBP12 and Cyclophilin A are knocked out, and of the glucocorticoid receptor gene.

The invention also provides a cell population comprising cells of the invention, preferably, exclusively cells of the invention. Preferably, unless the T cell comprises a transgenic TCR or a chimeric antigen receptor, the cell population is a polyclonal population of cells. It is however preferably a T cell population comprising, to a large extent e.g., at least 70%, at least 80%, at least 90%, at least 95% or at least 99% or 100% T cells that are homogenous with regard to specific characteristics, e.g., regulatory T cells, T cells specific to one or more antigens from a defined pathogen, e.g., virus, T cells specific to a defined common antigen (but not necessarily to a common epitope), NKT cells or γδ T cells.

The cells of the invention are obtainable by gene editing, comprising CRISPR/Cas-mediated gene editing with a RNA targeting a sequence of SEQ ID NO: 1. This employs regular double-strand cutting nuclease enzymes such as, but not only, Cas9 (derived from *Streptococcus pyogenes* or functional mutants thereof) or Cas12a (derived from *Acidaminococcus sp.* or functional mutants thereof). Other Cas enzymes, e.g., *Staphylococcus aureus* Cas9, *Streptococcus thermophiles* Cas9, or LbCas12a can also be used.

Thus, the cell population of the invention preferably comprises cells with InDel mutations in the genes (i.e. in both genes) encoding the immunophilin. For example, at least 10% of the cells of the population comprise said InDel mutations, optionally, at least 20%, preferably, at least 50%, e.g., at least 70%, at least 90% or 100%. InDel mutations are characteristic mutations or deletions introduced by Cas, e.g., Cas9, around the cut site, which is dependent on the sgRNA⁴³. The InDel mutations are obtainable by CRISPR/Cas (e.g., Cas9)-mediated gene editing with a sgRNA targeting SEQ ID NO: 1. Preferably, in case of an additional Cyclophilin^{k.o.}, the InDel mutations are obtainable by CRISPR/Cas (e.g., Cas9)-mediated gene editing with a sgRNA targeting SEQ ID NO: 4, 5, 6 or 7, preferably, 5 or 6.

Cas enzymes can also be genetically modified Cas enzymes. A functional mutant of a wildtype enzyme may, e.g., have at least 70%, at least 80% or at least 90% sequence identity to the wildtype enzyme and retains the ability to mediate gene editing, in particular, editing of the targets described herein. They can also be Cas derivative enzymes, including e.g. high fidelity enzymes (Vakulskas et al., 2018. Nat Medicine 24: 1216-1224), base editing enzymes (Komor et al. 2016. Nature 533:420-424; Rees and Liu 2019. Nat Rev Genet. 19(12):770-788; Walton et al., 2020. Science 368(6488): 290-296) or prime editors (Anzalone et al., 2019. Nature 576:149-157).

CRISP/Cas-mediated gene editing in the context of the invention is understood to comprise derivative technologies such as base editing or prime editing. Accordingly, if the cells of the invention are generated using CRISPR/Cas derivative technologies such as base editor proteins or prime editing, the genetic intervention can also be used to insert stop codons in the reading frame, e.g., the early reading frame, or disrupt splice acceptor/donor sites, to prevent generation of functional immunophilin protein. The cells of the invention then comprise a stop codon in the reading frame of the knocked out gene, and/or a disrupted splice acceptor/donor site.

Preferably, the cell population of the invention is obtainable from Cas (e.g., Cas9)-mediated gene editing of the immunophilin genes of a respective cell population obtained from a human subject, with a sgRNA targeting SEQ ID NO: 1 (for knockout of FKBP12).

If cyclophilin A is further knocked out, the cell population of the invention is preferably obtainable from Cas (e.g., Cas9)-mediated gene editing of the immunophilin genes of a respective cell population obtained from a human subject, e.g., with a sgRNA targeting any of SEQ ID NO: 4, 5, 6 and/or 7, preferably, a combination thereof.

The InDel mutations can be analysed, e.g., as described in the examples below under "efficiency analysis" for the respective sgRNAs.

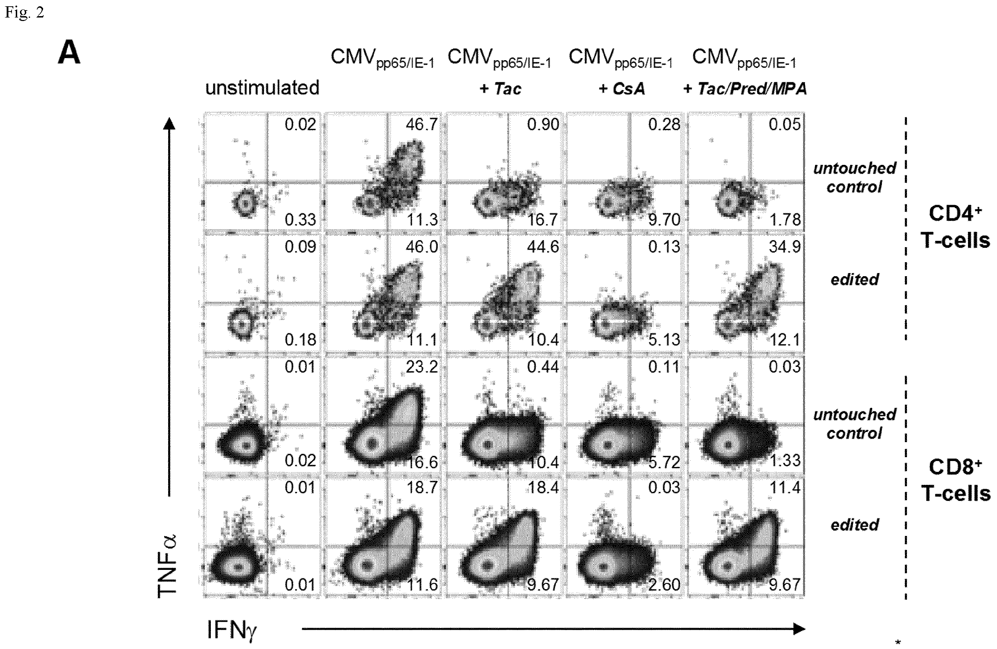

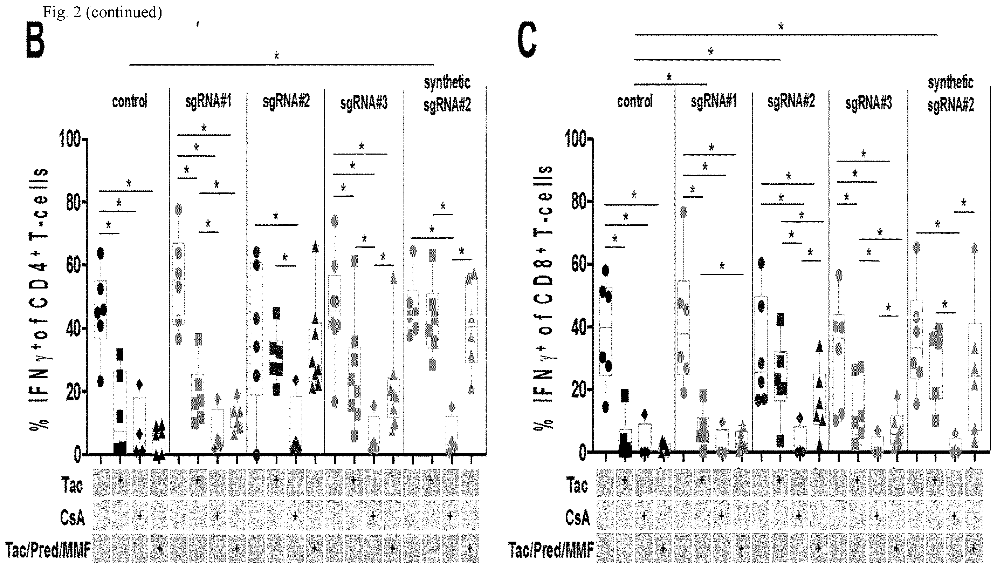

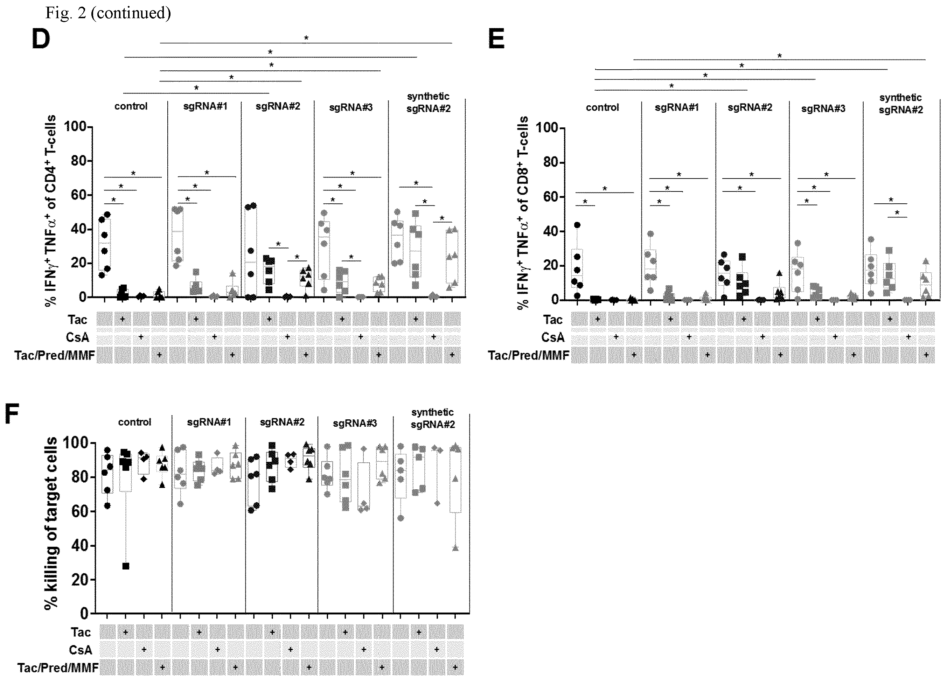

As a consequence of the knockout, the cells of the population, e.g., at least 10% of the cells of the population are resistant to the action of an immunosuppressant agent capable of interacting with the immunophilin, optionally, at least 20%, preferably, at least 50%, e.g., 70%, at least 90%, at least 99% or most preferably, 100%. In particular, resistant to the action of an immunosuppressant agent capable of interacting with the immunophilin means, that, in the presence of said immunosuppressant, the cells produce approximately the same amount of cytokines, e.g., IFNγ for antigen-specific T cells (or, for regulatory T cells, IL-10) under the conditions exemplified in the experiments underlying Fig. 2 b and c, using the relevant antigen).

The invention also provides a method for preparing a human FKBP12 knockout T cell or NK cell of the invention or a T cell or NK cell population of the invention, comprising
a) stimulating T cells or NK cells obtained from a subject,
b) isolating stimulated T cells or NK cells based on expression of a marker to obtain a composition of selected T cells or NK cells, and
c) gene editing the cells of said composition to knock out the gene encoding the immunophilin FKBP12, i.e. introducing a ribonucleoprotein complex comprising Cas and a guide RNA targeting a sequence of SEQ ID NO: 1, preferably, a sgRNA, targeting the gene encoding the immunophilin FKBP12 into the T cells of said composition.

The method may further comprise d) selecting FKBP12knockout cells by culturing the cells in the presence of an immunosuppressant agent capable of interacting with the immunophilin. Said step is however not required, as efficacy of knockout is commonly very high (>75%).

Preferably, the cell is a T cell. It is also preferred that the method is carried out with a plurality of T cells to obtain a cell population of the invention, e.g., a T cell population of the invention.

The cell can be obtained from a sample isolated from the subject. The subject preferably is a human subject. The cell, i.e., the T cell or NK cell, may be from the peripheral blood of the subject, e.g., from peripheral blood mononuclear cells (PBMC), e.g., isolated by density gradient or leukopheresis. Alternatively, the cells may be tumor-infiltrating lymphocytes, or they may be obtained from another tissue sample, or may be generated from iPSC.

The subject may be a patient, e.g., a patient requiring immunosuppression, for example because of a transplantation or another unwanted immune response, such as autoimmunity, allergy or unspecific immunopathology. The patient may already be immunosuppressed, or immunosuppression may be intended in the future, e.g., after the sample is taken. However, the subject may also be a healthy subject, from whom knockout cells of the invention may be produced for pharmaceutical use in a different patient, wherein said patient and said subject share at least one MHC allele, preferably, at least two, at least three, at least four or all MHC alleles. If the transferred cells are CD4+ cells, at least one MHC II allele should be shared, and/or if the transferred cells are CD8+ cells, at least one MHC I allele should be shared.

If the human immunophilin knockout cell of the invention is an antigen-specific T cell, in particular, a T cell specific for a virus, another pathogen or a cancer antigen, in step a), the T cells are stimulated with the antigen, e.g., a virus-derived antigen, another pathogen-derived antigen or a cancer antigen. The antigen can be part of an antigen mixture, e.g., a peptide mixture, a protein, a peptide- or protein pulsed antigen-presenting cell, or an antigen-presenting cell expressing said antigen. A single epitope may be used, but it is advantageous to use several, if not all epitopes of an antigen to stimulate a variety of antigen-specific T cells. For example, it may be preferable to use a combination of non-structural and structural antigens (or epitopes from antigens) e.g., from SARS-CoV-2. Of course, stimulation of T cells with an antigen is stimulation with an epitope of said antigen presented on a suitable HLA of an antigen presenting cell. The antigen-presenting cell may be, e.g., a B-cell, a macrophage or a dendritic cell, or a mixture thereof, a lymphoblastic cell line and/or a cell infected with the virus of another pathogen or a tumor cell. The antigen-presenting cell may be provided with the antigen as a whole, as a nucleic acid (e.g., an mRNA or an expression vector) or in peptide form. The antigen-presenting cell may also express the antigen. Further, in some embodiments stimulations with multiple antigens of the same or distinct pathogens, e.g., multiple viruses, can be performed to generate highly selected, but multi-specific T cell lines. Stimulation can be for a suitable period of time to allow for expression of an activation marker, e.g., for 3-48 hours, for 4-24 hours or for 6 hours to overnight stimulation, e.g., about 12 hours. About, if used in the context of the present invention, preferably means +/- 10%.

The cells, in particular, the T cells, may be primed by stimulation with an antigen from a virus, another pathogen or a cancer antigen. That priming may occur in the stimulation of step b), and/or the cells may already have been primed *in vivo,* e.g., cells may be obtained from a subject that has been infected with a virus or another pathogen, preferably, from a convalescent patient or vaccinated individual.

The stimulation leads to expression of an activation marker in T cells specific for the respective antigen. Consequently, in step b), antigen-specific T cell cells can be selected based on expression of the activation marker. The activation marker may be secretion of a cytokine, such as IFNy, TNFα, or IL-10, preferably, IFNy-secretion. Alternatively, the activation marker may be one surface molecule or a combination of surface molecules, e.g., CD154, CD137, OX40, and/or CD69, in particular, for CD4+ T cells, preferably, CD154. CD137 also is a preferred activation marker.

The expression of a marker may be assayed *via* techniques such as fluorescence microscopy, flow cytometry, ELISPOT, ELISA or multiplex analyses. Selection can be, *e.g., via* a commercially available cytokine, e.g. IFNy, secretion assay - magnetic cell enrichment and detection kit, or by flowcytometric means.

Surface molecule expression is typically assessed by adding detection antibodies to the cells, e.g., CD154, CD137, OX40, and/or CD69. In case CD154 is used, CD154 detection antibody may be added to culture at stimulation initiation or after stimulation. In the latter case, an antibody against CD40 may be added to facilitate CD154 detection.

If the human FKBP12 knockout cell of the invention is a regulatory T cell (Treg), in step b), the marker is a regulatory T cell marker such as CD25, usually a combination of regulatory T cell markers including CD25. Regulatory T cells may undergo a double selection for expression of CD25 and CD4. Said selection may be carried out simultaneously or in any order. Regulatory T cells may also be selected by the combination of CD4 and CD25 or CD137, but also the absence or low expression of CD127 or CD154. Furthermore, other common Treg identity markers can be used in combination with the ones mentioned before (such as high expression of CTLA-4, and others) in addition to those mentioned. CD8 Tregs can be isolated, e.g., through combination of positive selection for CD8 and negative selection for CD28 or CD45RB. If the Treg is antigen-specific it preferentially, but not exclusively, targets an allo-antigen or antigens causing autoimmunity or immunopathology.

If the human FKBP12 knockout cell of the invention is an NKT cell, in step b), the cell is isolated based on expression of an NKT cell marker, preferably, based on expression of both CD56 and CD3. Said selection may be carried out simultaneously or in any order.

If the human FKBP12 knockout cell of the invention is an NK cell, in step b), the cell is isolated based on expression of an NK cell marker, preferably, based on expression of CD56 and/or NKG2D, or CD16 and lack of expression of CD3. Said selection may be carried out simultaneously or in any order.

If the human immunophilin knockout cell of the invention is a γδ T cell, in step b), the cell is isolated based on expression of a γδ T cell marker, preferably, based on expression of γ and/or δ T cell receptor.

After selection, cells can be cultured in the presence of suitable cytokines, e.g., to expand them to the desired number. For example, they may be cultured in the presence of hIL-7 and hIL-15. Alternatively or additionally, they may be cultured in the presence of irradiated feeder cells. A GMP-compliant production is preferred. During expansion, cells may be split, e.g., 1:1 upon reaching confluency. Cells may be expanded, e.g., for 0-30 days, for 1-28 days, for 3-24 days, for 7-21 days or for about 7-14 days. No expansion is required if the cell numbers needed are already present in the sample comprising the isolated cells.

At a time point during culture and/or expansion, in step c), a ribonucleoprotein complex comprising Cas (e.g., Cas9) and the guide RNA targeting a sequence of SEQ ID NO: 1, e.g., a sgRNA, targeting the gene encoding the immunophilin FKBP12, is introduced into the cells of said selected cell composition. For example, it can be introduced after 0-30 days, for 1-28 days, for 3-24 days, for 7-21 days, or for about 7-14 days, preferably, about 7 days.

The guide RNAs, preferably, single guide (sg)RNAs targeting FKBP12 can be prepared according to methods known in the art. It is known in the art how such sgRNAs can be prepared, and sgRNAs are also commercially available. Exemplary sgRNAs may comprise SEQ ID NO: 14, 15 or 16. The inventors surprisingly found that excellent results are obtained when a sgRNA targeting SEQ ID NO: 1 is employed for knockout of the FKBP12 in the cells, particularly, the T cells of the invention. Thus, preferably, a single sgRNA is used for FKBP12 knockout that targets the sequence of SEQ ID NO: 1.

A guideRNA targeting SEQ ID NO: 1 may optionally comprise the spacer sequence SEQ ID NO: 14, or a sequence having at least 80% sequence identity thereto, e.g., at least 90% or at least 95% sequence identity thereto. As the skilled person knows, the guide RNA can be a two RNA system comprising of a crisprRNA that includes the target specific "spacer" sequence and a tracrRNA that allows binding to the Streptococcus pyogenes Cas9 enzyme. The guide RNA can also be chimeric single guide RNA that links the crisprRNA and the tracrRNA through different linkers. The sgRNA may represent a ribonucleic acid. It may also consist of a mix of both ribonucleosides as well as of desoxynucleosides (O'Reilly et al., 2019. Nucleic Acids Research 47(2):546-558). As the skilled person knows, the spacer sequence can be modified, e.g., by reducing its 5'-end to spacer length to 17 bases, or less (Fu et al., 2014. Nat Biotechnol 32(2):279-284), increasing the spacer length (Xu et al., 2015. Genome Res 25(8):1147-1157) and/or replacing one, or more, nucleosides within the target sequence while retaining high cutting efficacy (Dang et al., 2015 Genome Biology Art. 280). The guide RNA may be generated through *in vitro* transcription, or it may be synthesized chemically or enzymatically.

As the skilled person knows, the efficiency of guide RNA molecules may be enhanced by use of 2O'-methyl-3'phosphothioate modifications or others (Hendel et al. 2015. Nat Biotechnology 33(9):985-989). In the experiments described herein, a chemically synthesized sgRNA was employed. However, it is also possible to use a separate tracrRNA and crisprRNA. The sgRNA may e.g. have the sequence of SEQ ID NO: 17, or alternatively, at least 70% sequence identity thereto, optionally, at least 80%, at least 90% or at least 95% sequence identity.

The invention thus also relates to use of a sgRNA targeting SEQ ID NO: 1, for preparing a cell of the invention, in particular, for preparing a pharmaceutical T cell product of the invention.

The sgRNA preferably is a synthetic sgRNA. It may be modified to increase stability, e.g., the sgRNA may comprises at least one 2O'-methyl-3'phosphothioate modification, e.g. between the first and last three nucleotides, or 2O'-methyl-3'phosphothioate modifications between the first and the last three nucleotides. Other suitable modifications are disclosed e.g., by Hendel et al. 2015. Nat Biotechnology 33(9):985-989.

Additionally, the immunophilin may be cyclophilin A. Guide RNAs, preferably, sgRNAs targeting cyclophilin A can be prepared and selected according to methods known in the art. Optionally, the sgRNA targets the sequence of SEQ ID NO: 4, SEQ ID NO: 5, SEQ ID NO: 6 and/or SEQ ID NO: 7.

A sgRNA targeting SEQ ID NO: 4, 5, 6 or 7 may optionally comprise the spacer sequence SEQ ID NO: 20, 21, 22, 23 or 24, respectively, or a sequence having at least 80% sequence identity thereto, e.g., at least 90% or at least 95% sequence identity thereto. As described above, the spacer sequence can however be modified. In the experiments described herein, a sgRNA also taking over the function of tracrRNA was employed. However, it is also possible to use a separate tracrRNA and sgRNA. The sgRNA may e.g. have the sequence of SEQ ID NO: 24-27, or alternatively, at least 70% sequence identity thereto, optionally, at least 80%, at least 90% or at least 95% sequence identity.

Said sgRNA may also be a synthetic sgRNA, as described herein. Alternatively, it may be in vitro transcribed RNA.

The guide RNA may be a chimeric RNA, so that no additional tracrRNA is required. However, instead, a suitable tracrRNA may be employed in addition to a crRNA.

Step c) is, throughout the invention, preferably carried out by electroporation of the selected and, optionally, expanded, cells. For example, about 1-5 million T cells can be electroporated with e.g., 1-500 µg, e.g., 40-100 µg, preferably, about 30-40 µg of cas9 protein precomplexed with the sgRNA. The sgRNA may be used in an amount of about 1-50 µg, e.g., 10-40 µg or 15-30 µg, for example, about 15 µg sgRNA may be used. Chemical transfection is also possible.

The ribonucleoprotein complex leads to DNA double strand breaks at the target sequence, and consequently, introduction of mutations by the error prone cell inherent repair machinery, and loss of expression of the respective protein. As explained above, the pattern of mutation depends on the sgRNA used.

After step c), the cells may be further cultivated and, preferably, expanded. For example, said expansion may take place under the conditions explained herein. The cells may be expanded for 0-30 days, for 1-28 days, for 3-24 days, for 7-21 days, or for about 7-14 days, preferably, about 14 days. The length of the expansion depends on the number of cells required.

During said expansion, or during part of said expansion, it is possible to d) select FKBP12 knockout cells by culturing the cells in the presence of an immunosuppressant agent capable of interacting with the immunophilin FKBP12. Said step is however not required, as cells not comprising the knockout mutations may also be comprised in the generated cell product. If administered to a patient, said cells will however be inhibited by the calcineurin inhibitor, so they will be less efficient in performing their desired function than knockout cells.

The cells of the invention may be frozen before pharmaceutical application, preferably, in a suitable buffer, e.g., comprising a suitable amount of DMSO, such as 10%. After thawing and before administration, it may be formulated in a pharmaceutically acceptable composition, e.g., Ringer solution or phosphate buffered saline. Of course, freezing is not required but feasible. The pharmaceutical composition may, e.g., be in liquid form such as after thawing or from fresh production. It may also be in frozen form.

Typically, the cells of the invention are formulated in physiological NaCl solution for use in application via a peripheral venous access. They can be frozen and used after thawing without need for reformulation.

The invention also provides a cell or a cell population as described herein, wherein the cell is obtainable by carrying out the method of the invention.

The invention thus provides a cell product, preferably, a T cell product (TCP) which, advantageously, is suitable for medical applications, in particular, for treatment of a human subject or patient.

As another embodiment, the invention provides a pharmaceutical composition comprising the cell of the invention or the cell population of the invention, e.g., the TCP of the invention. The pharmaceutical composition typically further comprises at least one pharmaceutically acceptable carrier, diluent or excipient. Preferably, the pharmaceutical composition comprises an isotonic salt (NaCl) solution, e.g., Ringer solution, and/or an aqueous buffer in order to stabilize the cells. As used herein, the term pharmaceutically acceptable excipient includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

The amount of cells administered to a patient depends on several factors and may be judged by the skilled person, e.g., depending on the weight, age, and condition of the patient and the specificity of the cells. For example, 1*10⁵ cells or more, 5*10⁵ cells or more, or about 1*10⁶-5*10⁹ cells, e.g., 1*10⁷-1*10⁹ or 5*10⁷-5*10⁸ cells may be administered to a patient.

A kit comprising the pharmaceutical composition of the invention and an immunosuppressant agent capable of interacting with the immunophilin FKBP12 is also provided by the invention, i.e., Tacrolimus. Accordingly, a patient who has been administered the pharmaceutical composition may receive immunosuppressive treatment with the respective immunosuppressive agent to which the T cells of the invention are resistant, so that these T cells are not suppressed by the immunosuppressive agent. The kit may optionally further comprise an immunosuppressant capable of inhibiting an immune response of the cells, which, in case any undesired effects are caused by the T cells of the invention, may be administered to said patient to reduce or prevent said undesired effects.

Regardless of whether the cells of the invention are provided in a kit or separately, the treated subject may be immunosuppressed with an immunosuppressive agent capable of interacting with the immunophilin FKBP12. Said immunosuppressant may be administered simultaneously, or in any order. Typically, the subject is already immunosuppressed when the pharmaceutical composition of the invention is administered due to the time needed for preparation of the cells of the invention, if these are derived from the subject. Alternatively, in particular, if cells from a library are used, the cells may be administered first, followed by the immunosuppressive agent after engraftment, or the immunosuppressive agent may be administered first, or both may be administered essentially simultaneously, e.g., within a day. The patient may e.g. require a treatment with an antigen specific T cell product and an immunosuppressant agent, particularly a patient diagnosed with an infection (e.g., EBV, CMV, BKV, pneumonia or myocarditis), or wherein the patient has received an allogeneic transplant.

Virus-specific or tumor-specific T-cells can selectively target pathogen-infected or tumour cells. In fact, there are promising data on adoptive anti-viral or anti-cancer T-cell therapy in immunocompromised patients (e.g. against CMV, EBV, EBV-associated lymphoma). However, the cell therapy is not effective in all patients. The main reason is the reduced efficacy and survival after transfer by the negative effects of immunosuppression in those patients which cannot be reduced because of risk of alloreactivity. The pharmaceutical product of the present invention comprises cells resistant to immunosuppression mediated by the powerful immunosuppressive calcineurin inhibitor Tacrolimus by silencing key signalling molecules of the immunosuppressive drugs. In contrast to prior art approaches used so far, which have several limitations (low efficacy, complicated manufacturing process, targeting single molecules only), the present invention provides a vector-free targeted, highly efficient and robust CRISPR-Cas mediated gene editing approach with guide RNAs targeting SEQ ID NO: 1 allowing easy combinations (double/triple knock-outs for different immunosuppressive drugs - CNI + steroid). Resistance to immunosuppression can also be combined with other gene manipulations, e.g. redirecting specificities by a CAR or a tgTCR, or prevention of exhaustion by PD1^{ko}.

The provision of virus-specific immunosuppressant-resistant T cells, in particular, memory/effector T cells makes the combinatory use of those cells specific to the challenging virus (e.g. SARS-Cov2, Influenza) with the respective immunosuppressive drug possible, which further combats undesired bystander immune reactivity. In other words, this dissects protective specific anti-pathogen T cell response from immunopathologic unspecific pathogen-related immune response.

The pharmaceutical composition or kit of the invention, wherein the T cell is a T cell specific for a virus, another pathogen or a cancer antigen, or an NK cell, typically is for use in treatment or prevention of an infection with the virus or the other pathogen or for use in treatment of the cancer. For example, the virus infection can be an infection with EBV, CMV, BKV, influenza, MERS, SARS-CoV-1 or SARS-CoV-2. The T cell of the invention, and its pharmaceutical use for treatment of the relevant infection, e.g., virus infection, may be particularly relevant in cases wherein there are no other causative treatment options available. In one embodiment, the T cell thus is a T cell specific for an antigen from SARS-CoV2.

A T cell of the invention specific for a cancer antigen, e.g., a tumor-specific antigen, a differentiation antigen, a viral antigen, or a cancer-testis antigen, for example are MAGE-A1, CTAG1B, AFP, CEA, CA-125, ETA, tyrosinase, or NY-ESO-1 but also oncogenic viruses, such as EBV, HPV, HBV, may, e.g., be useful for adoptive T cell therapy of cancer, e.g., in a patient receiving immunosuppression to suppress unwanted immune reaction, e.g. transplantation, autoimmunity etc. It may also be required, e.g., if a cancer patient requires immunosuppression to prevent rejection of a transplantation or for controlling an autoimmunity or an overwhelming immune reaction as result of cancer immunotherapy. Also, e.g., in post-transplant lymphoproliferative disorder, a calcineurin inhibitor may be provided to the patient in combination with virus-specific, e.g. EBV-specific T cells. Immunosuppression may also be part of the cancer treatment, e.g., to suppress unwanted side effects of anti-cancer immunotherapy (for example caused by treatment with checkpoint inhibitors or CAR-T cells).

As mentioned, regulatory T cells may be able to reshape undesired immune responses. Extensive preclinical studies demonstrate the power of this approach to combat undesired immune reactions.

First studies in patients demonstrate some beneficial effects of adoptively transferred regulatory T cells in transplantation, but a complete weaning of immunosuppression has not been feasible yet. Moreover, regulatory T cells are less efficient in controlling already established pathogenic effector T cell responses compared to prevention of undesired *de novo* T cell responses. This typically makes the use of simultaneous immunosuppression necessary, not only in transplantation but also in the other diseases described above.

Again, the function and survival of regulatory T cells in the state of the art is strongly inhibited by immunosuppressive drugs like CNIs, lowering their efficacy.

T cells play a central role in the key task of the immune system - maintaining the integrity of the organism, such as in infections with pathogens, the removal of transformed cancer cells and the regeneration of tissue damage. This is a complex process that is finely regulated by a balance of effector cells (proinflammatory) and regulatory cells (so-called Treg) in the course of the immune response to avoid undesired immune reactions mediating pathogenic effects. However, a misdirected or exaggerated undesired T cell response can lead to acute and chronic immunopathologies, such as autoimmunity, hyperinflammation, or damaged tissue repair up to scarring and*functio lesea.* Undesired T cell reactions can also result from a physiological but unhelpful T cell response such as alloreactivity (rejection of organ transplants, GvH disease after HSCT) or immunoreactivity against therapeutics, such as protein-based biologics, or gene and cell therapy products.

Current therapy strategies to combat undesired immune reactions are based on broad immunosuppressive drugs, such as calcineurin inhibitors, kinase inhibitors, corticosteroids, antiproliferative drugs, or more targeted inhibitors of single effector molecules, such as anti-TNF, anti-IL-1, or anti-IL-17 mAbs. The options are typically selected based on the kind and strength of undesired reactions, the long-term outcome of the disease, and the safety and efficacy profile of a particular therapy in a patient.

The most powerful immunosuppression using calcineurin inhibitors (the most powerful inhibitors of T cell function and proliferation), steroids (having very broad anti-inflammatory profile and inducing lymphocyte death) and/or antiproliferative drugs (inhibition of clonal expansion) is used in transplantation (both solid organs and HSCT) as well as in very severe autoimmune diseases or hyperinflammatory diseases (e.g. atopic eczema).

These strategies have several limitations:
- Unspecific immunosuppression targets pathogenic T cells and their downstream products, but also the protective T cell response resulting in enhanced risk of infections and tumors.
- Despite availability of a broad portfolio of immunosuppressive drugs with good efficacy in many patients, there is a significant number of non-responders to standard-of-care.
- Most immunosuppressive drugs used target not only the effector T cells but also the useful regulatory T cells, resulting in a total suppression of all T cells, both "bad and good guys", which prevents reshaping to an immune balance with sustainable therapeutic effects. This makes immunosuppression in most immunopathologic disease to a life-long maintenance therapy with adverse effects and high direct and indirect costs.

Due to these limitations, immunosuppressive approaches are standard in transplantation medicine and for severe autoimmunopathies and hyperinflammatory diseases, but are rarely used in immunopathologies caused by "bystander" T cell activation. Such immunopathologies are associated with many severe viral infections (e.g. after infections by SARS-CoV2, influenza, hepatitis viruses etc.). If these immunopathologies occur, the useful effect of immunosuppressive drugs (inhibition of undesired bystander T cell activation) so far had to be weighed against the potential danger of inhibiting the protective anti-pathogen/cancer response.

This problem is addressed by the invention, meeting a high medical need for new therapeutic approaches to combat undesired T cell reactivity with increased efficacy, sustainability, and specificity. Consequently, the present invention provides a cell of the invention that may be a regulatory T cell, gene edited using CRISPR/Cas and a guide RNA targeting a sequence of SEQ ID NO: 1, for use in balancing an unwanted (undesired) immune response in a patient.

The T cell products of the invention comprising regulatory T cells may sustainably reshape a disturbed immune balance, e.g., in the cases mentioned above.

Thus, in this embodiment, the pharmaceutical composition of the invention comprising regulatory T cells is useful for balancing an undesired immune response, e.g., an immune response to a self-antigen, or an allogeneic antigen. The antigen may also be a xenogeneic antigen. The T cell may thus be useful for treatment of an autoimmune response, if the antigen is a self-antigen, or for reducing or preventing an immune response to a transplant, if the antigen is an allogeneic or xenogeneic antigen. They may also be useful for treatment of an allergy, if the antigen is a nonself-antigen, e.g., a harmless antigen, but also in patients receiving immunosuppression to prevent immunogenicity of gene therapy (e.g. following adeno-associated vectors, CRISPR/Cas *in vivo* gene editing, CAR cell constructs). Such therapy may be combined, at least at the beginning, with an immunosuppressant therapy including the calcineurin inhibitor Tacrolimus. During the course of the therapy, e.g., after stable establishment of the regulatory T cells in the patient, the dose of the calcineurin inhibitor may be reduced, or it may be altogether discontinued. If symptoms of the immunopathology re-appear, the immunosuppressant treatment may be re-introduced.

A cell of the invention that is a regulatory T cell specific for a defined antigen, e.g., from a virus such as hepatitis B/C, MERS, SARS-CoV1 and SARS-CoV-2, it may advantageously be used in the context of a pathologic immune response to said virus, e.g., causing a cytokine storm or symptoms of septic shock. However, antigen specificity is not required, because, as explained above, bystander activation is a well-known feature of such immunopathologies, optionally, in the context of a septic shock.

In another embodiment, a cell of the invention that is a regulatory T cell specific for an alloantigen, e.g., a particular MHC molecule expressed by a transplant such as an organ or stem cell transplant or allogeneic iPSC derived product a subject has received or is to receive, may advantageously be used in the context of application or transplantation.

The immunosuppressant agent capable of interacting with the immunophilin FKBP12, i.e., Tacrolimus may be for use in suppressing an undesired immune response in a patient, wherein the patient is administered a pharmaceutical composition of the invention. The terms immunosuppressant and immunosuppressive are used interchangeably herein. As explained above, administration can be essentially simultaneously (e.g., within a day) or in any order.

Said undesired immune response may be autoimmunity, auto inflammation, allergy, an immune response to a transplant, graft versus host disease or an immunopathology caused by bystander activation, optionally, in the context of a septic shock. The undesired immune response may, e.g., be an immunopathology caused by bystander activation, which may be caused by an infection, optionally, an infection that causes pneumonia or myocarditis.

The immunopathology caused by bystander activation may be due to a viral infection with a virus e.g., CMV, EBV, BKV, HPV, ADV, influenza virus, parvovirus, rubella virus, hepatitis viruses (HBV, HCV, HAV, HDV, HEV), coxsackie virus, respiratory syncytial virus (RSV), coronaviruses, such as but not only: MERS, SARS-CoV1 and SARS-CoV-2, preferably, SARS-CoV-2. With the pharmaceutical composition of the present invention comprising regulatory T cells, it is now possible to treat the patient in a causal and sustainable way, whereas, previously, said patient could not have been treated with the immunosuppressant to avoid lack of the desired immune response to the pathogen, e.g., the virus, or to a cancer.

As used herein, the term "treating" or "treatment" of any disease or disorder refers, in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter.

"Preventing" a disease or disorder refers to reducing the incidence or the likelihood of the subject getting the disease.

Administration of the pharmaceutical composition of the invention may be e.g., parenteral, intravenous, intrapulmonary or intramuscular.

The invention is further illustrated by the following examples and figures. These examples are meant to illustrate the invention but not to limit its scope.

### Brief Description of the Figures

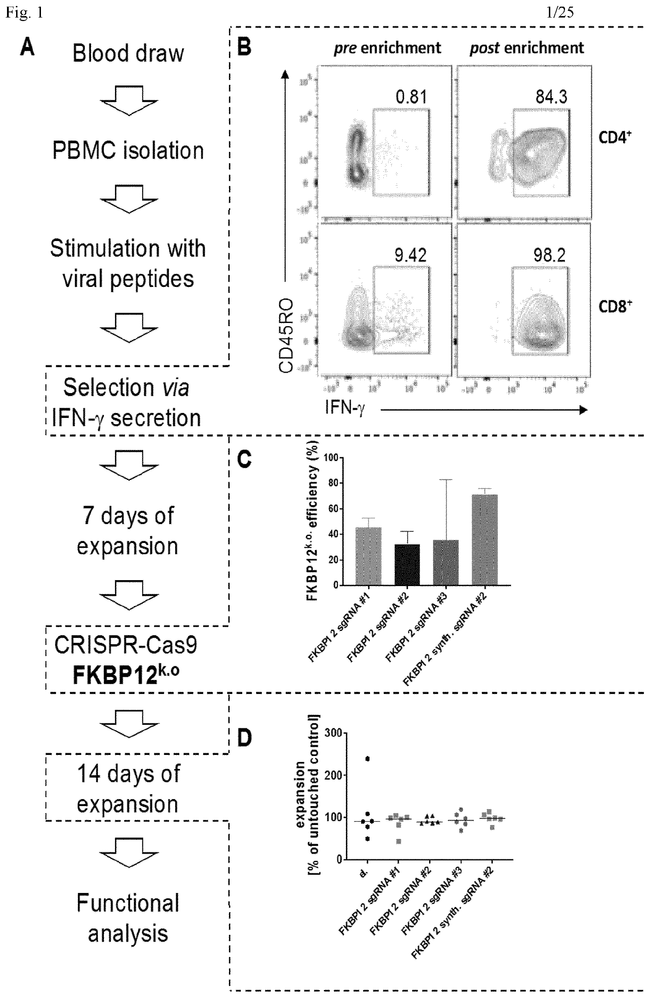

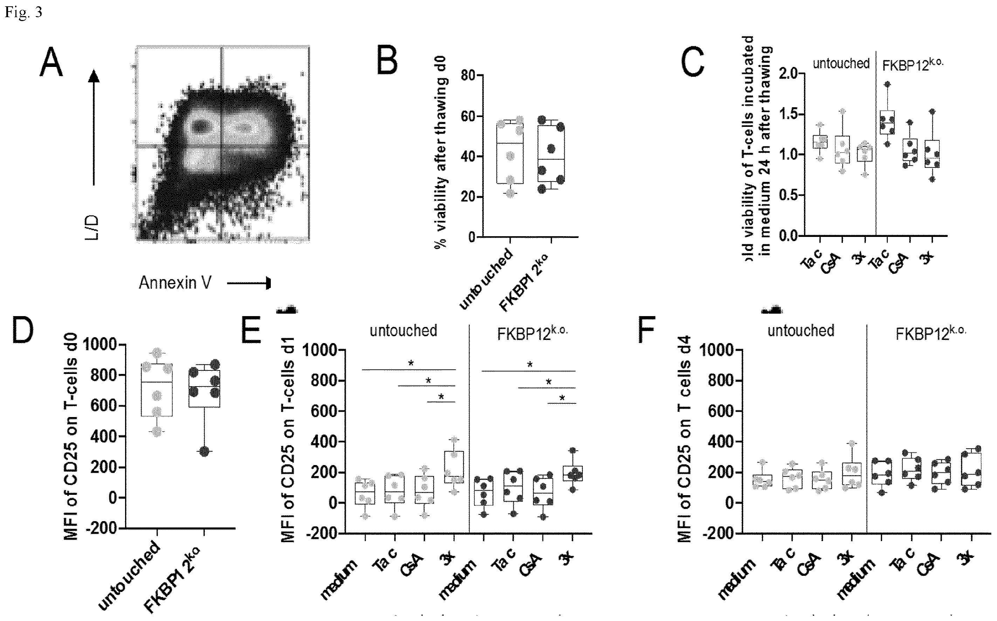

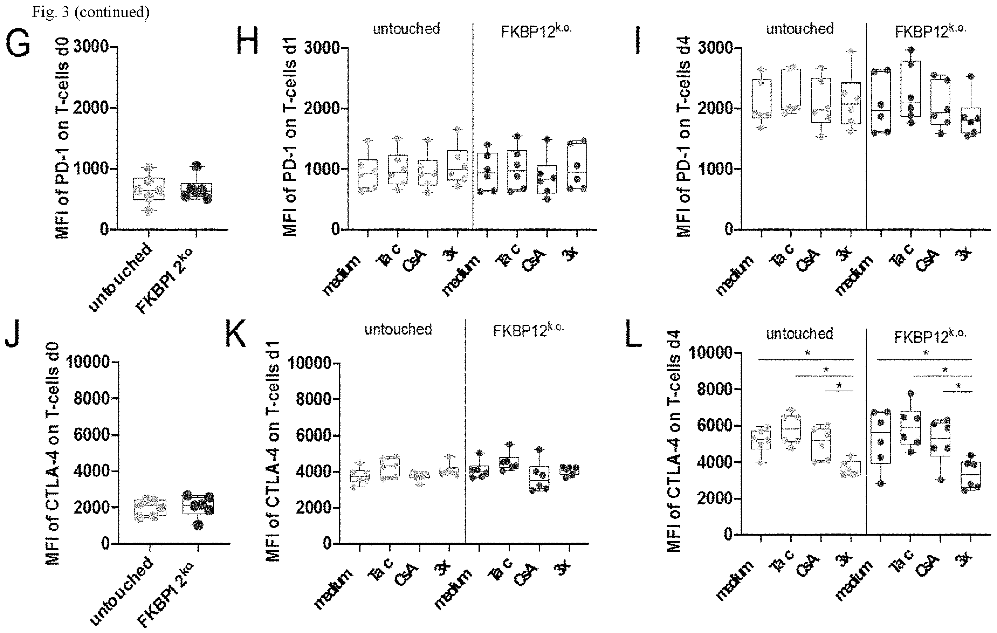

**Fig. 1****: Protocol outline for a GMP-compatible approach to induce Tacrolimus-resistance in a CMV-specific T-cell product using vector-free CRISPR-Cas9 technology**
   **a)** Individual steps of the production process.
   **b)** Representative purities of CMV-specific IFNγ-producing T-cells *pre-* and*post*-enrichment from peripheral blood mononuclear cells using an IFNγ capture assay after stimulation with overlapping peptide pools of CMV_{IE-1} and CMVₚₚ₆₅ for 6 h.
   **c)** K.O. efficiency of FKBP12 in CMV-specific T-cell products calculated using Synthego's ICE algorithm based on Sanger-sequencing of amplified DNA of the FKBP12 gene area.
   **d)** Expansion rates of electroporated CMV-specific T-cell products normalized to expansion rates of the unmodified control from d7 to d21 (following electroporation) calculated from living cell counts determined by trypan blue staining and counting in a Neubauer counting chamber.
**Fig. 2****: Functional profiles of CMV-specific T-cell products with disrupted FKBP12**
   CMV-specific stimulation of T-cell products at day 21 of culture using antigen-presenting cells (autologous lymphoblastic B-cell lines) pulsed with overlapping peptide pools of CMV_{IE-1} and CMVₚₚ₆₅ (6 h stimulation). Cytokines were captured in T-cells by addition of Brefeldin A after 1 h of stimulation and cells were stained using a fixation/permeabilization kit and fluorescently labelled antibodies. Data were acquired by multicolor flow cytometry. Immunosuppressants were added where indicated at the following clinically relevant doses: Tac = 6 ng/ml Tacrolimus; CsA = 120 ng/ml Cyclosporine A; Tac/Pred/MPA = 6 ng/ml Tacrolimus + 0.57 µg/ml Prednisolone + 2.7 µg/ml Mycophenolic acid. Stars symbolize p-values < 0.05, which were determined by tests for normal distribution (Shapiro-Wilk and Kolmogorov-Smirnov tests), followed by one-way ANOVA (normally distributed data sets) or Friedman test (not normally distributed data sets) and paired t-tests (normally distributed data sets) or Wilcoxon matched pairs signed rank tests (not normally distributed data sets) as posttests.
   **a)** Representative flow cytometry plots show CD4⁺ and CD8⁺ IFNγ and TNFα-producers in unmodified and FKBP12^{k.o.}-CMV-TCPs edited with synthetic sgRNA#2. Unstimulated and
   **b)** CMV -peptide-stimulated T-cell products are shown in the presence of the indicated immunosuppressants.
   **c)** Summary of CD4⁺ IFNγ-producers in unmodified and FKBP12-specific sgRNA#1-3/synthetic modified sgRNA#2 gene-edited FKBP12^{k.o.}-CMV-TCPs in the presence of the indicated immunosuppressants.
   **d)** Summary of CD8⁺ IFNγ-producers in unmodified and FKBP12-specific sgRNA#1-3/synthetic modified sgRNA#2 gene-edited FKBP12^{k.o.}-CMV-TCPs in the presence of the indicated immunosuppressants.
   **e)** Summary of CD4⁺ TNFα and IFNy-double-producers in unmodified and FKBP12-specific sgRNA#1-3/synthetic modified sgRNA#2 gene-edited FKBP12^{k.o.}-CMV-TCPs in the presence of the indicated immunosuppressants.
   **f)** Summary of CD4⁺ TNFα/IFNγ--double-producers in unmodified and FKBP12-specific sgRNA#1-3/synthetic modified sgRNA#2 gene-edited FKBP12^{k.o.}-CMV-TCPs in the presence of the indicated immunosuppressants.
   **g)** "VITAL" assays were performed to determine killing capacity of CMV-TCPs: percentage killing of CMV-specific peptide loaded autologous LCLs normalized to a population of allogenic LCLs by CMV-specific T-cells from FKBP12^{k.o.}- and unmodified CMV-TCPs added at a 10:1 ratio in absence or presence of the indicated immunosuppressants. Killing was calculated as described previously.³²
**Fig. 3****: FKBP12^{k.o.}-CMV-T-cell products after thawing: viability, activation/exhaustion and memory markers after culture in immunosuppressants**
   Unmodified and FKBP12^{k.o.}-CMV-TCPs (edited with synthetic sgRNA#2) were frozen in FCS with 10 % DMSO (indicated by the ice symbol) and stored in liquid nitrogen until thawing in a water bath at 37°C (indicated by the orange wavy lines). Culture was performed in complete medium without cytokines with addition of the indicated immunosuppressant (Tac = 6 ng/ml Tacrolimus; CsA = 120 ng/ml Cyclosporine A; 3x = 6 ng/ml Tacrolimus + 0.57 µg/ml Prednisolone + 2.7 µg/ml MPA) at d 0 and d 2. Addition of drugs is indicated by the pipette and a green teardrop. Stars symbolize p-values < 0.05, which were determined by tests for normal distribution (Shapiro-Wilk and Kolmogorov-Smirnov tests), followed by one-way ANOVA (normally distributed data sets) or Friedman test (not normally distributed data sets) and paired t-tests (normally distributed data sets) or Wilcoxon matched pairs signed rank tests (not normally distributed data sets) as posttests.
   **a)** Representative flow cytometry plot showing L/D and Annexin V staining of single cells after thawing and demonstrating the gating strategy: double negative staining (lower left quadrant) indicates the population of viable cells.
   **b)** Viability of single cells in unmodified and FKBP12^{k.o.}-CMV-TCPs directly after thawing (d0).
   **c)** Viability of single cells in unmodified and FKBP12^{k.o.}-CMV-TCPs cultured in the indicated immunosuppressant 24 h after thawing (d1).
   **d)** MFI of CD25 on single T-cells of unmodified and FKBP12^{k.o.}-CMV-TCPs directly after thawing (d0).
   **e)** MFI of CD25 on single T-cells of unmodified and FKBP12^{k.o.}-CMV-TCPs cultured in the indicated immunosuppressant 24 h after thawing (d1).
   **f)** MFI of CD25 on single T-cells of unmodified and FKBPI2^{k.o.}-CMV-TCPs cultured in the indicated immunosuppressant 96 h after thawing (d4).
   **g)** MFI of PD-1 on single T-cells of unmodified and FKBP12^{k.o.}-CMV-TCPs directly after thawing (d0).
   **h)** MFI of PD-1 on single T-cells of unmodified and FKBP12^{k.o.}-CMV-TCPs cultured in the indicated immunosuppressant 24 h after thawing (d1).
   **i)** MFI of PD-1 on single T-cells of unmodified and FKBP12^{k.o.}-CMV-TCPs cultured in the indicated immunosuppressant 96 h after thawing (d4).
   **j)** MFI of CTLA-4 on single T-cells of unmodified and FKBP12^{k.o.}-CMV-TCPs directly after thawing (d0).
   **k)** MFI of CTLA-4 on single T-cells of unmodified and FKBP12^{k.o.}-CMV-TCPs cultured in the indicated immunosuppressant 24 h after thawing (d1).
   **l)** MFI of CTLA-4 on single T-cells of unmodified and FKBP12^{k.o.}-CMV-TCPs cultured in the indicated immunosuppressant 96 h after thawing (d4).

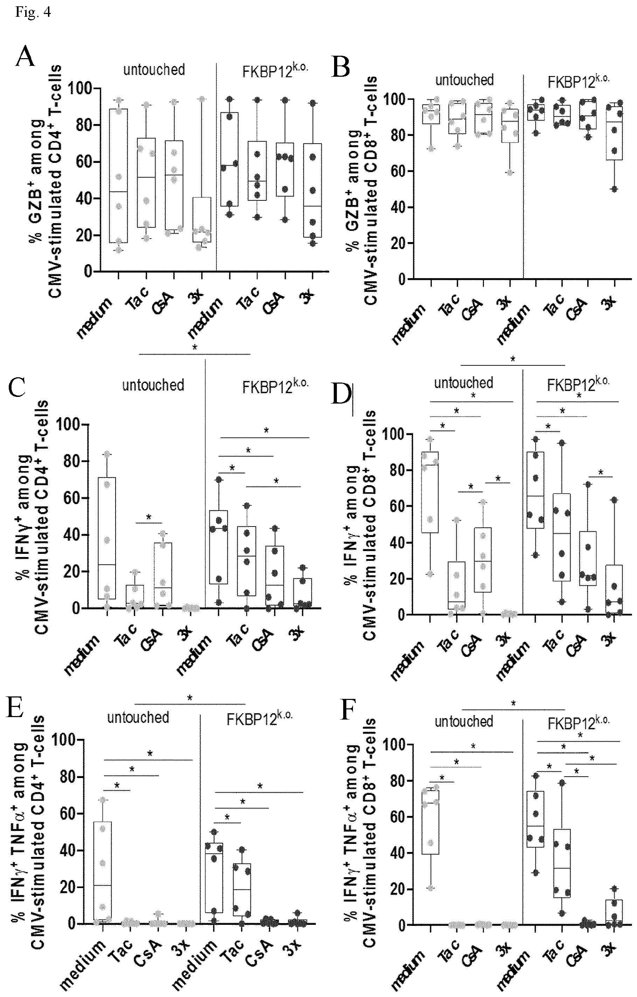

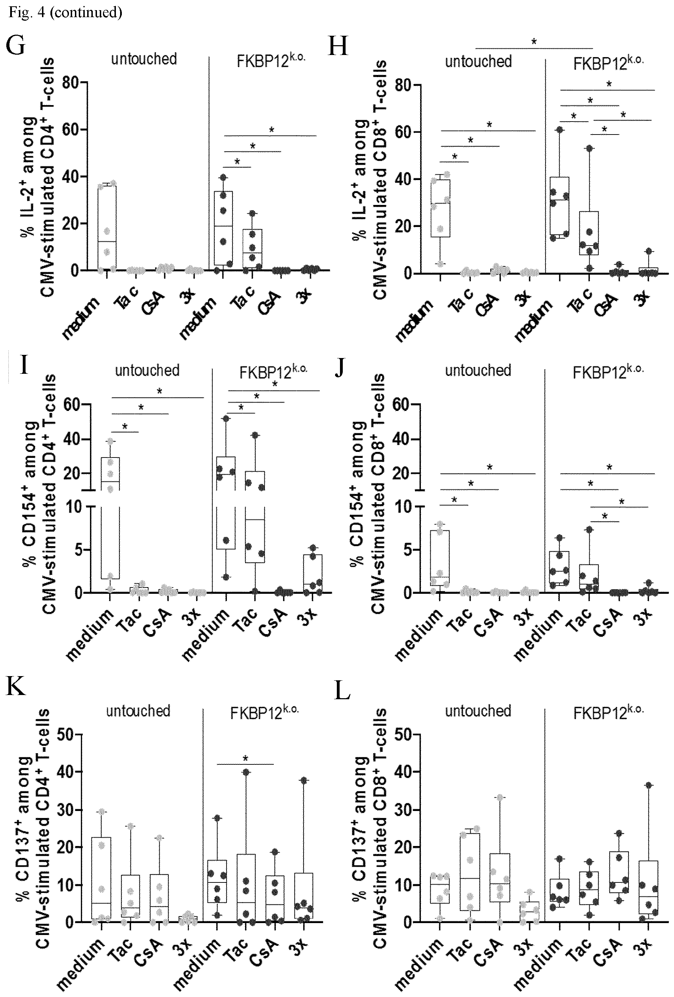

**Fig. 4****: FKBP12^{k.o.}-CMV-T-cell products after thawing: superior response to CMV-specific stimulation in presence of Tacrolimus compared to unmodified CMV-T-cell products**
   Unmodified and FKBP12^{k.o.}-CMV-TCPs (edited with synthetic sgRNA#2) were cultured in the indicated immunosuppressants (Tac = 6 ng/ml Tacrolimus; CsA = 120 ng/ml Cyclosporine A; 3x = 6 ng/ml Tacrolimus + 0.57 µg/ml Prednisolone + 2.7 µg/ml MPA) for 5 days after thawing. TCPs were re-stimulated with CMV -specific peptide pools delivered by autologous LCLs as antigen presenting cells for 6 h on day 5 after thawing. Autologous LCLs without peptides were added to unstimulated controls. Cytokines were captured intracellularly by addition of Brefeldin A after 1 h of stimulation. Stars symbolize p-values < 0.05, which were determined by tests for normal distribution (Shapiro-Wilk and Kolmogorov-Smirnov tests), followed by one-way Anova (normally distributed data sets) or Friedman test (not normally distributed data sets), respectively, and paired t-tests (normally distributed data sets) or Wilcoxon matched pairs signed rank tests (not normally distributed data sets), respectively, as posttests.
   **a)** Summary of proportions of GZB⁺ T-cells within the CD4⁺ T-cell population of unmodified and FKBP12^{k.o.}-CMV-TCPs following CMV-specific re-stimulation after 5 days of culture in the indicated immunosuppressants.
   **b)** Summary of proportions of GZB⁺ T-cells within the CD8⁺ T-cell population (gated as illustrated in c) of unmodified and FKBPI2^{k.o.}-CMV-TCPs following CMV-specific re-stimulation after 5 days of culture in the indicated immunosuppressants.
   **c)** Summary of proportions of IFNγ⁺ T-cells within the CD4⁺ T-cell population of unmodified and FKBPI2^{k.o.}-CMV-TCPs following CMV-specific stimulation after 5 days of culture in the indicated immunosuppressants. The background was subtracted to determine exclusively CMV-specific IFNγ-producers.
   **d)** Summary of proportions of IFNγ⁺ T-cells within the CD8⁺ T-cell population of unmodified and FKBPI2^{k.o.}-CMV-TCPs following CMV-specific stimulation after 5 days of culture in the indicated immunosuppressants. The background was subtracted to determine exclusively CMV-specific IFNγ-producers.
   **e)** Summary of proportions of TNFα⁺IFNγ⁺ T-cells within the CD4⁺ T-cell population of unmodified and FKBP12^{k.o.}-CMV-TCPs following CMV-specific stimulation after 5 days of culture in the indicated immunosuppressants. The background was subtracted to determine exclusively CMV-specific TNFαIFNγ double-producers.
   **f)** Summary of proportions of TNFα⁺IFNγ⁺ T-cells within the CD8⁺ T-cell population of unmodified and FKBP12^{k.o.}-CMV-TCPs following CMV-specific stimulation after 5 days of culture in the indicated immunosuppressants. The background was subtracted to determine exclusively CMV -specific TNFα⁺IFNγ⁺ double-producers.
   **g)** Summary of proportions of IL-2⁺ T-cells within the CD4⁺ T-cell population of unmodified and FKBPI2^{k.o.}-CMV-TCPs following CMV-specific stimulation after 5 days of culture in the indicated immunosuppressants. The background was subtracted to determine exclusively CMV-specific IFNγ-producers.
   **h)** Summary of proportions of IL-2⁺ T-cells within the CD8⁺ T-cell population of unmodified and FKBPI2^{k.o.}-CMV-TCPs following CMV-specific stimulation after 5 days of culture in the indicated immunosuppressants. The background was subtracted to determine exclusively CMV-specific IFNγ-producers.
   **i)** Summary of proportions of CD154⁺ T-cells within the CD4⁺ T-cell population of unmodified and FKBP12^{k.o.}-CMV-TCPs following CMV-specific stimulation after 5 days of culture in the indicated immunosuppressants. The background was subtracted to determine exclusively CMV-specific CD154⁺ CD4⁺ T-cells.
   **j)** Summary of proportions of CD154⁺ T-cells within the CD8⁺ T-cell population of unmodified and FKBP12^{k.o.}-CMV-TCPs following CMV-specific stimulation after 5 days of culture in the indicated immunosuppressants. The background was subtracted to determine exclusively CMV -specific CD154⁺ CD8⁺ T-cells.
   **k)** Summary of proportions of CD137⁺ T-cells within the CD4⁺ T-cell of unmodified and FKBP12^{k.o.}-CMV-TCPs following CMV-specific stimulation after 5 days of culture in the indicated immunosuppressants. The background was subtracted to determine exclusively CMV -specific CD137⁺ CD4⁺ T-cells.
   **l)** Summary of proportions of CD137⁺ T-cells within the CD8⁺ T-cell population of unmodified and FKBP12^{k.o.}-CMV-TCPs following CMV-specific stimulation after 5 days of culture in the indicated immunosuppressants. The background was subtracted to determine exclusively CMV-specific CD137⁺ CD4⁺ T-cells.

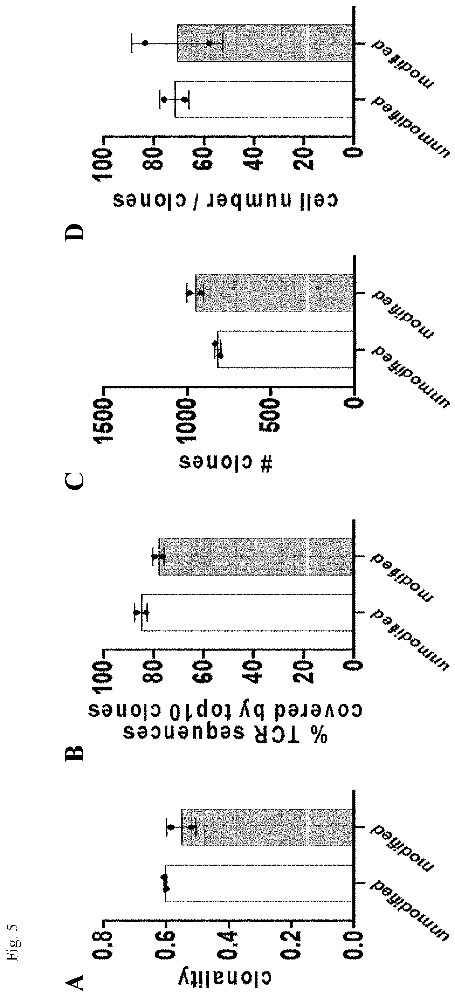

**Fig. 5****: FKBP12^{k.o.}-CMV-T-cell products and unedited products have a similar TCRβ repertoire**
   **a)** Clonality of FKBP12^{k.o.}-CMV-TCPs and CMV-TCPs. n=2. A clonality of 1 would signify a product consisting of only one clone (monoclonal), the lower the clonality, the more diverse the clonal composition of the product. The clonality is the inverse of the normalized Shannon's entropy.
   **b)** Proportion of total productive frequencies covered by the top 10 most represented clones. n=2.
   **c)** Total number of clones. n=2.
   **d)** Total productive frequencies divided by the number of clones.
**Fig. 6****: Cas9 protein is undetectable in FKBP12^{k.o.}-CMV-T-cell products**
   Proportions of T-cells in TCPs that stained positive for Cas9-specific fluorescently labelled antibodies at different time points after electroporation.

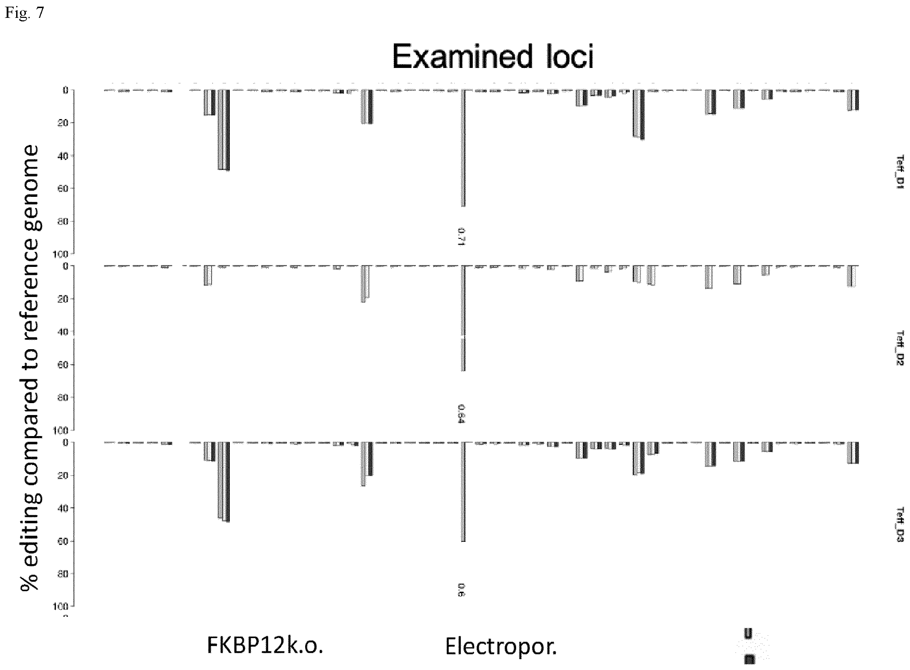

**Fig. 7****: Potential predicted off-target sites show no preferential editing in CMV-specific FKBP12^{k.o.} -TCPs.**
   A custom pooled primer pool was used to amplify predicted possible off-target sites and the on-target region and amplicons were subjected to next-generation sequencing. The sequences were aligned to a reference genome and aberrations are shown as bars. If bars do not deviate between edited (dark grey) and non-edited TCPs (untouched: black, electroporated: light grey), no preferential off-target editing is detected.

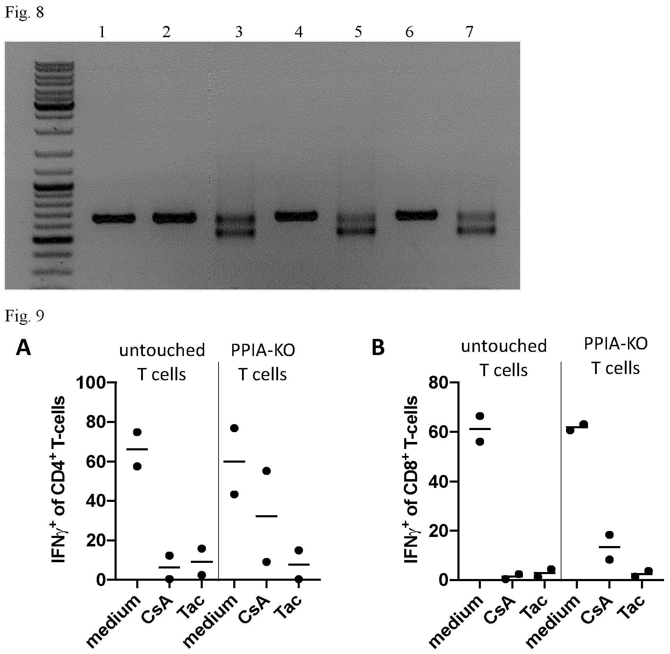

**Fig. 8****: Successful KO of PPIA in T cells**
   Illuminated agarose gel after electrophoresis shows PPIA-exon 1 PCR amplicons of untouched T cells as well as T cells electroporated with a control sgRNA-Cas9 complex (CD45 sgRNA 1³⁰) or four sgRNA-Cas9 mix targeting PPIA exon 1 (SEQ ID NO: 4-7). PPIA-specific CRISPR-Cas9 complexes efficiently introduced deletions in exon in three biological replicates (different healthy donors). Lanes 1: D1: untouched; 2: D1: control sgRNA; 3: D1: 4xPPIA sgRNA; 4:D2: control sgRNA; 5 D2 4xPPIA sgRNA; 6: D3: control sgRNA; 7: D3: 4xPPIA sgRNA.
**Fig. 9****: PPIA^{KO} Treg show partial resistance to CsA-induced reduction of cytokine production, but are sensitive to Tacrolimus.**
   Therapeutic doses of calcineurin inhibitors efficiently prevent cytokine production upon 6 h PMA/ionomycin stimulation in untouched wildtype T cells. In contrast, T cells with PPIA-KO partially retained ability to produce IFN-gamma in in the presence of Cyclosporine A, but remained unable to produce IFNγ in the presence of the calcineurin inhibitor Tacrolimus. Rescue of IFNγ production was more pronounced in CD4 T cells with PPIA-KO (A) compared to the CD8 T cells with PPIA-KO (B).

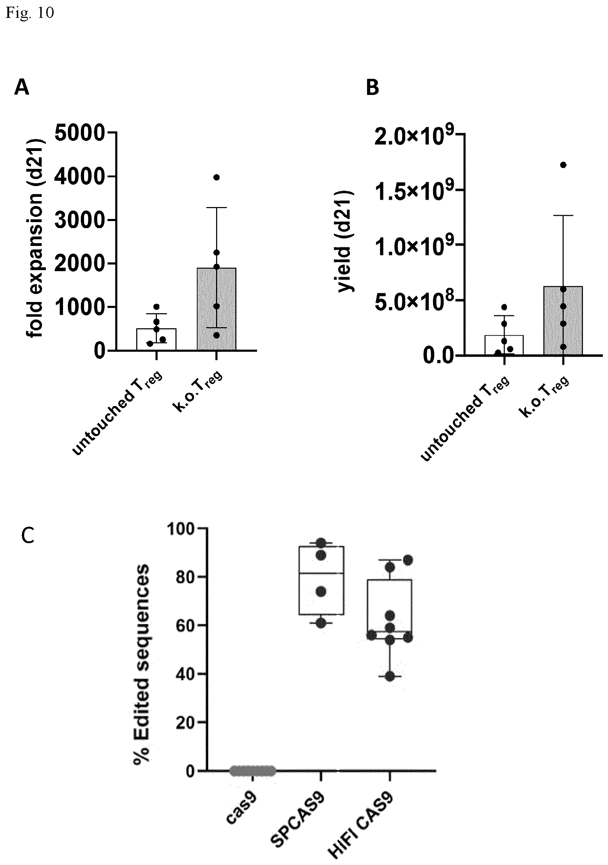

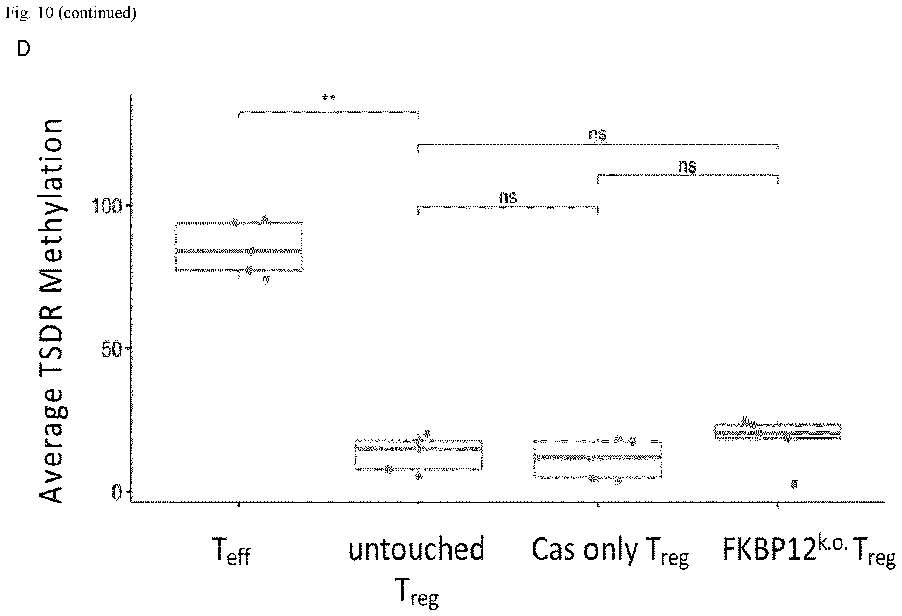

**Fig. 10****: Functionality of gene-edited FKBP12-/- Treg products**
   Cells were prepared as described in Example 3. After expansion for 21 days, the cells were analysed and compared with untouched Tregs regarding expansion rate and final yield (A: Expansion Rate k.o. Treg> conv. Treg; B: Yield k.o. Treg> conv. Treg), gene editing efficacy measured by Sanger sequencing of PCR amplified FKBP12 gene area and ICE analysis (Synthego) (C) and epigenetic identity, through bisulfite sequencing-based quantification of demethylation of the PCR-amplified Treg-specific demethylation region (TSDR) (D).

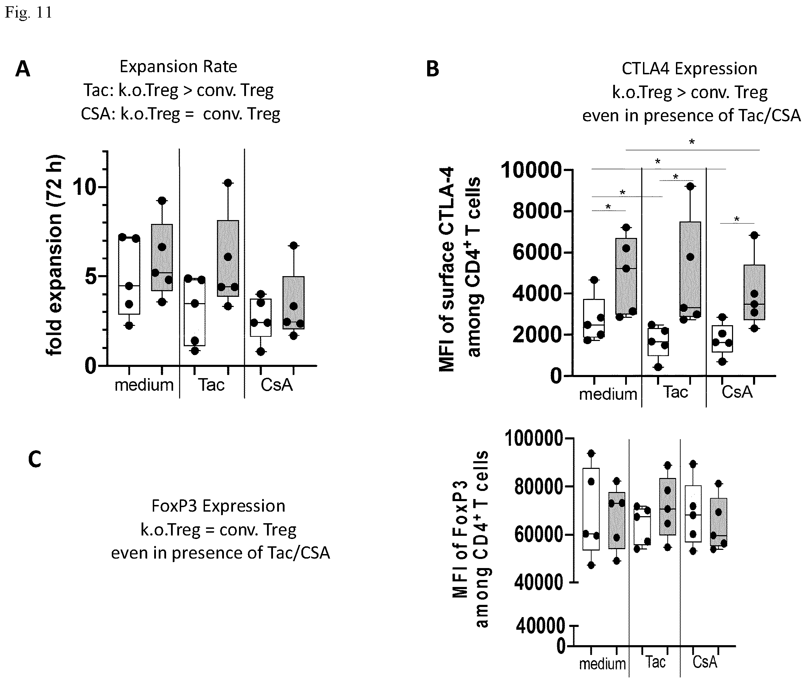

**Fig. 11****: Functional k.o.: FKBP12-/Treg are resistant to Tac but still sensitive to CSA (for rescue).**
   Exemplary regulatory T cells, expanded and treated as described in Figure 10. Edited and control Treg cells were subsequently expanded in medium with or without different calcineurin inhibitors and subsequently analysed for cell expansion (A) and with flow cytometry (B+C). As expected, Tacrolimus treatment reduced the proliferation of untouched Tregs, while FKBP12-KO Tregs retained fully proliferative capacity despite the drug treatment (A). FKBP12-KO Treg displayed higher expression of the inhibitory receptor CTLA4 (B). CTLA4 is known to suppress T cells and modulate pro-inflammatory responses by dendritic cells. Further, Foxp3 transcription factor expression assessed by flow cytometry was comparable between wildtype and FKBP12-KO, with a trend toward increased Foxp3 expression of FKBP12KO-Treg in the presence of Tacrolimus indicating potential for increased stability (C). Light bars are untouched expanded Treg, dark bars are FKBP12^{k.o.} expanded Treg. Tac was used at 6 ng/ml, CsA was used at 125 ng/ml, which corresponds to pharmacological doses that may, e.g., found in patient serum.

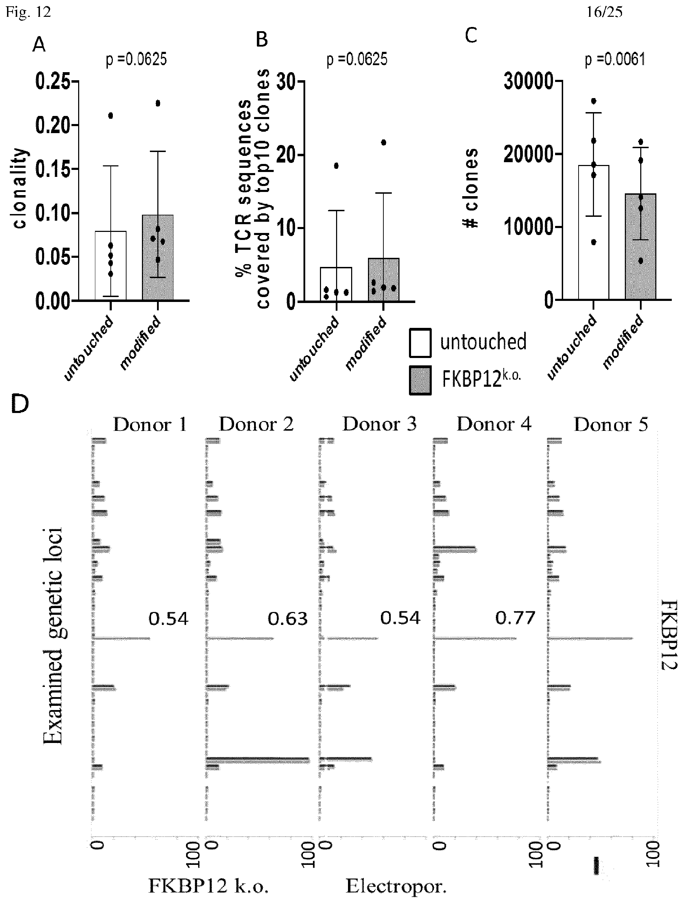

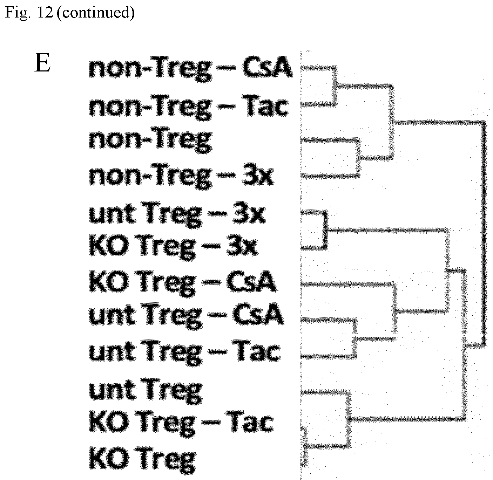

**Fig. 12****: FKBP12^{KO} Treg show normal clonal composition, no preferential off-target editing and a beneficial proteome.** TCR-beta sequencing revealed clonality (A), percental coverage by the top 10 clones (B) and total numbers of T cell clones in Treg products (C). A custom primer pool was used to amplify predicted possible off-target sites and the on-target region and amplicons were subjected to next-generation sequencing. The sequences were aligned to a reference genome and aberrations are shown as bars. If bars do not deviate between edited (dark grey) and non-edited (untouched or electroporated, light grey and black) TCPs no preferential off-target editing is detected (D). Clustering of samples calculated based on DigiWest technology, which was used to characterise the proteome of FKBP12^{KO} (KO) and untouched (unt) TCPs after exposition to the indicated immunosuppressant (CsA = Cyclosporine A, Tac = Tacrolimus) at therapeutic concentrations (E).

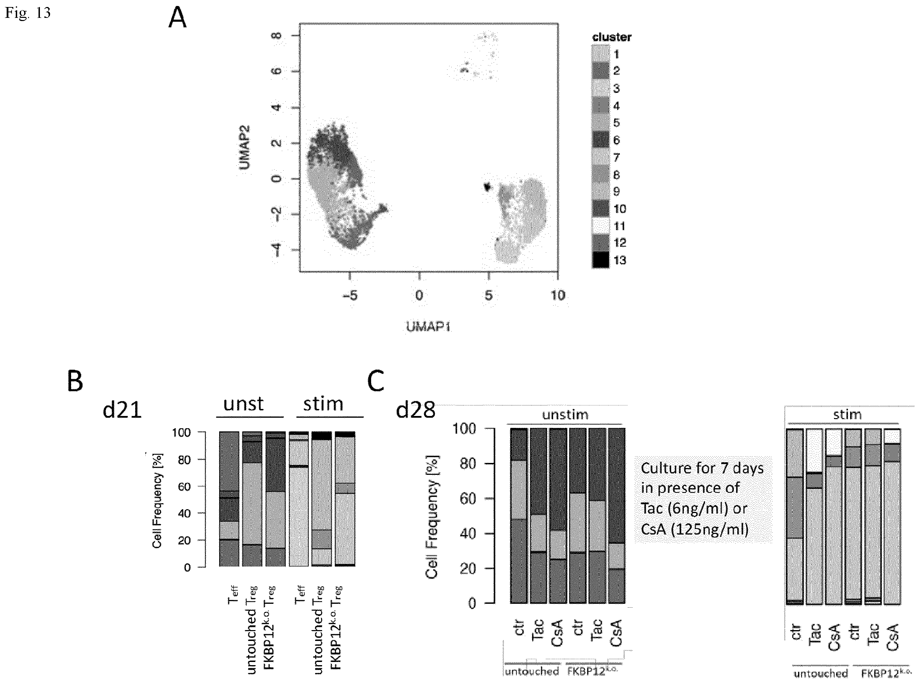

**Fig. 13****: CITEseq reveals FKBP12^{KO} Treg products resemble untouched Treg products, are resistant to Tacrolimus-induced cluster distribution changes, but remain sensitive to Cyclosporine A.**
   CITE-seq analysis of Treg products. Immunosuppressant Tacrolimus (Tac) or Cyclosporin A (CsA) were added where indicated, n=3. UMAP representation of cells from unmodified and *FKBP12*^{KO} Treg products. Transcriptionally similar clusters were identified using shared nearest neighbor (SNN) modularity optimization. Cluster distribution within unmodified/*FKBP12*^{KO} Treg products and non-Treg (Teff) in unstimulated and stimulated conditions at d21 (B). Cluster distribution within unmodified and *FKBP12*^{KO} Treg products exposed to Tacrolimus (Tac) or Cyclosporine A (CsA) or medium for 7 days in unstimulated and stimulated conditions at d28 (C).

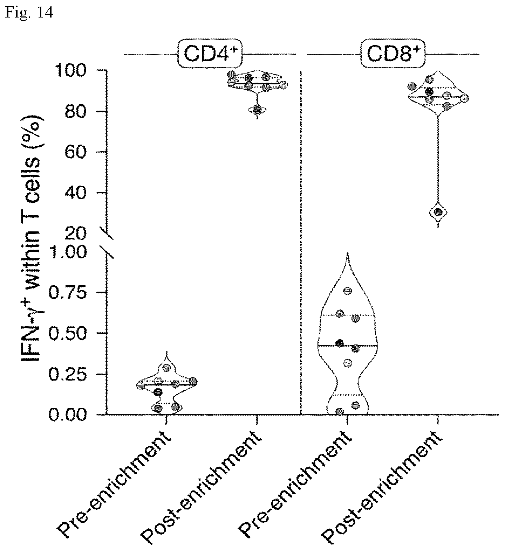

**Figure 14****: Schematic outline of the experimental setup to isolate and expand SARS-CoV-2-specific T-cells following CRISPR-Cas9-mediated knockout of *FKBP12* to induce Tacrolimus-resistance**
   Purities of SARS-CoV-2-specific IFN-γ-producing CD4⁺ and CD8⁺ T-cells *pre-* and post-enrichment, where each dot color represents one individual donor (n=8).

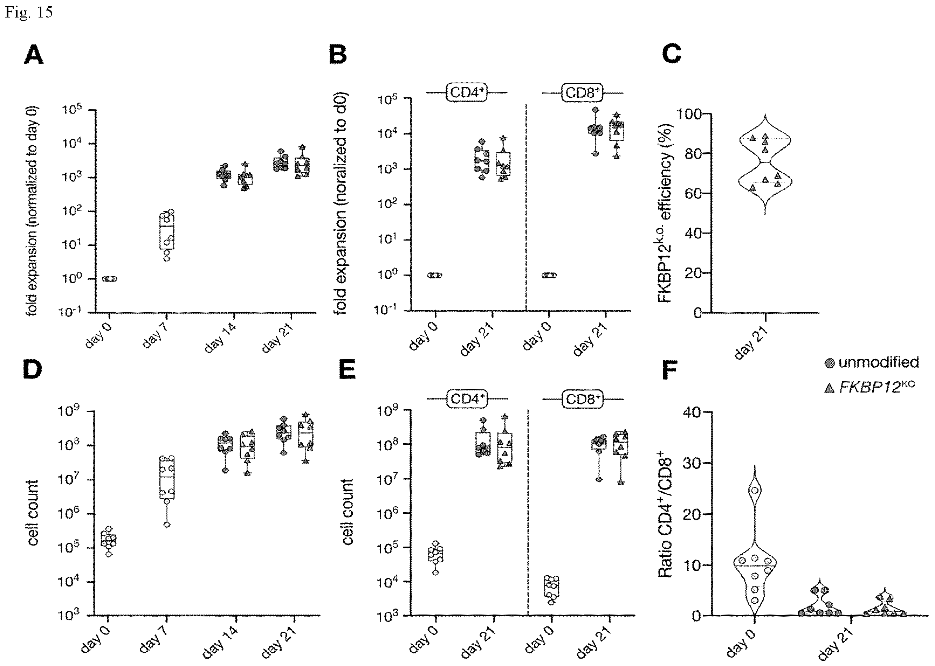

**Figure 15****: Expansion rates and CD4⁺/CD8⁺ T-cell ratio of unmodified control and *FKBP12*^{KO} SARS-CoV-2-specific TCPs over 21 days of culture**
   Experimental setup same as in Figure 14. n=8
   A) Expansion rates (fold-expansion) from day 0 to day 21 of unmodified control and *FKBP12*^{KO} SARS-CoV-2-specific TCPs normalized to day 0.
   B) Expansion rates (fold-expansion) from day 0 to day 21 of CD4⁺ and CD8⁺ T-cells of unmodified control and *FKBP12*^{KO} SARS-CoV-2-specific TCPs normalized to day 0.
   C) Knockout efficiency of *FKBP12*^{KO} SARS-CoV-2-specific TCPs at day 21.
   D) Expansion (cell count) from day 0 to day 21 of unmodified control and *FKBP12*^{KO} SARS-CoV-2-specific TCPs.
   E) Expansion (cell count) from day 0 to day 21 of CD4⁺ and CD8⁺ T-cells of unmodified control and *FKBP12*^{KO} SARS-CoV-2-specific TCPs.
   F) Ratio of CD4⁺/CD8⁺ T-cells at day 0 and day 21 of unmodified control and *FKBP12*^{KO} SARS-CoV-2-specific TCPs.

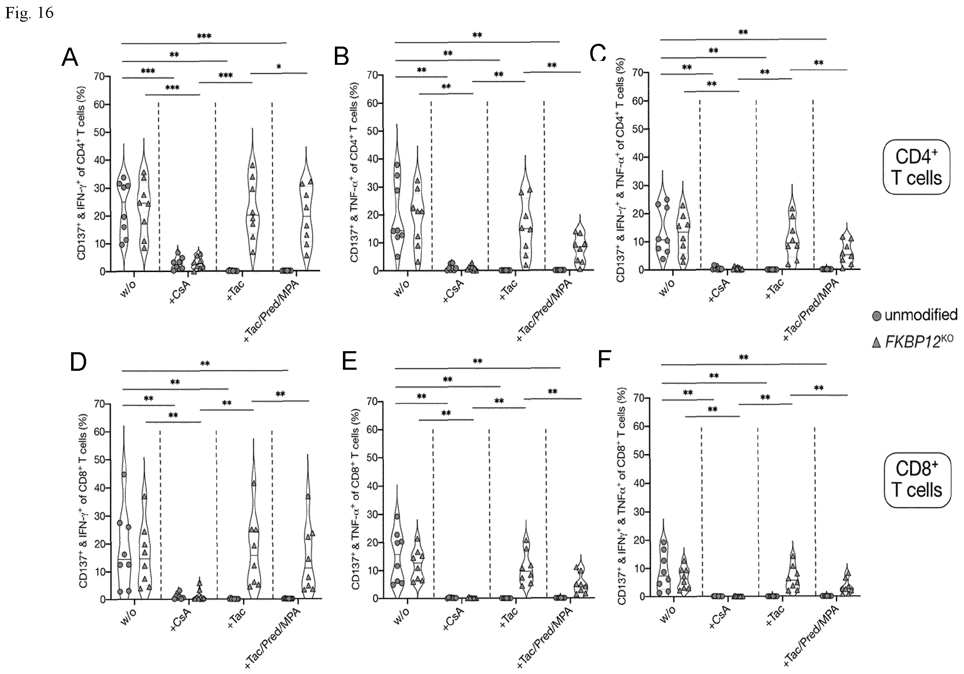

**Figure 16****: Functional analysis of SARS-CoV-2-specific unmodified control and *FKBP12*^{KO} TCPs**
   SARS-CoV-2-specific stimulation of unmodified control and *FKBP12*^{KO} TCPs on day 21 of culture. Immunosuppressants were added where indicated: CsA Cyclosporine A; Tac = Tacrolimus; Tac/Pred/MPA = Tacrolimus + Prednisolone + Mycophenolic acid. n=8; *p< 0.05, **p < 0.01, ***p < 0.001.
   A) Quantified data for the IFN-γ production of SARS-CoV-2-activated (CD137⁺) CD4⁺ T-cells in unmodified and *FKBP12*^{KO} SARS-CoV-2-specific TCPs after 16 h of stimulation with SARS-CoV-2 peptide pool in the presence or absence of respective immunosuppressants.
   B) Quantified data for the TNF-α production of SARS-CoV-2-activated (CD137⁺) CD4⁺ T-cells in unmodified and *FKBP12*^{KO} SARS-CoV-2-specific TCPs after 16 h of stimulation with SARS-CoV-2 peptide pool in the presence or absence of respective immunosuppressants.
   C) Quantified data for the IFN-y & TNF-α production of SARS-CoV-2-activated (CD137⁺) CD4⁺ T-cells in unmodified and *FKBP12*^{KO} SARS-CoV-2-specific TCPs after 16 h of stimulation with SARS-CoV-2 peptide pool in the presence or absence of respective immunosuppressants.
   D) Quantified data for the IFN-γ production of SARS-CoV-2-activated (CD137⁺) CD8⁺ T-cells in unmodified and *FKBP12*^{KO} SARS-CoV-2-specific TCPs after 16 h of stimulation with SARS-CoV-2 peptide pool in the presence or absence of respective immunosuppressants.
   E) Quantified data for the TNF-α production of SARS-CoV-2-activated (CD137⁺) CD8⁺ T-cells in unmodified and *FKBP12*^{KO} SARS-CoV-2-specific TCPs after 16 h of stimulation with SARS-CoV-2 peptide pool in the presence or absence of respective immunosuppressants.
   F) Quantified data for the IFN-y & TNF-α production of SARS-CoV-2-activated (CD137⁺) CD8⁺ T-cells in unmodified and *FKBP12*^{KO} SARS-CoV-2-specific TCPs after 16 h of stimulation with SARS-CoV-2 peptide pool in the presence or absence of respective immunosuppressants.

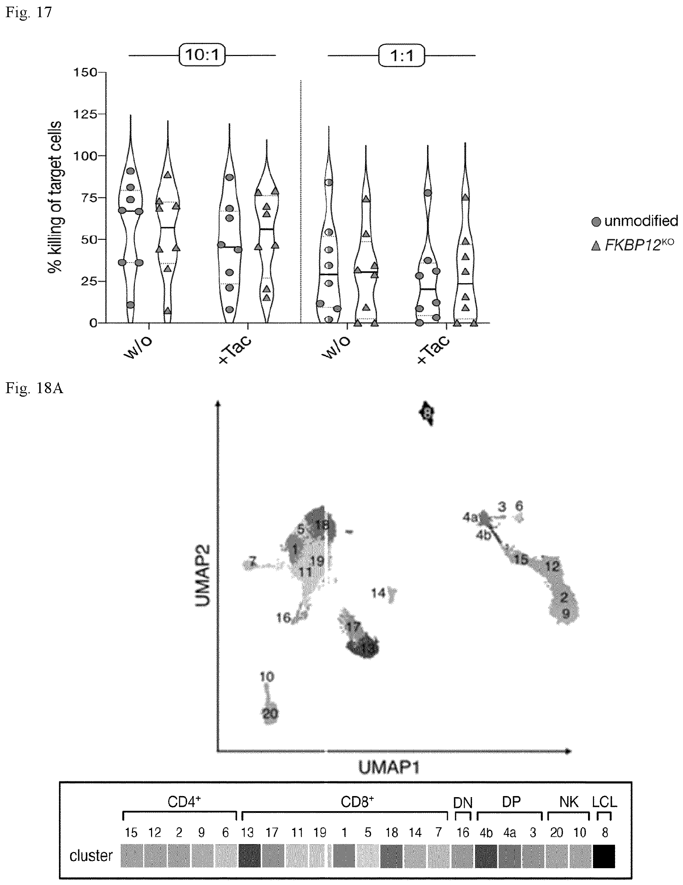

**Figure 17****: Killing capacity of SARS-CoV-2 peptide loaded autologous target T-cells by unmodified control and *FKBP12*^{KO} SARS-CoV-2-specific TCPs**
   SARS-CoV-2-specific T-cell-mediated cytotoxicity of unmodified control and *FKBP12*^{KO} TCPs at day 21 of culture. Percentage killing of SARS-CoV-2 peptide pool loaded autologous target T-cells by unmodified control and *FKBP12*^{KO} SARS-CoV-2-specific TCPs at 10:1 and 1:1 ratio (T-cells:LCLs) in the presence or absence of Tac.

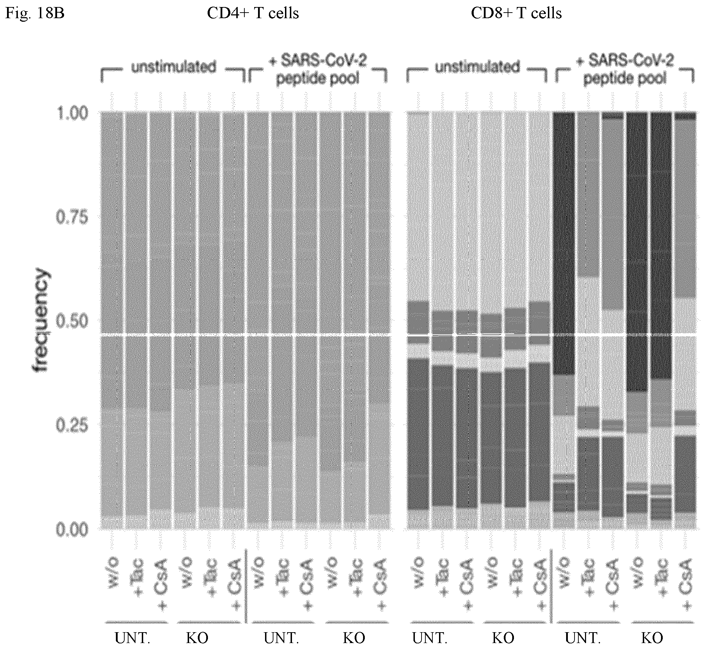

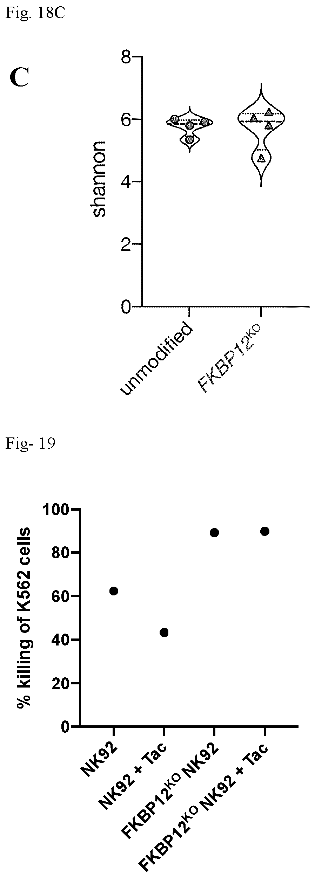

**Figure 18****: Single cell CITEseq and TCR repertoire of unmodified control and *FKBP12*^{KO}. SARS-CoV-2-specific TCPs**
   CITE-seq and TCR analysis of SARS-CoV-2-specific TCPs at day 21 of culture. Immunosuppressant Tacrolimus (Tac) or Cyclosporin A (CsA) were added where indicated. n=4
   **A)** UMAP representation of unmodified and *FKBP12*^{KO} TCPs. Transcriptionally similar clusters were identified using shared nearest neighbor (SNN) modularity optimization.
   **B)** Cluster distribution within unmodified control (UNT.) and *FKBP12*^{KO} (KO) SARS-CoV-2-specific TCPs in the presence or absence of IS as well as in unstimulated and stimulated condition. Left panel: CD4+ T cells, right panel: CD8+ T cells.
   **C)** TCR diversity represented by Shannon entropy of unmodified control and *FKBP12*^{KO} TCPs.
**Figure 19****: Killing efficiency pf FKBP12^{KO} NK cells**
   NK92 were electroporated with Cas9 protein and sgRNA#1(Table 1). After 5 days they were incubated with K562 cells at a ratio of 1:1. Killing capacity was calculated by the reduction in frequency of K562 cells. n = 1.

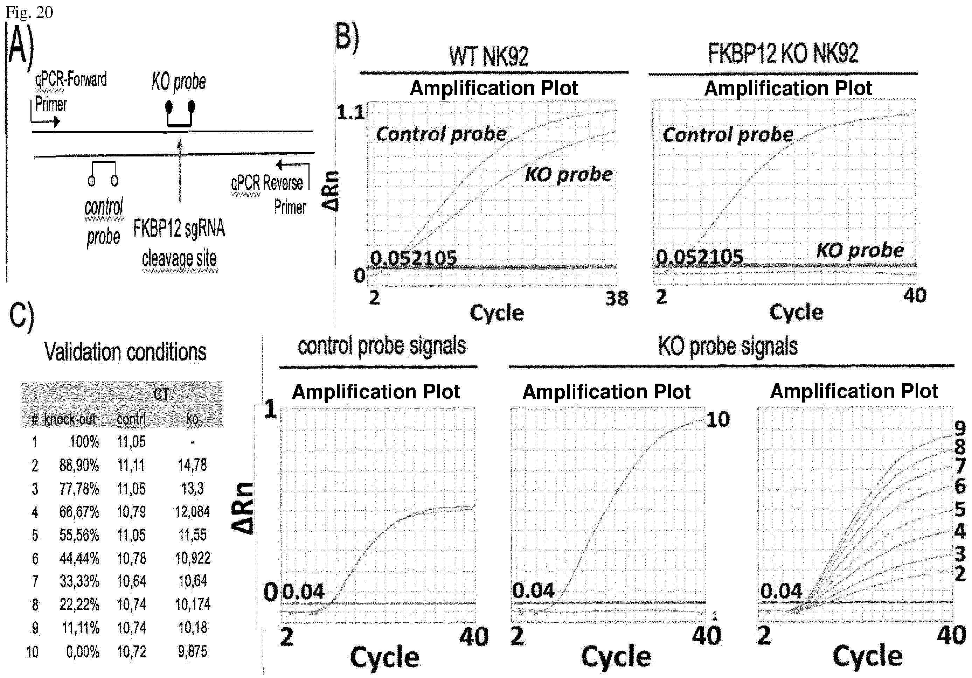

**Fig. 20** **Editing efficacy in NK92 estimated using a quantitative real time (qRT) PCR-based assay.**
   **A)** A 547bp fragment covering the targeted FKBP12 locus was amplified from genomic DNA by PCR, and the purified PCR product is used as template during TacMan qRT-PCR Assay with primers to amplify a 184bp DNA fragment. Reporter dyes: 1) a FAM-labelled KO probe designed to overlap the FKBP12 sgRNA#1 cleavage site, and 2) a SUN-fluorophore labelled control probe located upstream to the cleavage site which serves as an internal control for efficient amplification.
   **B)** Analysis of wildtype (WT) NK92 showed efficient signaling of both control and KO probe.
   **C)** In a FKBP12-edited NK92 cell line, no signal was detected from the KO probe indicating a high degree of editing (>90% KO) based on previous experiment from validation experiments. In validation experiments, FKBP12-KO Treg gDNA was used as reference and then mixed with control unedited gDNA.

### Tables

**Table 1: Target sequences and sequences of exemplary FKBP12-specific guide RNAs (sgRNA targeting SEQ ID NO: 1 is the guide RNA of the invention. sgRNA targeting SEQ ID NO: 2 or 3 are comparative guide RNAs).**

| | DNA Target sequence of sgRNA (SpCas9) | Spacer sequence of sgRNA | Full sequence of sgRNA (with standard scaffold sequence for SpCas9) |
|---|---|---|---|
| sgRN A#1 | [?] GCACTACAC- CGGTGAG- TCGG (SEQ ID NO: 2) | GGACUACAC- CGGUG- AGUCGG (SEQ ID NO: 15) | GCACUACACCGGUGAGUCGGGUUUUA- GAGCUAGAAAUAGCAAGUUAAAAU- AAGGCUAGUCCGUUAUCAACUUGAAAAA GUGGCACCGAGUCGGUGC (SEQ ID NO: 18) |
| sgRN A#2 | GGGCGCAC- CTTCCCCAA- GCG (SEQ ID NO: 1) | GGGCGCAC- CUUCCCCAA- GCG (SEQ ID NO: 14) | GGGCGCACCUUCCCCAAGCGGUUUUA- GAGCUAGAAAUAGCAAGUUAAAAU- AAGGCUAGUCCGUUAUCAACUUGAAAAA GUGGCACCGAGUCGGUGC (SEQ ID NO: 17) |
| sgRN A#3 | CGGTGAG- TAG- TGGCGCGCGG (SEQ ID NO: 3) | GGGUG- AGUAGUG- GCGCGCGG (SEQ ID NO: 16) | CGGUGAGUAGUGGCGCGGUUUUA- GAGCUAGAAAUAGCAAGUUAAAAU- AAGGCUAGUCCGUUAUCAACUUGAAAAA GUGGCACCGAGUCGGUGC (SEQ ID NO: 19) |

**Table 2: Target sequences and sequences of exemplary cyclophilin A-specific guide RNAs which may be used in addition to the guide RNA of the invention (sgRNA targeting SEQ ID NO: 5 and/or 6 is preferred).**

| | DNA Target sequence of sgRNA (SpCas9) | Spacer sequence of sgRNA | Full sequence of sgRNA (with standard scaffold sequence for SpCas9) |
|---|---|---|---|
| sgRN A#4 | CGTGG- TATAAAAGGG GCGGG (SEQ ID NO: 4) | GGUG- GUAUAAAAGG GGCGGG (SEQ ID NO: 20) | CGUGGUAUAAAAGGGGCGGGGUUUUA- GAGCUAGAAAUAGCAAGUUAAAAU- AAGGCUAGUCCGUUAUCAACUUGAAAA AGUGGCACCGAGUCGGUGC (SEQ ID NO: 24) |
| sgRN A#5 | CGGCGGTGGC GTCTGCAAAA (SEQ ID NO: 5) | GGGCGGUG- GCGUCUG- CAAAA (SEQ ID NO: 21) | CGGCGGUGGCGUCUGCAAAAGUUUUA- GAGCUAGAAAUAGCAAGUUAAAAU- AAGGCUAGUCCGUUAUCAACUUGAAAA AGUGGCACCGAGUCGGUGC (SEQ ID NO: 25) |
| sgRN A#6 | CGCCGCCCGCC CGACCTCAA (SEQ ID NO: 6) | GGCCGCCCGC CCGACCUCAA (SEQ ID NO: 22) | CGCCGCCCGCCCGAC- CUCAAGUUUUAGAGCUAGAA- AUAGCAAGUUAAAAUAAGGCUAGUC- CGUUAUCAACUUGAAAAAGUGGCACCG AGUCGGUGC (SEQ ID NO: 26) |
| sgRN A#7 | GCCGCGTCTCC TTTGAGGTC (SEQ ID NO: 7) | GCCGCGUCUC CUUUGAGGUC (SEQ ID NO: 23) | GCCGCGUCUCCUUUGAGGUCGUUUUA- GAGCUAGAAAUAGCAAGUUAAAAU- AAGGCUAGUCCGUUAUCAACUUGAAAA AGUGGCACCGAGUCGGUGC (SEQ ID NO: 27) |

### EXAMPLES

### Example 1 - FKBP12^{k.o.} T cells

### Results

### Integration of FKBP12 knockout into the clinical protocol for CMV-specific T-cell product generation

The inventors adapted a vector-free protocol for electroporation and ribonucleoprotein-based knockout³⁰ of FKBP12 to make a virus-specific Tacrolimus-resistant TCP (FKBP12^{k.o.}-CMV-TCP)¹⁹ (**Fig. 1a**). For this, CMV-specific T-cells were isolated from peripheral blood mononuclear cells (PBMCs), based on their interferon (IFN)γ secretion¹³, after activation by CMV_{IE-1/pp-65} overlapping peptide pools (**Fig. 1b**). These cells were expanded for one week. FKBP12 knockout was achieved by electroporation with ribonucleoprotein complexes of Cas9 and different sgRNAs, selected based on specificity *in silico*-prediction (**Fig. 1c**)^{33,34}. The sgRNAs used, targeting SEQ ID NO: 1, 2 and 3, and comprising SEQ IS Nos 17-19, differed according to the respective target sequence within the coding gene for FKBP12 and the mode of production (*in vitro* transcribed vs. fully synthetic [synt.]). The knockout efficiencies differed between the sgRNAs tested. For GMP compatibility reasons, we investigated one synt. sgRNA with 2O'-methyl-3'phosphothioate modifications in the first and last 3 nucleotides, which are reported to enhance editing frequencies in T-cells³⁵. Indeed, the synthetic version of sgRNA#2 improved k.o. efficacy of the sgRNA (**Fig. 1c**). The sgRNA#2 (targeting SEQ ID NO: 1) was chosen to be produced as a GMP compliant fully synthetic sgRNA due to its superior function in maintaining T-cell function in the presence of Tacrolimus compared to the other two sgRNAs (**Fig. 2** etc). We adapted an existing electroporation protocol for polyclonal T-cells³⁰ to our CMV-TCPs using an electroporation device suitable for GMP applications, using closed cartridges and electroporation conditions that preserved similar expansion rates to unmodified controls (**Fig. 1d**). The expansion rates are normalized to the unmodified controls, which did not undergo electroporation, to determine whether the electroporation/gene editing procedures altered the expansion capability of the cells (**Fig. 1d**). The expansion rate of the electroporated and gene edited cells was not different to the control, indicating that the electroporation procedure and gene editing did not influence the overall cell expansion rate. (**Fig. 1d**).

For functional analyses, we exposed TCPs to clinical doses of immunosuppressive drugs. The calcineurin inhibitor (CNI) Tacrolimus, which requires FKBP12 as an adaptor protein, is the main target in this project. To confirm specificity of the Tacrolimus-targeted approach, we used an alternative CNI, CsA, which depends on cyclophiloinA/Peptidylprolyl isomerase A (PPIA) as its adaptor protein. To test the functionality of the modified T-cells in the clinical context of solid organ transplant (SOT) recipients receiving triple immunosuppression (IS), we used classical triple IS consisting of Tacrolimus, Prednisolone (requiring the glucocorticoid receptor, which acts itself as transcription factor) and Mycophenolic acid, which directly acts on nucleotide synthesis.

### Cytokine production in the presence of Tacrolimus is rescued by FKBP12^{k.o.} in T-cell products

A major function of effector T-cells is the production of anti-viral cytokines to instruct other immune cells and induce an antiviral state in infected cells. Producers of multiple cytokines show particularly high antiviral functionality³⁶. Upon CMV-specific re-stimulation on day 21 of culture, we recorded similar proportions of producers of the antiviral cytokines IFNγ and TNFα among CD4⁺ and CD8⁺ T-cells in FKBP12^{k.o}-CMV-and unmodified CMV-TCPs **(****Fig. 2a****-e).** Addition of immunosuppressive drugs during the stimulation significantly decreased IFNγ and TNFα /IFNy -double-producers among CD4⁺ (**Fig. 2b****, d**) and CD8⁺ T-cells (**Fig. 2c****, e**), which was partially rescued by FKBP12^{k.o.} **(****Fig. 2b****-e).** Although cytokine production improved in all FKBP12^{k.o.} conditions in the presence of a clinically relevant dose of Tacrolimus, only samples electroporated with sgRNA#2 retained physiological levels of cytokine producers in stimulated TCPs (**Fig. 2b****-e**). We therefore tested sgRNA#2 as a GMP-compliant synthetic modified sgRNA, with 2O'-methyl-3'phosphothioate modifications in the first and last 3 nucleotides, which are reported to enhance editing frequencies in T-cells³⁵. Using synthetically modified sgRNA#2, we achieved higher editing efficacy (**Fig. 1c**) and better recovery of cytokine production, especially in the presence of clinically relevant doses of triple IS, during stimulation **(****Fig. 2a****-e)**.

### FKBP12^{k.o.}-CMV-TCPs demonstrate comparable killing capacity to unmodified CMV-TCPs

Since antiviral T-cells induce targeted elimination of infected cells by cytotoxicity, we tested the cytotoxic killing capacity of TCPs^{32,37}. Unlike antiviral cytokines, which were strongly affected by short-term classical immunosuppressive treatment (**Fig. 2a****-e**), the T-cell mediated killing of CMV-peptide loaded target cells was unaffected by short-term incubation with immunosuppressive drugs at clinical doses **(****Fig. 2f****)**. Notably, the data demonstrated no differences in killing capacity of FKBP12^{k.o}-CMV-TCPs compared to unmodified CMV-TCPs, irrespective of the applied sgRNA.

### Enhanced survival of FKBP12^{k.o.} CMV-specific T-cell products in presence of Tacrolimus

Before clinical application, antiviral TCPs are typically cryopreserved until all release criteria and safety tests are accomplished. Therefore, we froze all edited and non-edited CMV-TCPs. **We** focused on synthetic sgRNA#2 in subsequent experiments, as it was the most effective **(****Fig. 2****)** and preferred GMP compliant material. Synthetic sgRNA#2-treated FKBP12^{k.o}-CMV- and unmodified CMV-TCPs were thawed and evaluated for viability and functionality post-thawing **(****Fig. 3a****).** The viability of the T-cells did not differ directly after thawing (d0; **Fig. 3b**), however, we found a slightly bigger proportion of living cells in thawed FKBPI2^{k.o.}-CMV-TCPs compared to unmodified CMV-TCPs following 24h incubation with Tacrolimus (**Fig. 3c**; p = 0.06, normalized to culture in medium without immunosuppressants). Of note, 24 h incubation with other immunosuppressants revealed no differences in viability (**Fig. 3c**).

### Exhaustion and phenotypic memory markers of FKBP12^{k.o.}- and unmodified CMV-specific T-cell products following 4 days of consecutive culture in the presence of immunosuppressants

We evaluated the cell surface expression of distinct phenotypic markers for T-cell activation (CD25) and exhaustion (programmed death (PD)-1 and Cytotoxic T-lymphocyte-associated Protein (CTLA)-4 (CTLA-4)) in the presence of clinical doses of immunosuppressants at 0, 1- and 4-days post-thawing by flow cytometry. We detected high CD25 expression directly after thawing **(****Fig. 3d****),** which diminished after 24 h **(****Fig. 3e****-f).** The mean fluorescence intensity (MFI) of CD25 remained significantly higher in TCPs cultured for 24 h with clinical doses of classical triple IS, irrespective of whether FKBP12 was genetically edited or not **(****Fig. 3d****).** However, no differences in CD25 expression were recorded following 4 days of culture in the presence of immunosuppressants (added every 48 h) **(****Fig. 3f****).** Next, we assessed exhaustion markers in the cell products. To this end, we determined the expression of PD-1 and CTLA-4 during 4 days of culture post-thawing. For all conditions, PD-1 and CTLA-4 expression increased equally over the observed time course (**Fig. 3g****-l**). Interestingly, CTLA-4 protein levels (MFI) were significantly lower in TCPs treated with triple IS irrespective of FKBP12 editing (**Fig. 3l**).

### Functional assessment of CMV-specific T-cell products exposed to Tacrolimus

Next, we assessed the functional capacity of TCPs for virus-specific effector functions after the freezing/thawing process and 5-day culture in immunosuppressants, similar to the environment faced upon injection into an immunosuppressed patient. To this end, TCPs were stimulated with CMV-specific peptide pools and assessed for intracellular accumulation of cytokines as well as the presence of activation markers.

Granzyme B (GZB) mediates apoptosis of virus-infected target cells in conjunction with perforin. Therefore, we measured intracellular accumulation of GZB in TCPs upon CMV-specific re-stimulation and found a stimulation-dependent increase of GZB in CD4⁺ T-cells. Notably, the majority of CD8⁺ T-cells presented with pre-formed GZB even before re-stimulation. Neither gene-editing nor culture with immunosuppressants at clinical doses impacted the proportion of CMV-stimulated GZB⁺ CD4⁺ (**Fig. 4a**) or GZB⁺ CD8⁺ T-cells (**Fig. 4b**) in the CMV - TCPs. Next, we measured IFNγ and TNFα-production in response to CMV-specific stimulation after thawing of the TCPs and culture in immunosuppressants **(****Fig. 4c, d****).** CD4⁺ and CD8⁺ T-cells produced IFNγ in response to CMV-stimulus after culture with Tacrolimus, although IFNγ-producers among CD4⁺ and CD8⁺ T-cells were significantly more frequent in FKBP12^{k.o.}-CMV-TCPs compared to unmodified CMV-TCPs after culture with Tacrolimus (**Fig. 4c****, d**). Remarkably, CD4⁺ and CD8⁺ IFNy/TNFα-double-producers were also significantly more abundant among CD4⁺ and CD8⁺ T-cells in FKBP12^{k.o.}- compared to unmodified CMV-TCPs, in which they were almost absent (**Fig. 4e****, f**). CD8⁺ IFNγ/TNFα-double-producers were significantly more frequent among CD8⁺ T-cells from FKBP12^{k.o.}-CMV-TCPs cultured in Tacrolimus compared to CsA, which was not evident with unmodified CMV-TCPs (**Fig. 4f**). The tendency of enhanced cytokine producers among cells cultured in Tacrolimus vs. cell cultured in Cyclosporine A was also observed in CD4⁺IFNγ/TNFα-double-producers and both CD4⁺ and CD8⁺ IFNγ producers from FKBP12^{k.o.}-CMV-, but not unmodified TCPs (**Fig. 4c****-f**). In contrast, in unmodified TCPs, IFNγ producers were more frequent among the CsA-treated compared to the Tacrolimus-treated cultures (**Fig. 4c****, d**). CD4⁺ and CD8⁺ IFNγ-producers (**Fig. 4c****, d**) and CD8⁺IFNγ/TNFα-double-producers (**Fig. 4e****, f**) were more frequent in cultures of FKBP12^{k.o.}-CMV-TCPs cultured in triple IS compared to their unmodified counterpart.

IL-2 is mainly produced by memory T-cells with a low differentiation state and high proliferative potential, which contribute to the establishment of long-term memory³⁸. A fraction of CD4⁺ (**Fig. 4g**) and CD8⁺ T-cells (**Fig. 4h**) in thawed TCPs produced IL-2 upon CMV-specific stimulation. CD4⁺ IL-2-producers were more frequent in FKBP12^{k.o.}-CMV-TCPs cultured in Tacrolimus, compared to the corresponding unedited CMV-TCPs (Fig. 4g). This observation of increased IL-2 producers in FKBP12^{k.o.}-CMV-TCPs cultured in Tacrolimus, compared to the corresponding unedited CMV-TCPs, was significant for CD8⁺ IL-2-producers **(****Fig. 4h****).** CD8⁺ IL-2-producers from FKBP12^{k.o.}-CMV-TCPs cultured in Tacrolimus were significantly more frequent compared to CD8⁺ IL-2 producers from FKBP12^{k.o.}-CMV-TCPs cultured in CsA or triple immunosuppression **(****Fig. 4h****).**

Next, we assessed the expression of the activation markers CD154 (CD40L) and CD137 (41BB) on CMV-stimulated TCPs. CD154 increases the activation of antigen presenting cells and is important for antibody production by B-cells³⁹. CD154 is also induced upon antigen recognition by the TCR⁴⁰, and is reported to be expressed on T-cells protective against CMV⁴¹. We found CMV-specific CD154 upregulation on CD4⁺ (**Fig. 4i**), but also on a subset of CD8⁺ T-cells (Fig. 4j). CD154-expressing CD4⁺ **(****Fig. 4i****)** and CD8⁺ T-cells **(****Fig. 4j****)** were more frequent among FKBP12^{k.o.}-CMV-TCPs cultured in Tacrolimus compared to their unmodified counterpart (**Fig. 4i****, j**). After culture in triple IS, CD154-expressing CD4⁺ T-cells increased in FKBP12^{k.o.}- compared to unmodified CMV-TCPs upon CMV-specific stimulation (**Fig. 4i**). After culture in CsA and triple IS, CMV-specific CD154 expression by CD8⁺ T -cells in FKBPI2^{k.o.}-CMV-TCPs was significantly reduced compared to Tacrolimus-treated cultures of FKBP12^{k.o.}-CMV-TCPs **(****Fig. 4j****).** CD137 (41BB) is another antigen-dependent activation marker on T-cells **(****Fig. 4k, l****).** Except for a significant reduction in CMV-specific CD137-expressing CD4⁺ T-cells from FKBP12^{k.o.}-CMV-TCPs upon culture in CsA compared to culture without immunosuppressants **(****Fig. 4k****),** we did not detect differences in CMV-specific CD137-expression on T-cells among CMV-TCPs, neither after culture in immunosuppressants nor upon FKBP12^{k.o.} **(****Fig. 4i****-k).**

Taken together, these results show that, after culture in Tacrolimus, FKBPI2^{k.o.}-CMV-TCPs mediate superior effector function and activation compared to unedited CMV-TCPs. The results thus indicate that FKBP12^{k.o.}-CMV-TCPs have acquired resistance to Tacrolimus.

### FKBP12^{k.o.}-CMV-TCPs have a similar TCRβ repertoire as CMV-TCPs after expansion

We performed TCRβ-sequencing to compare the clonality and number of clones included in the expanded FKBP12^{k.o.}- *vs.* CMV-TCPs. There was no similarity between the TCRβ repertoires of different donors defined by the Morisita index. However, we detected high similarity between FKBP12^{k.o.}- and CMV-TCPs generated from the same donor. We detected comparable clonality **(****Fig. 5a****),** percentage of all **TCRβ** sequences covered by the top 10 clones **(****Fig. 5b****)** and total numbers of clones represented **(****Fig. 5c****)** in FKBP12^{k.o.}- *vs.* CMV-TCPs. Hence, we conclude that gene editing did not drastically skew the TCRβ repertoire or induce excessive clonal expansion.

### Cas9 protein is undetectable in edited T-cell products after 5 days of culture

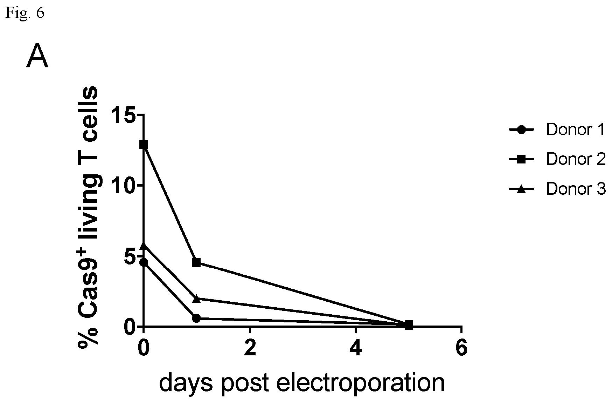

Studies on human T-cell reactivity towards the Cas9 protein^{42,43} may raise the concern that Cas9-edited cells are potentially immunogenic. Therefore, we stained edited T-cells for Cas9 protein after electroporation. We detected Cas9 protein expression in low proportions of T-cells, which progressively decreased over time. Cas9 protein was undetectable by day 5 post-electroporation (Fig. 6). Thus, T cells of the invention may or may not comprise Cas9 protein, depending on the time point after electroporation they are used.

### Differentiation states of the different T-cell products at different stages in production

The majority of the CD8⁺ T-cells have a terminally differentiated phenotype after the isolation of CMV-reactive T-cells (CCR7⁻ CD45RA⁺), and thus, may arise the concern that these TCPs have limited longevity **(****Fig. 1b****).** In fact, after expansion and thawing this population was almost undetectable in the majority of TCPs, allowing the assumption that less differentiated memory T-cells expanded and represent the mass of the expanded TCP (data not shown). Knockout of FKBP12 did not have a major impact on the T-cell memory subset composition of the TCPs and there was no clear trend towards a particular subset preference (data not shown).

### Discussion

The invention provides a GMP-compliant approach for targeted gene editing of specific T-cells to achieve Tacrolimus or CsA resistance. This approach can be used, e.g., to optimize antiviral T-cell therapy for use in a setting requiring immunosuppression, for example SOT. The method of the invention comprises an antigen-specific T-cell isolation step²⁷, which is superior in terms of safety to other published protocols that start with a mixed PBMC population^{26,28}. This safety step is important to exclude allograft-reactive IS-resistant T-cell contamination, that could cause damage to the transplanted organ. The invention provides a functional FKBP12^{k.o.}-CW-TCP with superior cytokine production and activation in the presence of Tacrolimus and triple IS (used in SOT) compared to unedited CMV-TCPs. The retained sensitivity of the gene-edited FKBP12k.o.-CMV-TCPs to CsA represents an important safety switch. CsA is a clinically approved drug that may be used to limit any toxicity possibly associated with the Tacrolimus-resistant FKBP12^{k.o.}-CMV-TCP in case it occurs. The method of the invention utilizes a cartridge-based GMP-compatible electroporation system and adapted electroporation conditions. Although gene knockouts using multiple sgRNAs are more efficient³⁰, immunophilin, namely, FKBP12, knockout using the single sgRNA of the invention is preferred to minimize potential off-target effects and to ensure safety and GMP-compatibility. Moreover, advantages of a GMP-compatible synthetic sgRNA with 2O'-methyl-3'phosphothioate-bonds³⁵ were shown. The synthetic sgRNA enhanced knockout efficiency and functionality of the product in the presence of Tacrolimus and triple IS. Recently, a similar process was reported for production of glucocorticoid-resistant antiviral T-cells^{44, 71}. All material used for the described process is either available at GMP-grade or has sufficient documentation and quality to be accepted by regulatory authorities for GMP production. This qualifies the whole process as a GMP-compliant ready to translate process.

We detected no impact of classical immunosuppressive drugs on cytotoxic killing capacity as previously reported^{45,46}. Cytotoxicity in the "VITAL" assay used for determination of killing capacity is mainly based on perforin/granzyme-mediated killing³². The transcription of perforin in T-cells is reported to be induced by IL-2 and IL-15, thus not exclusively dependent on calcineurin^{47,48.} Substantial release of granzymes has also been reported in the presence of Tacrolimus⁴⁹, which is in line with our findings for intracellularly captured GZB and increased GZB-producers among CD4⁺ T-cells upon viral stimulus in the presence of immunosuppressants.

Addition of CNIs decreased IFNγ-producers among CD4⁺ and CD8⁺ T-cells, yet IFNγ production was not completely abolished. This has been observed by others for CD4⁺memory T-cells at higher doses of IS than used here²¹. Indeed, it was reported that IFNγ can also be induced by a calcineurinindependent pathway in T-cells^{50,51}. This suggests continued IFNγ -production even in presence of CNIs. Additionally, some downstream signalling events of the TCR are unaffected by inhibition of calcineurin and may lead to IFNγ induction⁵². However, Tacrolimus significantly reduced the proportion of IFNγ -producers. Cytokine production in the presence of Tacrolimus was rescued after FKBP12^{k.o.}, even in presence of triple IS, although MPA and corticosteroids are reported to further downregulate TCR-mediated IFNγ-production^{22,24}. In contrast, TNFα-producers were much more sensitive to inhibition by calcineurin inhibitors. Nonetheless, FKBP12^{k.o.}-CMV-TCPs recovered their ability of TNFα production in the presence of Tacrolimus, confirming reports identifying calcineurin as the crucial inducer of TNFα5³. As previously reported⁵⁴, we found IL-2-production by T-cells to be highly sensitive to immunosuppressants. Our data revealed significant recovery of IL-2-producers in FKBPI2^{k.o.}-CMV-TCPs in the presence of Tacrolimus. Notably, IL-2 is primarily produced by less differentiated long-lived memory T-cells with high proliferative potential, which are a very attractive cell subset for antiviral T-cell therapy approaches³⁸. Polyfunctional IFNγ⁺ TNFα⁺IL-2⁺ CD4⁺ and CD8⁺ anti-viral T-cells were found to be protective against CMV disease after SOT⁵⁵. Our strategy for FKBP12^{k.o.} retained IFNy/TNFα-double-producers and IL-2-producers despite immunosuppression, hence, the FKBP12^{k.o.}-CMV-TCP is an attractive adoptive T-cell transfer approach for the induction of protective immunity *in vivo*⁵⁵. The activation marker CD154, which is associated with polyfunctionality⁵⁶ and protection from CMV after SOT⁴¹, was also highly sensitive to immunosuppressants. Of note, CMV-specific CD154 upregulation in the presence of Tacrolimus could be restored by FKBP12^{k.o.}, underpinning the high antiviral potential of FKBPI2^{k.o.}-CMV-TCPs in presence of Tacrolimus. CD154 is also expressed on a small subset of virus-specific CD8⁺ T-cells (here <10%), which may exert helper-like functions⁴⁰. We did not observe any effect of CNIs on the CMV-stimuli-induced upregulation of the activation marker CD137 (4-1BB) on the T-cells examined.

Glucocorticoid treatment is reported to transiently increase proportions of CD25⁺CD4⁺ T-cells⁵⁷. Indeed, following 24 h of incubation with triple IS including prednisolone, we recorded a transiently elevated expression of CD25 that progressively declined by day 4 of culture in triple IS. MPA was reported to downregulate CD25 expression on T-cells⁵⁸. This possibly counteracted the glucocorticoid-mediated upregulation, resulting in the decline observed by day 4 of culture in triple IS. We could not confirm data implying a glucocorticoid- or MPA-mediated upregulation of PD-1 on T-cells^{24,59}. MPA and dexamethasone have been suggested to upregulate expression of CTLA-4 on CD4⁺ T-cells^{24,60}. However, we observed significant attenuation of CTLA-4 after 4 days of culture in triple IS. CTLA-4 expression requires antigen stimulation⁶¹. We presume the single initial antigenic T-cell stimulation at day 0 was insufficient to sustain CTLA-4 expression.

Gene editing may raise concerns about transformation of the edited cells, which could lead to uncontrolled proliferation and eventually tumor formation. An increase in clonality of the TCPs could be an indicator of transformation, since certain transformed clones gain a growth advantage and are able to outgrow other clones. Thus, we performed TCR sequencing to exclude that there were any transformative events in the TCPs. We found that the FKBP12^{k.o.}-CMV-TCPs did not have higher clonality than their non-edited counterparts, implying the cells were not transformed. Furthermore, the percentage covered by the top 10 most frequent clones was also not increased, confirming that none of the clones had acquired proliferative advantages as a result of gene editing. Use of microbial compounds for genetic engineering and introduction of genetically modified cells into humans may elicit immune reactions⁶². We have demonstrated that FKBPI2^{k.o.}-CMV-specific T-cells degrade the Cas9 protein within 5 days post-electroporation. Thus, recombinant bacterial Cas9, which was reported to elicit adaptive immune responses^{42,43}, is unlikely to cause immune reactions in patients treated with the TCP described here. In general, the use of autologous cells bears a low risk of immunogenicity¹⁹. Nonetheless, potential mutations inserted during the repair of double strand breaks may result in immunogenicity of the edited cells and cause T-cell activation. The major concern when applying CRISPR-Cas9 technology for genetic engineering remains off-target gene-editing posing the risk of uncontrollable mutagenesis⁶³.

Within the initially sorted CMV-specific CD8⁺ T-cells, there was a high percentage of terminally differentiation T_{EMRA}. This could raise concerns about the efficacy, longevity and induction of functional memory by the resulting TCP. However, T_{EMRA} are strongly associated with CMV infection, which induces them at high levels⁶⁴. They were shown not to expand in polyclonally activated CAR-TCPs, when cultured either alone or in bulk cultures of different T-cell memory subsets⁶⁵. This is in line with our findings for the expanded and thawed TCPs, which are likely to result from proliferation of less differentiated memory T-cell subsets.

In summary, the invention provides a GMP-compatible protocol for the manufacture of Tacrolimus -resistant T cells, e.g., Tacrolimus-resistant CMV-specific TCPs, which are functionally superior to unmodified antiviral TCPs in the presence of calcineurin inhibitor Tacrolimus, and triple IS. Furthermore, they contain features of highly potent protective early memory T-cells as described herein. With an FKBP12^{KO} the sensitivity to CsA represents an inherent safety switch, which is exploitable in case the product should elicit adverse effects. Additionally, compared to previous approaches to generate CNI-resistant TCPs²⁶⁻²⁸, our product is vector free and utilizes targeted mutagenesis. As such, it provides a safer methodology for TCP generation, and a safer T cell product. This manufacturing process can be applied to generate TCPs directed against other viruses or tumors using the respective peptide pools for the initial sorting of T-cells with the desired specificity. The invention thus provides novel pharmaceutical compositions for use in human patients comprising the cells of the invention, e.g., the Tacrolimus-resistant anti-viral TCPs of the invention.

### Materials and Methods

### Blood sampling, isolation and culture of virus-specific T-cells

The study was approved by the Charité-Universitätsmedizin Berlin Ethics Committee and peripheral blood was obtained from healthy donors, who had given their written informed consent. PBMCs were isolated using Biocoll (Biochrom) gradient centrifugation. Virus-specific T-cells were isolated following a 6 h stimulation with overlapping CMV-specific phosphoprotein (pp)65 and immediate early (IE)-1 peptide pools (JPT Peptide Technologies; 0.5 µg/ml each) using an IFNγ Secretion Assay-Cell Enrichment and Detection Kit according to the manufacturer's instructions (Miltenyi Biotec) and cultured in complete media (VLE RPMI 1640 supplemented with penicillin (100 IU/ml) and streptomycin [all from Biochrom] and 10% fetal calf serum [FCS, PAA]), supplemented with 10 ng/ml recombinant human (rh)IL-7 and rhIL-15 (CellGenix) on 96- or 24-well plates, respectively, in humidified incubators at 37 °C and 5 % CO₂ as described previously^{13,14}. Cells were split 1:1 upon reaching 100 % confluency. After 21 days, TCPs were frozen in FCS with 10 % dimethyl sulfoxide (Sigma-Aldrich) at - 80°C and transferred to liquid nitrogen after 1 day. TCPs were thawed in a water bath at 37 °C and washed twice with complete medium. One half was analyzed immediately and the other half was seeded into 24-well plates and cultured in complete medium with or without immunosuppressive drugs at clinically relevant doses (6 ng/ml Tacrolimus [Prograf, Astellas]; 120 ng/ml CsA [Sandimmun, Novartis]; triple IS = 6 ng/ml Tacrolimus + 0.57 µg/ml Prednisolone [Urbason solubile, Sanofi] + 2.7 µg/ml mycophenolic acid [MPA, active substance of mycophenolate mofetil, Sigma-Aldrich]) until further analysis.

### Knockout procedure

FKBP12-specific sgRNAs (Table 1) were *in vitro* transcribed from DNA templates as previously described⁶⁶, using Hi-Scribe T7 High Yield RNA Synthesis Kit (New England Biosciences). Where indicated, synthetic modified sgRNA (Synthego Corporation) with 2O'-methyl-3'phos-phothioate modifications between the first and last 3 nucleotides was used.

2.5 million antiviral T-cells were electroporated at day 7 after isolation using Amaxa P3 primary cell 4D-Nucleofector X Kit L and the Amaxa-Nucleofector-4D (Lonza, program CO-115) to transfer ribonucleoprotein complexes of 30 µg of recombinant Alt-R *Streptococcus pyogenes* Cas9 protein V3 (Integrated DNA Technologies) precomplexed with 30 µg of in-vitro-transcribed or 15 synthetic sgRNA (Synthego Corporation). The same number of unmodified antiviral T-cells and antiviral T-cells electroporated without additives were expanded as controls.

### Phenotypic and functional assays assessed by flow cytometry

For assessment of CMV-specific cytokine production/activation, lymphoblastic B-cell lines (LCLs) were generated as described previously⁶⁷ and used as antigen presenting cells at a 1:10 ratio for a 6 h CMV-specific stimulation with 0.5 µg/ml CMV-specific peptide pools (IE-1 and pp-65 each) in the presence or absence of immunosuppressants at clinical doses indicated above. Unstimulated controls included LCLs without CMV-peptides. Intracellular cytokine production was captured by addition of 2 µg/ml of Brefeldin A (Sigma-Aldrich) after 1 h of stimulation and cells were stained using antibodies (all from BioLegend, unless stated otherwise) and the FoxP3/Transcription Factor Staining Buffer Set (eBioscience). Staining was performed using fluorophore-conjugated human anti-CD3 (OKT3), -CD4 (SK3), -CD8 (RPA-T8), -IFNγ (4S.B3, eBioscience), -TNFα (MAb11), -IL-2 (MQ1-17H12), -CD137 (4B4-1), -CD154 (24-31) and -Granzyme B (GZB; GB11; BD Pharmingen) antibodies. LIVE/DEAD Fixable Blue Dead Cell Stain (L/D; Invitrogen) was used to identify living cells, which was also used for determination of viability in combination with fluorescently labelled Annexin V (BioLegend).

A "VITAL" assay was performed to assess killing capacity of TCPs ^{14,32,37}. Briefly, cells from TCPs were incubated at distinct ratios with CMV-peptide-loaded autologous LCLs stained with 10 µM Carboxyfluorescein Diacetate Succinimidyl Ester (CFSE-DA; Sigma-Aldrich) for 4 min and unloaded allogenic LCLs stained with 5 µM CellTrace^{™} Far Red (Invitrogen) for 10 min. T-cell-free LCL mixtures served as internal controls to calculate the CMV-specific killing capacity³². After 14 h of incubation, co-cultures were stained with L/D.

All flow cytometry samples were analyzed using an LSR-II-Fortessa flow cytometer (Becton Dickinson) and FlowJo-10 software (Tree Star).

### Efficiency analysis

Analysis of on-target editing was performed from isolated DNA (Zymo Research) of day 21 cell samples. The FKBP12 locus was amplified using KAPA HiFi HotStart ReadyMix (Roche) and the following primer pairs: TCTGACGGGTCAGATAACACCTAG (F, SEQ ID NO: 8) and TCTTCCGGAGGCCTGGGTTT (R, SEQ ID NO: 9) for sgRNA#1&2; ACAGCTGTATCCG-GAGGCCT (F', SEQ ID NO: 10) and TCACAGCCGCCGATTCAGAC (R', SEQ ID NO: 11) for sgRNA#3 (Eurofins Scientific SE) with the following touchdown-PCR program in an automated thermocycler: 1. 95 °C, 3 minutes, 2. 98 °C, 30 seconds 3. 72-64 °C for sgRNA#1&2/68-64 °C for sgRNA#3, 15 seconds (-0.5 °C for each cycle starting at the highest until the lowest temperature was reached; 20 cycles, 64 °C); 4. 72 °C, 15 seconds; 5. repeat from step 2 with decreasing annealing temperature (as specified); 6. 72 °C, 1 minute; 7. 4 °C. PCR products were purified using DNA purification & enrichment kit (Zymo Research) prior Sanger sequencing with primer F/F' by LGC Genomics GmbH. Editing frequencies were calculated using the Inference of CRISPR Edits (ICE) algorithm (Synthego Corporation)⁶⁸.

### T-cell receptor-β sequencing

T-cell receptor-β (TCRβ) sequencing was performed from genomic DNA by Adaptive Biotechnologies and analyzed using ImmunoSEQ ANALYZER 3.0 (Adaptive Biotechnologies). The data are available at the immuneACCESS platform.

### Off-target identification and validation

The major concern when applying CRISPR-Cas9 technology for genetic engineering remains off-target gene-editing posing the risk of uncontrollable mutagenesis⁶³. To evaluate potential off-target editing, two different approaches were performed to identify potential off-target sites: Firstly, we used the CRISPR Off-target Sites with Mismatches, Insertions, and Deletions (COSMID) online tool to identify potential off-target sites *in silico³³.* assay using donor DNA mixed with Cas9 protein and the specific single guide RNA followed by next generation sequencing to identify sites cut *in vitro,* which they term Abnoba-Seq (WO2021078645). Sites identified with these two techniques were validated from DNA extracted from our CMV-specific FKBP12^{k.o.-}TCPs by next generation amplicon sequencing and showed no preferential off-target editing, whereas editing of the target gene could be confirmed (Figure 11).

### Comparative Example 2 - cyclophilin A^{k.o} T cells

Peripheral blood mononuclear cells were freshly isolated from healthy human donors and stimulated with plate-bound anti-CD3/anti-CD28 antibodies in the presence of cytokine containing medium (RPMI 1640 medium [Biochrom], 10% heat-inactivated fetal calf serum [Biochrom], 10 ng/ml IL-7 [Cellgenix], 5 ng/ml IL-15 [Cellgenix]). Two days after stimulation, activated T cells were harvested and electroporated with sgRNA-Cas9 complexes using the Neon Electroporation System (Invitrogen). PPIA specific sgRNAs of SEQ ID NO: 4-7 and control CD45 targeting sgRNA as previously described were used³⁰. Prior to electroporation, sgRNA were incubated with recombinant Cas9 protein (PNAbio) for 15 minutes at room temperature and electroporation was performed as previously described³⁰. Four days after electroporation, genomic DNA was extracted of 200.000 T cells. The PPIA locus was amplified using polymerase chain reaction (Forward primer: 5'- ACTCGCGGACCTCCCAAAATG-3' (SEQ ID NO: 12); Reverse primer: 5'- AC-GCGCCTCATCGCTTGACA-3' (SEQ ID NO: 13)). KO of PPIA was confirmed in an agarose gel (Fig. 8). Seven days after electroporation, T cells were restimulated with PMA/Ionomycin for 6 hours in medium alone or in medium supplemented with either Cyclosporine A (therapeutic dose, 120 ng/ml [Sandimmun, Novartis]) or Tacrolimus (therapeutic dose, 6 ng/ml; [Prograf, Astellas]). One hour after beginning of stimulation, Brefeldin A was added to prevent cytokine secretion and allow for intracellular staining of cytokines with fluorochrome-labeled antibodies. Subsequent flow cytometry analysis allowed evaluation of cytokine production in alive T cells of different subsets (e.g. CD4+ and CD8+). PPIA^{KO} T cells partially retained their cytokine production potential in the presence of Cyclosporine A, which was more pronounced in CD4⁺ compared to CD8⁺ T cells (Fig. 9). The PPIA^{KO} T cells remained sensitive to Tacrolimus (Fig. 9).

### Example 3 - FKBP12^{k.o.} regulatory T cells

Regulatory T cells were isolated from peripheral blood mononuclear cells of healthy human adults based on the expression of CD4 and high expression of CD25 and low expression of CD127 using a GMP grade fluorescently activated cell sorting machine (Tyto, Miltenyi) or a combination of magnetic and fluorescently activated cell sorting. Isolated Treg were subsequently stimulated with anti-CD3/anti-CD28 coated expansion beads in the presence of IL-2 (500 IU/ml) and rapamycin. Seven days after initial activation and expansion, Treg were electroporated with CRISPR-Cas9 ribonucleoprotein complexes comprising synthetic sgRNA FKBP12 (SEQ ID NO: 1) and recombinant Cas9 protein using the cartridge based Nucleofector 4D (Lonza). After further expansion using Treg expansion beads, IL-2 and Rapamycin for 21 days, the cells were analysed and compared with untouched Tregs regarding expansion rate and final yield (Fig. 10 A+B), gene editing efficacy using either classical Staphylococcus pyogenes-derived Cas9 or a high fidelity version measured by Sanger sequencing and ICE analysis (Synthego) (Fig. 10 C). FKBP12-KO could be performed with high efficiency and even increased overall yield, presumably due to reduced inhibition by rapamycin treatment. Epigenetic stability was assessed by quantification of demethylation within the Treg-specific demethylation region (TSDR) (Fig. 10 D). FKBP12-KO Treg retained epigenetic identity of natural Treg as indicated by high TSDR demethylation in contrast to effector T cells expanded under the same conditions (Fig. 10 D).

Fig. 11 shows regulatory T cells, expanded and treated as described in Fig. 10. FKBP12^{KO} and control Treg cells were subsequently expanded in medium with or without different calcineurin inhibitors and subsequently analysed for cell expansion (Fig. 11 A) and with flow cytometry (Fig. 11 B+C). As expected, Tacrolimus treatment reduced the proliferation of untouched Tregs, while FKBP12-KO Tregs retained fully proliferative capacity despite the drug treatment (Fig. 11 A). FKBP12^{KO} Treg displayed higher expression of the inhibitory receptor CTLA4 (Fig. 11 B). CTLA4 is known to suppress T cells and modulate pro-inflammatory responses by dendritic cells. Further, Foxp3 transcription factor expression assessed by flow cytometry was comparable between wildtype and FKBP12-KO, with a trend toward increased Foxp3 expression of FKBP12KO-Treg in the presence of Tacrolimus indicating potential for increased stability (Fig. 11 C).

To ensure safety and exclude chromosomal aberrations caused by genetic editing, we evaluated the karyotype of FKBP12^{k.o.} Treg products, which did not show any abnormalities. Furthermore, we assessed the clonal composition of the Treg products by TCR sequencing to assess putative clonal outgrowth of transformed T cells, which did not reveal major differences in the diversity or percentage of total TCR sequences covered by the top 10 clones between FKBP12^{k.o.} or untouched control TCPs (Fig. 12 A-B). However, the total amount of clones was significantly reduced in FKBP12^{k.o.} compared to untouched control TCPs, probably because FKBP12^{k.o.} T cells overgrew non FKBP12^{k.o.} T cells due to the proliferative advantage in presence of Rapamycin (Fig. 12 C). We performed validation of predicted off-target sites as described above for the virus-specific T cells. Sites identified with the described two techniques were validated from DNA extracted from out Treg products by amplicon sequencing and showed no preferential off-target editing, whereas editing of the target gene could be confirmed (Fig. 12 D).

Moreover, we analysed whether FKBP12 editing impacted the global DNA methylome of Treg products using an EPIC array, which was not the case. Factors like donor origin had more substantial effects on the DNA methylome.

In addition, we evaluated the proteome of the FKBP12^{k.o.} and untouched control Treg products in presence of CNIs using two different approaches, the DigiWest approach and a mass spectrometry-based approach. Interestingly, based on the data generated in the DigiWest approach, Tacrolimusexposed FKBP12^{k.o.} Treg products clustered with medium-exposed FKBP12^{k.o.} and untouched control Treg products, whereas negative controls were completely distinct from Treg products (Fig. 12 E). If FKBP12^{k.o.} Treg products were exposed to Cyclosporine A or triple immunosuppression, they clustered close to the corresponding untouched control Treg product (Fig. 12 E). Proteins, which were maintained in Tacrolimus exposed samples of FKBP12^{k.o.} Treg products, but less expressed in the corresponding untouched control samples were: CTLA-4, Arginase-2, GBP-2, LAG-3, phospho-Erk and PD-1. In contrast, the stress fiber protein Zyxin was expressed at higher levels in untouched control Treg products exposed to Tacrolimus compared to Tacrolimus exposed samples of FKBP12k.o. Treg products. The mass spectrometry-based approach confirmed FKBP12^{k.o.} at the protein level and showed beneficial upregulation of Arginase-2 and Legumain in FKBP12^{k.o.} Treg products compared to untouched control Treg products irrespective of the treatment. In presence of Tacrolimus, FKBP12k.o. Treg products showed higher protein expression of GBP-2 and lower protein expression of Zyxin, confirming the data from the DigiWest analysis. The proteomics data imply a benefit of the FKBP12^{k.o.} at protein level.

Furthermore, we investigated the transcriptome of FKBP12^{k.o.} and untouched control Treg products using single cell Cellular Indexing of Transcriptomes and Epitopes (CITE) sequencing (seq). Clustering based on transcriptomes and selected surface proteins revealed 13 cell clusters (Fig. 13A). FKBP12^{k.o.} Treg products showed cluster distribution similar to untouched control Treg products, which differed from expanded non-Tregs especially upon activation at d21 (Fig. 13B). After 7-day exposure to CNIs, FKBP12^{k.o.} and untouched control Treg products showed similar cluster distributions in an unstimulated state, however, after stimulation a clear influence of CNIs on cluster distribution was observed, except for the FKBP12^{k.o.} Treg products exposed to Tacrolimus, which showed the same cluster distribution as samples not exposed to CNIs, confirming the resistance to this immunosuppressant (Fig. 13C). The sensitivity of FKBP12^{k.o.} Treg products to Cyclosporine A (Fig. 13C) displays an inherent safety switch by switching patients from Tacrolimus to Cyclosporine A in case any severe complications should be caused the gene edited Treg product.

### Example 4 - Abating immunopathology by combined use of Tacrolimus and Tacrolimus-resistant SARS-CoV-2-specific T-cells

Severe acute respiratory syndrome coronavirus 2 (SARS-CoV-2) infection frequently leads to coronavirus disease 2019 (COVID-19). Recent studies suggest severe COVID-19 results from an overshooting immune response, associated with ineffective viral clearance despite unspecific T-cell activation. As corticosteroid-based immunosuppression is controversial dependent on the stage of infection, Tacrolimus is being investigated as an alternative. On the other hand, systemic immunosuppression can prevent immunopathology but may prolong disease course by diminishing antiviral immunity, a risk particularly in transplant patients. To achieve protection by Tacrolimus without impacting antiviral defence, we generated SARS-Cov-2-specific Tacrolimus-resistant T-cell products (TCPs) from the blood of asymptomatic or mildly affected SARS-CoV-2 convalescent donors, which can also be applied to immunosuppressed patients such as transplant recipients or autoimmune patients who are known to show suboptimal vaccine responses. We functionally and phenotypically characterized these products, including single cell CITE- and TCR sequencing analyses. Our approach allows the adoptive transfer of functional SARS-Cov-2-specific memory T cells in presence of Tacrolimus for preventing of either undesired alloreactivity or even overshooting nonspecific endogenous T-cell activation in non-transplant patients.

### Introduction

The novel pathogen, severe acute respiratory syndrome coronavirus 2 (SARS-CoV-2) emerged in 2019, causing respiratory tract disorders, referred to as coronavirus disease 2019 (COVID-19) and leading to a worldwide pandemic. SARS-CoV-2 infects host-cells *via* viral spike protein binding specifically to the cellular entry receptor angiotensin-converting enzyme 2 (ACE-2).⁶⁹

While most SARS-CoV-2 infections show a mild course, severe COVID-19 is associated with extrapulmonary systemic hyperinflammation syndrome characterized by an overshooting innate and adaptive immune response (sometimes referred to as "cytokine storm") that further results in tissue damage and multi-organ failure⁷⁰. Studies indicate dysregulated T-cell function with increased expression of inflammatory genes and co-expression of activation markers contributes to COVID-19-associated hyperinflammation and impaired viral clearance.⁷¹⁻⁷³ Moreover, peripheral CD4⁺ and CD8⁺ T-cells of patients suffering from severe COVID-19 exhibit a transcriptional signature associated with T-cell exhaustion, which can potentially be linked to T-cell dysfunction and could be driven by overactivation.⁷³ Published data suggest this phenomenon is a result of bystander activation, not antigen-specific activation⁷⁴, which may partly explain why critical outcomes are disproportionately more frequent in elderly patients. Aging is associated with loss of naive and early memory T-cells in the peripheral blood and a relative expansion of late-differentiated memory T-cells, which are more susceptible to bystander activation.⁷⁵ Therefore, COVID-19-associated hyperinflammation might be augmented by non-specific endogenous T-cell activation contributing to immunopathology in elderly patients.

Corticosteroids are currently the first line of treatment for patients with severe COVID-19-associated hyper-inflammation and reduce mortality in patients requiring respiratory support⁷⁶. However, their generalized use in treating coronavirus diseases has been controversial. Therefore, it remains unclear whether all COVID-19 patients benefit from corticosteroid treatment. Surprisingly, while infections with SARS-CoV-2 were reported to be more common in transplant recipients, COVID-19 associated mortality is not increased among this patient group⁷⁷. Moreover, case reports and a European study suggest protective effects of Tac in patients infected with MERS-CoV or SARS-CoV-2 respectively⁷⁸⁻⁸⁰ after solid organ transplantation. Thus, Tac may be an attractive alternative to corticosteroids, which is reported to inhibit both proinflammatory cytokine production and the replication of human coronavirus SARS-CoV-1, HCoV-NL63 and HCoV-229E^{77,81} and be able to mitigate viral replication in COVID-19 patients in addition to reducing T-cell-associated hyperinflammation. A Spanish group is currently investigating Prednisolone (Pred) pulses combined with Tac as a novel therapeutic treatment strategy for hospitalized COVID-19 patients in a phase II clinical trial (ClinicalTrials.gov Identifier: NCT04341038) and also cyclosporine A is investigated as first line therapy for COVID-19-associated pneumonia⁸².

Multiple studies detected SARS-CoV-2-specific T-cells in convalescent patients indicating their important role in viral clearance and the development of protective immunity. Therefore, SARS-CoV2-specific adoptive T-cell therapy (ACT) has been suggested as an early treatment or preventive strategy for COVID-19 in immunocompromised or immunosuppressed individuals or for treatment of acute COVID-19^{83,84}. Indeed ACT has been used with a very low incidence of adverse effects to prevent and treat infections in patients after hematopoietic stem cell transplantation and solid organ transplantation, including Epstein-Barr-Virus (EBV)- and Cytomegalovirus (CMV)-associated diseases. ^{85,86} Recent studies have shown the feasibility of generating SARS-CoV-2-specific T-cell products from the blood of patients who have recovered from SARS-CoV-2 infection. ^{83,84} However, clinical trials are needed to demonstrate safety, efficiency, persistence and longevity of antiviral T-cell products (TCPs) *in vivo* to determine the therapeutic benefit of ACT for COVID-19. Severe COVID-19 patients being treated with immunosuppressants may not benefit from adoptive antiviral T-cell therapy, because, although immunosuppression may prevent overshooting immune responses, endogenous antiviral immunity is also suppressed. These patients, including transplant recipients and autoimmunity patients, may benefit from adoptive transfer of Tac-resistant SARS-CoV2-specific TCPs. Both T- and B-cell responses are considered necessary for protective antiviral immunity, leading to establishment of cellular and humoral immunity. Reported data imply adoptive T-cell therapy enhances induction of antibody responses,⁸⁷ thus Tac-resistant adoptive SARS-CoV-2-specific T-cell therapy may help to establish protective immunity in immunosuppressed patients, who often fail to mount protective long-term antibody responses after vaccination^{88,89}.

Furthermore, there is an urgent need for effective and safe strategies aimed at supporting viral clearance whilst preventing SARS-CoV-2-associated hyperinflammation and tissue damage in severe COVID-19 patients in general. Thus, we suggest combination therapy for severe COVID-19 using Tacrolimus to prevent immunopathology in combination with Tacrolimus-resistant adoptive antiviral T-cell therapy to improve viral control.

We report the feasibility of generating SARS-CoV-2-specific Tac-resistant antiviral T-cells suitable for ACT from re-convalescent SARS-CoV-2 infected patients utilizing our vector-free gene-editing approach targeting the FKBP12 gene coding for the adapter protein required for the immunosuppressive function of Tac in antiviral T-cells.³¹ Functional analysis confirmed that FKBP12^{KO} SARS-CoV-2-specific T-cells show a high degree of resistance to Tac treatment while maintaining effector function as measured by antigen-specific cytokine production. In contrast, the antiviral cytokine production of FKBP12^{KO} SARS-CoV-2-specific T-cells was efficiently suppressed by FKBP12-independent immunosuppression via calcineurin inhibitor (CNI) Cyclosporine A (CsA). We thus provide an inherent safety switch against potential adverse effects of FKBP12^{KO} T-cells *in vivo.* The GMP-compatible manufacturing process allows for clinical grade production of these T-cell products as a pre-requisite for a first in human clinical trial investigating the suggested combination therapy of Tac and SARS-CoV-2-specific FKBP12^{KO} T-cells. This ACT strategy also allows to continue treating transplant recipients or autoimmunity patients, who also have COVID-19, with the critical Tacrolimus therapy in parallel with antiviral treatment.

### Results

### SARS-CoV-2-specific T-cells are found in blood of SARS-CoV-2⁺ convalescent donors

To assess the feasibility of isolating SARS-CoV-2-reactive T-cells we analyzed the antiviral T-cell responses to SARS-Cov-2 structural and accessory proteins in individuals with a history of asymptomatic or mild SARS-CoV-2 infection (convalescent donors). Thus, we obtained peripheral blood mononuclear cells (PBMCs) from 20 donors who had cleared an asymptomatic or mild SARS-CoV-2 infection, and 19 healthy SARS-CoV-2 naive control donors. To exclude previous SARS-CoV-2 infection in healthy donors, we assessed humoral immunity in sera of all donors by ELISAs detecting Spike S1 IgG and IgA. In contrast to convalescent donors, all healthy donors were seronegative for Spike S1-specific IgG and IgA antibodies. The SARS-CoV-2-specific T-cell responses were evaluated by stimulating PBMCs with overlapping peptide pools (15-mers, 11aa overlap), encompassing the amino acid sequence of structural proteins (NCAP, Spike S1 + S2, VEMP, VME1) and accessory proteins (AP3a, NS6, NS7a, NS7b, NS8, ORF9b, ORF10, Y14) of SARS-CoV-2. Cells were stimulated for 16 h to analyze the reactivity of T-cells by flow cytometry using a set of markers for T-cell activation and effector cytokine production. In all the SARS-CoV-2 convalescent donors we observed upregulation of CD137 (4-1BB) and production of either IFN-y, TNF-α or both cytokines consistent with an effector T-cell activation following SARS-CoV-2-specific stimulation.

### Vector-free CRISPR/Cas9-based FKBP12^{KO} in SARS-CoV-2-specific T-cell products generated from convalescent donors.

The vector-free protocol for electroporation and ribonucleoprotein (RNP)-based KO of *FKBP12* described herein was used to generate Tacrolimus-resistant SARS-CoV-2-specific Tacrolimus-resistant T-cell products (TCPs). We isolated high purity SARS-CoV-2-specific T-cells from PBMC based on their IFN-y secretion after 6 h stimulation with SARS-CoV-2 peptide pools (NCAP, Spike S1 + S2, VEMP, VME1, AP3a, NS6, NS7a, NS7b, NS8, ORF9b; ORF10, Y14) **(****Fig. 14****).** These SARS-CoV-2-specific T-cells were cultured, expanded and split on day 7. One half of the culture was subsequently electroporated with RNP complexes of Cas9 and a single guide (sg)RNA, the other half served as the unmodified control. The unmodified and *FKBP12*^{KO} SARS-CoV-2-specific T-cells were expanded for two more weeks. The expansion rates and cell yields were similar for both fractions at days 14 and 21, illustrated in **Figures 15A and 15D****.** Similarly, as shown in **Figures 15B and 15E** the expansion rates and total counts of CD4⁺ and CD8⁺ T-cells of the TCPs at day 21 were comparable between unmodified and FKPB12^{KO} fractions. Although on day 0 we found the CD4⁺/CD8⁺ ratios were high among the SARS-CoV-2-reactive T-cells, these gradually became more balanced during expansion in both unmodified and *FKBP12*^{KO} TCPs **(****Fig. 15F****).** On day 21, the KO efficiency of *FKBP12* ranged from 63 to 89 % in the SARS-CoV-2-specific TCPs, as assessed by peak-shift analysis after sanger sequencing **(****Fig. 15C****).**

### FKBP12^{k.o.} SARS-CoV-2-specific TCPs do not differ in differentiation state from unmodified control SARS-CoV-2-specific TCPs

We next evaluated the cell surface expression of T-cell differentiation markers of expanded unmodified control and *FKBP12*^{KO} TCPs after culture on day 21. Overall, the *FKBP12* KO did not have a major effect on T-cell differentiation, the subset composition of the TCPs nor did it confer a discernible advantage to any particular subset (data not shown).

### Effector cytokine production in the presence of tacrolimus is rescued by FKBP12 KO in SARS-CoV-2-specific T-cell products

To show both efficacy against SARS-Cov2 and to confirm Tacrolimus-resistance of our *FKBP12* KO, we re-stimulated the distinct TCPs with SARS-CoV-2 peptide pools and analyzed production of antiviral cytokines (IFN-y, TNF-α and IL-2) in the presence or absence of clinical doses of immunosuppressive drugs. To confirm the specificity of the *FKBP12* KO approach, we re-stimulated unmodified control and *FKBP12*^{KO} TCPs in presence of Tac as well as an alternative CNI, CsA, which depends on peptidylprolyl isomerase A (PPIA) as its adaptor protein. Thus, *FKBP12* KO should not affect the immunosuppressive function of CsA in edited TCPs. We also tested the functionality of SARS-CoV-2-specific T-cells in the unmodified and *FKBP12*^{KO} TCPs by exposure to triple immunosuppression therapy commonly administered *post* solid organ transplantation, namely, Tac, Prednisolone (glucocorticosteroid), and Mycophenolic acid (MPA). Upon SARS-CoV-2-specific re-stimulation on day 21 of culture, unmodified control and *FKBP12*^{KO} TCPs showed comparable frequencies of activated (CD137⁺) cytokine producers among CD4⁺ and CD8⁺ T-cells **(****Fig. 16A-F****).** Exposing TCPs to immunosuppressive drugs during stimulation resulted in a significant decrease in activated cytokine producers among CD4⁺ **(****Fig. 16A-C****)** and CD8⁺ T-cells **(****Fig. 16 D-F****).** These were partially rescued by the *FKBP12*^{KO} **(****Fig. 16A-F****).** Both CD4⁺ and CD8⁺ *FKBP12*^{KO} T-cells produced effector cytokines in the presence of Tac but not in the presence of CsA **(****Fig. 16A-F****).** The proportions of cytokine producers among CD4⁺ and CD8⁺ T-cells in *FKBP12^{KO}* TCPs were similar in Tac treated and untreated TCPs. Exposure to triple IS during stimulation decreased the capacity to produce effector cytokines among both the SARS-CoV-2 stimulated CD4⁺ and CD8⁺ *FKBP12*^{KO} T-cells **(****Fig. 16A-F****).** Among the CD4⁺ and CD8⁺ T-cells of unmodified control and *FKBP12^{KO}* TCPs, we identified polyfunctional T-cells based on their ability to secrete multiple cytokines including IFN-γ, TNF-α and IL-2 **(****Fig. 16C, F****).** Unlike CD4⁺ T-cells, the frequency of activated SARS-CoV-2-specific CD8⁺ T-cells, identified by CD137 expression, did not significantly decrease when TCPs were exposed to CsA during stimulation (data not shown). However, both CD4⁺ and CD8⁺ CD137-expressing T-cells were unable to produce effector cytokines in presence of CsA, both in unmodified and *FKBP12*^{KO} TCPs (Fig. 16A-F).

### FKBP12 KO SARS-CoV-2-specific TCPs demonstrate a killing capacity comparable with unmodified control SARS-CoV-2-specific TCPs

Since viral antigen-targeted elimination of virus-infected cells is an essential characteristic of antiviral T-cells, we tested the cytotoxic killing capacity of SARS-CoV-2-specific TCPs. Although, as shown above, short-term incubation with the IS Tac showed a strong effect on antiviral cytokine production in TCPs, we found that the T-cell-mediated cytotoxic killing of target cells loaded with SARS-CoV-2 peptides was not affected by short-term treatment with Tac either in unmodified or *FKBP12*^{KO} TCPs **(****Fig. 17****).**

To identify the dominant antigens driving T-cell-mediated killing of SARS-CoV-2 peptide loaded target cells, we analyzed the killing capacity of TCPs with regard to individual antigens of SARS-CoV-2. Both unmodified control and *FKBP12*^{KO} TCPs showed efficient killing of NCAP as well as AP3a loaded target cells, followed by target cells loaded with VME1 and Spike S1 and S2. We also found T-cell-mediated killing toward the accessory proteins NS7a and ORF9b for some of the TCPs but not towards the remaining accessory proteins NS6, NS7b, NS8, ORF10 and Y14 nor the structural protein VEMP (data not shown). This was in contrast to antiviral cytokine production, for which we did not observe notable antigen-reactivity toward accessory proteins in the expanded unmodified control and *FKBP12*^{KO} TCPs with the exception of AP3a. Therefore, in addition we examined the killing capacity of CD4⁺ and CD8⁺ T-cells separately to determine whether T-cell-mediated cytotoxic killing of the dominant target antigens occurs due to CD4⁺ or CD8⁺ T-cells. The observation suggested CD8⁺ T-cells were the main drivers of cytotoxic elimination of SARS-CoV-2 peptide loaded target cells (data not shown). Among CD8⁺ T-cells of unmodified control and *FKBP12*^{KO} TCPs, we detected the most efficient killing of NCAP, AP3a and SARS-CoV-2 peptide pool loaded target cells, followed by target cells loaded with VME1, Spike S1 and Spike S2 peptide pools (data not shown).

### Cellular indexing of transcriptomes and epitopes sequencing analysis confirms resistance of FKBP12^{KO} TCPs to Tacrolimus

To determine whether *FKBP12* editing affects the transcriptome and specific surface protein levels and how these are influenced by different CNIs, we performed single cell Cellular Indexing of Transcriptomes and Epitopes sequencing (CITE-seq) on unmodified control and *FKBP12*^{KO} TCPs. SARS-CoV-2-specific unmodified control and *FKBP12*^{KO} TCPs were re-stimulated with SARS-CoV-2 peptide pool loaded target cells for 6 h on day 21, either in the presence or absence of CNI. According to their transcriptomes and specific protein expression identified by CITE-seq, cells were clustered by Uniform Manifold Approximation and Projection for Dimension Reduction (UMAP). 20 distinct cell clusters were identified, **(****Figure 18A****).** Among both CD4⁺ and CD8⁺ T-cells, CNI treatment as well as *FKBP12*^{KO} had no impact on the cluster distribution in unstimulated TCPs. In contrast, upon SARS-CoV-2-specific re-stimulation the frequencies of some T-cell clusters in both unmodified control and *FKBP12*^{KO} TCPs increased. The frequency change of some T-cell clusters was inhibited upon treatment with Tac in the unmodified control but not in *FKBP12*^{KO} TCPs **(****Figure 18B****).** However, in the presence of CsA, these clusters were under-represented in both unmodified control and *FKBP12*^{KO} TCPs **(****Figure 18B****).** Gene expression analysis confirmed downregulation of *FKBP12* mRNA in CD4⁺ and CD8⁺ T-cells of *FKBP12*^{KO} TCPs. Gene expression analysis also showed that upon SARS-CoV-2-specific stimulation the top 25 differentially expressed genes were similar between unmodified and *FKBP12*^{KO} TCPs in the absence of CNI as well as under Tac treatment in the *FKBP12*^{KO} TCPs. This was the case for both CD4⁺ and CD8⁺ T-cell populations. Remarkably, the presence of Tac further upregulated *SLA* mRNA in CD4⁺ and *IL2* mRNA in CD8⁺ *FKBP12*^{KO} T-cells respectively, compared to untreated *FKBP12*^{KO} and unmodified controls.

### The TCR repertoire is comparable between unmodified control and FKBP12^{KO} TCPs

We additionally performed TCR repertoire analysis on single cell level to determine the effect of *FKBP12* editing on TCR diversity. Comparison of the TCR clonality and total number of clones included in SARS-CoV-2-specific unmodified control and *FKBP12*^{KO} TCPs revealed higher variations within largely expanded but low variations within single, small and medium expanded clone types **(****Figure 18C****).** Importantly, there was no overrepresentation of largely expanded TCR clones in the *FKBP12*^{KO} TCPs **(****Figure 18C****).** The TCR diversity represented by Shannon entropy was largely comparable between unmodified control and *FKBP12*^{KO} TCPs **(Figure 18D).** the distribution of the five most represented TCR clones within CD4⁺ and CD8⁺ T-cells of unmodified control and *FKBP12*^{KO} TCPs revealed a shared TCR repertoire with overall comparable representation of TCR clones between unmodified and *FKBP12*^{KO} TCPs of one donor (data not shown). The proportion of all TCR sequences represented by the top 5 clones ranged from around 5 to maximum 32 %, but was comparable between *FKBP12*^{KO} and unmodified TCPs (data not shown).

### Discussion

ACT is an attractive treatment strategy to prevent and treat viral infections in immunocompromised or immunosuppressed patients and has been suggested as an early treatment strategy for SARS-CoV-2 infection or even to treat acute COVID-19⁸²⁻⁸⁴. Corticosteroid treatment currently remains the first choice to treat severe COVID-19. However, the CNI Tac which has antiviral properties^{77,79,81} is an attractive alternative IS to not only reduce COVID-19-associated hyperinflammation but also mitigate viral replication in COVID-19 patients. A combination therapy of Pred and Tac is currently under clinical investigation to treat hospitalized COVID-19 patients with respiratory failure (ClinicalTrials.gov Identifier: NCT04341038). Nevertheless, immunosuppressants likely suppress endogenous antiviral immunity and could undermine the benefits of adoptive antiviral T-cell therapy. Here, we demonstrate the feasibility of generating Tac-resistant SARS-CoV-2-specific TCPs, from the blood of donors who have cleared SARS-CoV-2 infection, that show superior cytokine production when exposed to clinical doses of Tac or triple IS compared to unedited TCPs. These may be applied for prevention or acute treatment of COVID-19 and enhancement of vaccination in immunosuppressed patients on Tac therapy (e.g. transplant recipients and autoimmunity). Furthermore, we suggest combination therapy for severe COVID-19 using Tac in combination with adoptive transfer of Tac-resistant antiviral T-cells to prevent immunopathology and to achieve viral control.

Upon SARS-CoV-2-specific stimulation of *ex vivo* PBMCs from convalescent SARS-CoV-2⁺ donors, we detected antigen-specific CD4⁺ and CD8⁺ T-cells. While CD8⁺ T-cells are incremental for elimination of infected cells, CD4⁺ T-cells contribute to affinity-maturated and protective antibody responses. Indeed, it was shown that spike-specific CD4⁺ T-cell responses correlate with serum levels of anti-spike IgG titers in recovered SARS-CoV-2 infected donors.⁹⁰ Patients that experienced mild symptoms following SARS-CoV-2 infection show higher proportions of CD8⁺ T-cell responses compared to those suffering from severe infection⁹¹, suggesting a potential protective role of CD8⁺ T-cell immunity against SARS-CoV-2. CD8⁺ T-cells are known to contribute to effective clearing of the virus during acute viral infections. We found that CD8⁺ T-cells were the main drivers of SARS-CoV-2-directed cytotoxicity to SARS-CoV-2-loaded target cells. Although we started with a small proportion of SARS-CoV-2-reactive CD8⁺ T-cells on day 0, we obtained a more balanced CD8⁺ to CD4⁺ T-cell ratio on day 21. Contrary to our observation, Keller *et al.* ⁸³ who used peptide libraries consisting only of structural proteins of SARS-CoV-2 to isolate SARS-CoV-2-specific T-cells, did not obtain strong SARS-CoV-2-specific CD8⁺ T-cell responses even after expansion. A combination of structural and accessory proteins of SARS-CoV-2 might be superior in driving not only SARS-CoV-2-directed CD4⁺ but also CD8⁺ T-cell expansion.⁹² Thus, combinations of such antigens, or peptides from such antigens, are preferably used in the context of the present invention. Furthermore, the cytokine cocktail in the culture medium may affect the expansion of either CD4⁺ or CD8⁺ T-cells.¹⁴ Therefore, the use of different cytokine cocktails may explain the observed variations in the expansion of SARS-CoV-2-specific CD8⁺ T-cells.

Upon SARS-CoV-2-specific *ex vivo* stimulation, multiple studies reported high proportions of T_{EMRA} in the SARS-CoV-2-specific CD8⁺ T-cell compartment of patients recovered from SARS-CoV-2 infection^{93,94}.⁴ At the start of the expansion of SARS-CoV-2-specific IFN-γ-secreting CD8⁺ T-cells there was a substantial frequency of T_{EMRA} cells,³⁷ this highly differentiated memory phenotype might therefore be a characteristic feature of SARS-CoV-2 infection that would hint at short-term CD8⁺ T-cell immunity. TCPs with effector memory and central memory phenotypes have previously been shown to have an increased persistence after adoptive transfer and to induce long-term immunity⁶⁸, while a terminally differentiated phenotype may impair the success of ACT⁶⁹. Analysis of the T-cell phenotype of the unmodified control and *FKBP12*^{KO} TCPs revealed a predominant T_{EM} and T_{CM} phenotype among CD4⁺ T-cells before and after SARS-CoV-2-directed T-cell expansion, whereas CD8⁺ T-cells displayed mainly a T_{EMRA} phenotype followed by T_{EM} as the second most represented memory T-cell subset. Such cells should be sufficient to clear an acute SARS-CoV-2 infection. Moreover, T_{EMRA} can be divided into several subsets that have been shown to differ in their ability to differentiate, proliferate and produce effector cytokines⁷⁰⁻⁷² and might even comprise cells evolved from a naive differentiation stage⁷¹. It might also be conceivable that phenotypically late-differentiated effector SARS-CoV-2-specific CD8⁺ T-cells dedifferentiate into long-lived memory cells.⁷² Low-dose rapamycin supplementation during cell expansion may be a potential strategy to arrest differentiation of SARS-CoV-2 reactive T-cells or to generate early-differentiated T-cells.^{66,73}

Our functional analysis of the SARS-CoV-2-specific TCPs has shown that *FKBP12*^{KO} TCPs possess superior cytokine production upon SARS-CoV-2-specific re-stimulation in the presence of Tac and triple IS compared to unmodified SARS-CoV-2-specific TCPs. The sensitivity of *FKBP12*^{KO} SARS-CoV-2-specific TCPs towards CsA shows specificity of the approach and represents an important safety switch that could limit undesired toxicity associated with Tac-resistant FKBP12^{KO} SARS-CoV-2-specific TCPs *in vivo*⁴³. In line with previous observations, Tac did not influence the T-cell-mediated cytotoxic killing capacity of T-cells.^{45,74} The expression of the activation marker CD137 on the surface of T-cells, was only partially affected by CNIs. Virus-specific T-cell polyfunctionality is a correlate of T-cell efficacy and immune protection.^{75,76} Since *FKBP12*^{KO} SARS-CoV-2-specific TCPs contain multiple effector cytokine-producers (even in the presence of Tac), our approach is well suited for adoptively inducing protective immunity in patients with COVID-19.

CITE-seq identified distinct cell clusters based on specific protein expression and their transcriptomes. Cluster distributions were comparable among CD4⁺ and CD8⁺ T-cells between non-activated unmodified control and *FKBP12*^{KO} SARS-CoV-2-specific TCPs even in the presence of Tac or CsA. Upon SARS-CoV-2-specific activation, a distinct cluster distribution was observed. Some clusters increased in the absence of CNI for unmodified and *FKBP12*^{KO} TCPs as well as in presence of Tac for *FKBP12*^{KO} TCPs, which demonstrates that neither *FKBP12* editing nor Tac treatment impacted the transcriptome or expression of specific proteins of *FKBP12*^{KO} TCPs. Gene expression patterns confirmed downregulation of *FKBP12* in *FKBP12*^{KO} TCPs. The top 25 differentially expressed genes among SARS-CoV-2-specific activated CD4⁺ and CD8⁺ T-cells of unmodified control and *FKBP12*^{KO} TCPs in absence of CNI as well as under Tac treatment in the *FKBP12*^{KO} TCPs comprised genes mainly associated with metabolism, zinc-finger proteins as well as proteins of the transcriptional machinery. Interestingly, *IL-2* was overexpressed in SARS-CoV-2 stimulated *FKBP12*^{KO} CD8⁺ T-cells exposed to Tac compared to non-exposed *FKBP12*^{KO} CD8⁺ T-cells and unmodified controls. IL-2-producing CD8+ antiviral T-cells are associated with high proliferative potential and are promising candidates to induce sustained immunity after adoptive transfer.⁷⁸ Moreover, upregulation of *CXC3CR1* and *IL7R* mRNA in *FKBP12*^{KO} CD4⁺ T-cells compared to untouched controls in presence of Tac points at T-cells with high effector function and migratory capacity.⁷⁹⁻⁸¹ In CD8⁺ *FKBP12*^{KO} T-cells in presence of Tac, we found overexpression of *ID2* and *ID3* compared to untouched controls in presence of Tac, which are reported to promote survival and differentiation of mature effector CD8⁺ T-cells.⁸² Furthermore, upregulation of *HAVCR2* mRNA in CD8⁺ *FKBP12*^{KO} T-cells compared to untouched controls in presence of Tac could have beneficial effects on TCR-dependent activation and therefore, could enhance effector functions of our TCPs.⁸³ A slight increase in *CD247* mRNA levels in CD8⁺ *FKBP12*^{KO} T-cells compared to untouched controls in presence of Tac could result in reduced susceptibility to apoptosis.⁸⁴ The TCR diversity was largely comparable between unmodified control and *FKBP12*^{KO} TCPs. The distribution of the top 5 most represented TCR clones within CD4⁺ and CD8⁺ T-cells of unmodified control and *FKBP12*^{KO} TCPs revealed a shared TCR repertoire with no signs of abnormal proliferation of individual clones, which would be the case if *FKBP12*^{KO} or potential off-target editing transformed certain clones. Therefore, we conclude that *FKBP12* editing neither skewed the TCR repertoire of TCPs nor induced excessive clonal expansion.

Our GMP-compliant protocol to generate FKBP12^{KO} SARS-CoV-2 specific TCPs qualifies for transfer into clinical application.⁴⁵ We are preparing for clinical translation of Tac-resistant SARS-CoV-2-specific TCPs for first in human use in solid organ transplant recipients or autoimmune patients on Tac therapy. With this strategy, we expect successful treatment of acute CoVID-19, and we hope to be able to provide long-term immunity towards SARS-CoV-2 infection, as it is reported that ACT can not only prevent or treat viral infections in patients after transplantation with a very low incidence of adverse effects³⁹⁻⁴¹, but may even be able to induce protective humoral immunity⁴⁴. The transplant patients may specifically benefit from ACT as their responses to SARS-CoV-2 vaccination are reported to be poor^{85,86} and therefore could be improved if patients have previously undergone ACT. Additionally, we herein present the novel therapeutic concept of combination therapy of Tac with Tac-resistant SARS-CoV-2-specific ACT for prevention of immunopathology whilst allowing efficient viral clearing.

Taken together, we have demonstrated the feasibility of manufacturing clinical-grade Tac-resistant SARS-CoV-2-specific TCPs that are superior in function compared to unmodified control SARS-CoV-2-specific TCPs in the presence of Tac and triple IS. The retained sensitivity to CsA represents an important safety switch to inhibit potential adverse effects elicited by *FKBP12-edited* TCPs *in vivo.* Tac-resistant SARS-CoV-2-specific TCPs are attractive to boost antiviral immunity and vaccine responses in immunosuppressed transplant recipients and autoimmune patients on Tac therapy, in which we plan proof-of-concept in a first-in-human clinical trial. Importantly, Tac therapy to prevent bystander T-cell activation and immunopathology plus its antiviral activity combined with adoptive transfer of Tac-resistant SARS-CoV-2 TCPs may be an attractive novel treatment concept for severe COVID-19.

### Materials and Methods

### Blood sampling and PBMC isolation

The study was approved by the Charité - Universitätsmedizin Berlin Ethics Committee and peripheral blood was obtained from convalescent individuals with a history of asymptomatic or mild COVID-19 or healthy donors, who had given their written informed consent. PBMCs were isolated using Biocoll (Biochrom) gradient centrifugation.

### Determining SARS-CoV-2-specific T-cell responses

2*10⁶ PBMCs were stimulated with peptide pools of individual proteins of SARS-CoV-2 (JPT Peptide Technologies, *i.e.* NCAP, Spike S1, Spike S2, VEMP, VME1, AP3a, NS6, NS7a, NS7b, NS8, ORF9b, ORF10, Y14) at 1 µg/mL each in the presence of 1 µg/mL purified anti-CD28 antibody (clone CD28.2, Biolegend). Unstimulated controls were supplemented with equal concentrations of DMSO, 4 mg/mL Staphylococcus enterotoxin B (SEB) (Sigma-Aldrich) and CMV peptide pool (pp65 and IE-1; 0.5 µg/mL each - JPT Peptide Technologies) served as positive controls. Stimulated PBMCs were incubated in a humidified incubator at 37 °C, 5 % CO₂ for 16 h. Intracellular cytokine production was captured by addition of 2 µg/ml Brefeldin A (Sigma-Aldrich) after 2 h of stimulation and cells were stained using antibodies (all from BioLegend) and the FoxP3/Transcription Factor Staining Buffer Set (eBioscience). Staining was performed using fluorophore-conjugated human anti-CD3 (OKT3), -CD4 (SK3), -CD8 (RPA-T8), -IFN-γ (4S.B3), - TNF-α (MAb11), -IL-2 (MQ1-17H12), -CD137 (4B4-1), -CD154 (24-31) -CCR7 (G043H7) and CD45RA (HI100) antibodies. LIVE/DEAD Fixable Blue Dead Cell Stain (L/D; Invitrogen) was used to identify living cells. Samples were analyzed using a CytoFLEX flow cytometer (Beckman Coulter) and FlowJo-10 software (Tree Star).

### Serology

Serum IgG and IgA levels of antibodies targeting the S1-domain of the spike glycoprotein were determined by using anti-SARS-CoV-2 spike 1 IgG and IgA ELISA and carried out according to the manufacturers protocol (EUROIMMUN).

### Isolation and culture of SARS-CoV-2-specific T-cells

Virus-specific T-cells were isolated from PBMCs derived from 100 mL peripheral blood from convalescent donors following a 6 h stimulation with overlapping SARS-CoV-2-specific peptide pools (JPT Peptide Technologies; 1 µg/mL each) using an IFN-γ Secretion Assay-Cell Enrichment and Detection Kit according to the manufacturer's instructions (Miltenyi Biotec). Isolated virus specific T-Cells were cultured in complete media (VLE RPMI 1640 (PAN-Biotech) supplemented with penicillin (100 IU/mL), streptomycin (Biochrom), 10 % fetal calf serum [FCS, PAA]), 10 ng/mL recombinant human (rh)IL-7 and rhIL-15 (CellGenix) in 24-well plates, in humidified incubators at 37 °C and 5 % CO₂ as described previously.¹⁴ Cells were split 1:1 upon reaching 100 % confluency.

### Knockout procedure

2 to 10 million antiviral T-cells (half of the culture derived from 100 mL peripheral blood) were electroporated with RNPs on day 7 after isolation using Amaxa P3 primary cell 4D-Nucleofector X Kit L and the Amaxa-Nucleofector-4D (Lonza, program CO-115) to transfer ribonucleoprotein complexes of 30 µg of recombinant Alt-R^{®} S.p. HiFi Cas9 Nuclease V3 (Integrated DNA Technologies)⁹⁵ precomplexed with 15 µg synthetically modified sgRNA of SEQ ID NO: 1 (Synthego Corporation) with 2O'-methyl-3'phosphothioate modifications between the first and last 3 nucleotides (Synthego Corporation). The same number of unmodified antiviral T-cells were expanded as controls.

### Phenotypic and functional assays assessed by flow cytometry

For assessment of SARS-CoV-2-specific cytokine production/activation, lymphoblastoid cell lines (LCLs) were generated as described previously¹³ and used as antigen presenting cells at a 1:10 ratio for a 16 h SARS-CoV-2-specific stimulation with 0.65 µg/ml of pooled SARS-CoV-2-specific peptides in the presence or absence of immunosuppressants at clinical doses (6 ng/mL Tacrolimus [Prograf, Astellas]; 120 ng/mL CsA [Sandimmun, Novartis]; triple IS = 6 ng/ml Tacrolimus + 0.57 µg/mL Prednisolone [Urbason solubile, Sanofi] + 2.7 µg/mL mycophenolic acid [MPA, active substance of mycophenolate mofetil, Sigma-Aldrich])³¹. Additionally, T-cells were restimulated using peptide pools of individual SARS-CoV-2 proteins (NCAP, Spike S1, Spike S2, VEMP, VME1, AP3a, NS6, NS7a, NS7b, NS8, ORF9b, ORF10, Y14) at 0.5 µg/mL. To assess potential cross-reactivity to other common human coronaviruses TCPs were re-stimulated with a pool of peptides spanning the sequences of spike proteins of common human endemic coronaviruses (hCoV-229E, hCoV-NL63, hCoV-OC43, HKU1) (JPT Peptide Technologies; 0.5 µg/mL). Re-stimulation with CEFX Ultra Superstim pool (JPT Peptide Technologies; 0.5 µg/mL) served as control to exclude nonspecific T-cell expansion. Unstimulated controls included LCLs without SARS-CoV-2-specific peptides. Intracellular cytokine production was captured by addition of 2 µg/mL of Brefeldin A (Sigma-Aldrich) after 2 h of stimulation and cells were stained using antibodies (all from BioLegend, unless stated otherwise) and the FoxP3/Transcription Factor Staining Buffer Set (eBioscience). Staining was performed using fluorophore-conjugated human anti-CD3 (OKT3), -CD4 (SK3), -CD8 (RPA-T8), -IFN-γ (4S.B3), -TNF-α (MAb11), -CD137 (4B4-1), - CCR7 (G043H7) and CD45RA (HI100) antibodies. LIVE/DEAD Fixable Blue Dead Cell Stain (L/D; Invitrogen) was used to identify living cells.

A "VITAL" assay was performed to assess killing capacity of TCPs^{14,32,37}. Briefly, cells from TCPs were incubated at distinct ratios with autologous LCLs loaded with SARS-CoV-2-peptide pool or with peptide pools of individual SARS-CoV-2 proteins and stained with 10 µM Carboxyfluorescein Diacetate Succinimidyl Ester (CFSE-DA; Sigma-Aldrich) for 4 min whereas unloaded allogenic LCLs serving as non-target T-cells for control purposes were stained with 5 µM CellTrace^{™} Far Red (Invitrogen) for 10 min. T-cell-free LCL mixtures served as internal controls to calculate the SARS-CoV-2-specific killing capacity. After 14 h of incubation, co-cultures were stained with L/D.

All flow cytometry samples were analyzed using either a CytoFLEX or Navios flow cytometer (both from Beckman Coulter) and FlowJo-10 software (Tree Star).

### Knockout efficiency analysis

Analysis of on-target gene editing was performed on isolated DNA (Zymo Research) from day 21 cell samples. The FKBP12 locus was amplified using KAPA HiFi HotStart ReadyMix (Roche) and the following primer pairs: TCTGACGGGTCAGATAACACCTAG (F, SEQ ID NO: 8) and TCTTCCGGAGGCCTGGGTTT (R, SEQ ID NO: 9) with the following touchdown-PCR program in an automated thermocycler: 1. 95 °C, 3 minutes, 2. 98 °C, 30 seconds 3. 72-64 °C, 15 seconds (-0.5 °C for each cycle starting at the highest until the lowest temperature was reached; 20 cycles, 64 °C); 4. 72 °C, 15 seconds; 5. repeat from step 2 with decreasing annealing temperature (as specified); 6. 72 °C, 1 minute; 7. 4 °C. PCR products were purified using DNA purification & enrichment kit (Zymo Research) prior to Sanger sequencing with primer F by LGC Genomics GmbH. Editing frequencies were calculated using the Inference of CRISPR Edits (ICE) algorithm (Synthego Corporation)⁶⁸.

### CITE-seq and single cell TCR sequencing

*In vitro* expanded SARS-CoV-2-specific unmodified and *FKBP12*^{KO} T-cells were labelled with TotalSeq^{™}C anti-human hashtag antibodies (Biolegend) allowing the pooling of samples followed by labelling with TotalSeq^{™}C anti-human antibodies (Biolegend) targeting a panel of extracellular proteins. Single cell suspensions were loaded onto Next GEM Chip G (10X Genomics), which were placed into a 10X Genomics controller for initiation of the 10X workflow. Tagged antibody, transcriptome and TCR libraries were prepared using the Chromium Single Cell 5' Library & Gel Bead Kit as well as the Single Cell 5' Feature Barcode Library Kit (10x Genomics). TCR targeting was performed using the Chromium Single Cell V(D)J Enrichment Kit for Human T-cells (10X Genomics). Gene expression and TCR libraries were prepared using the Single Index Kit T Set A, whereas the Single Index Kit N Set A (10X Genomics) was used for CITE-seq libraries.

Qubit HS DNA assay kit (Life Technologies) was used for library quantification and fragment sizes were obtained using the 2100 Bioanalyzer using the High Sensitivity DNA Kit (Agilent). Sequencing was performed on a NextSeq500 device (Illumina) using High Output v2 Kits (150 cycles) with the recommended sequencing conditions for 5' GEX libraries (read1: 26 nt, read2: 98 nt, index1: 8 nt, index2: n.a.) and Mid Output v2 Kits (300 cycles) for TCR/BCR libraries (read1: 150 nt, read2: 150 nt, index1: 8 nt, index2: n.a., 20 % PhiX spike-in).

### CITE-seq and single cell TCR sequencing analysis

Raw sequence reads were processed using cellranger-5.0.0, including the default detection of intact cells. Mkfastq and count were used in default parameter settings for demultiplexing and quantifying the gene expression. Refdata-cellranger-hg19-1.2.0 was used as reference. Raw UMI-counts were further processed and analyzed using R 4.0.2 according to the osca workflow by Lun et al.⁹⁶ including normalization, filtering of low-quality cells, clustering and UMAP dimensionality reduction. Differentially abundant genes and clusters were identified using edgeR's quasi-likelihood methods and test for interaction between *FKBP12*^{KO} and Tacrolimus-treatment in LCL-stimulated cells. TCR repertoire analysis was conducted on the filtered_contig_annotations. csv outputs from the 10x Genomics Cell Ranger pipeline using the scRepertoire package⁹⁷. Clonotypes were called using the combination of CDR3 nucleotide sequences and genes.

### Statistics

P-values were determined by tests for normal distribution (Shapiro-Wilk and Kolmogorov-Smirnov tests), followed by one-way ANOVA (normally distributed data sets) or Friedman test (not normally distributed data sets) and paired t-tests (normally distributed data sets) or Wilcoxon matched pairs signed rank tests (not normally distributed data sets) or Man-Whitney-U test (not normally distributed data sets) as posttests.

### Example 5: FKBP12^{KO} NK cells

NK cells represent an attractive cell type for adoptive cell therapies especially in the context of cancer therapeutics. Their lytic granule exocytosis and pro-inflammatory cytokine production are calcineurin dependent and therefore influenced by calcineurin inhibitors such as Tacrolimus^{98,99}.

We performed a proof-of-principle experiment to confirm feasibility of FKBP12^{KO} in the NK cell line NK92. Four days prior to the experiment, FKBP12^{KO} NK92 cells were electroporated with Cas9 + FKBP12 sgRNA#1 (Table 1). NK cell cytotoxicity was assessed by co-culture with the MHC class I deficient erythroleukemia cell line K562. To this end, NK92 and CFSE-labeled K562 cells were mixed at a 1:1 ratio. After a total of 8 hours, the reaction was stopped by washing with cold 1X PBS. The cells were stained with Life Dead UV before fixation and permeabilization (IC Fix/Perm Kit, Thermo Fisher). Fixation was performed for 30 minutes at RT. The stained cells were analyzed with a Cytoflex LX. Analysis was performed with FlowJo v10. Interestingly, FKBP12^{KO} lead to higher killing efficacy in this experiment (Fig. 19). Tacrolimus-exposed FKBP12^{KO} NK92 cells showed similar killing efficiencies as non-exposed FKBP12X^{KO} NK92 cells, whereas exposition to Tacrolimus decreased killing efficiency in non-edited NK92 cells (Fig. 19).

Editing efficacy in NK92 was estimated using a quantitative real time (qRT) PCR-based assay (Fig. 20). To this end, 547bp fragment covering the targeted FKBP12 locus was amplified from genomic DNA by PCR (Forward primer: 5'-CGAGGTACTAGGCAGAGCCGTGGAAC-3' (SEQ ID NO: 28); Reverse primer: 5'-CTGTGCAGCGGGCATAAGGGC-3' (SEQ ID NO: 29)). PCR product was purified using DNA Clean&Concentrator-5 (Zymo Research). Subsequently, the purified PCR product was used as template during TacMan qRT-PCR Assay as indicated in Fig. 20 A with primers to amplify a 184bp DNA fragment (qPCR-Forward primer: 5'-atgggagtgcaggtggaaaccatc-3' (SEQ ID NO: 30), qPCR-Reverse primer: 5'-cgctgggcccccgactca-3' (SEQ ID NO: 31)). The assay was performed on a QuantStudio^{™} 6 Flex Real-Time PCR System (Thermo Fisher) and included two TacMan probes with different reporter dyes: 1) a FAM-labelled KO probe (sequence: 5'-ttccccaagcgcggccag-3'(SEQ ID NO: 32)), which is designed to overlap the FKBP12 sgRNA#1 cleavage site, and 2) a SUN-fluorophore labelled control probe (sequence: 5'-agccgccgcgcgccac-tact-3' (SEQ ID NO: 33)) located upstream to the cleavage site which serves as an internal control for efficient amplification. Analysis of wildtype (WT) NK92 showed efficient signaling of both control and KO probe (Fig. 20. B). In a FKBP12-edited NK92 cell line, no signal was detected from the KO probe indicating a high degree of editing (>90% KO) based on previous experiment from validation experiments (Fig. 20. C). In validation experiments, FKBP12-KO Treg gDNA was used as reference and then mixed with control unedited gDNA. Assays were performed as described before.

### References

1. Chen, JH, Mao, YY, He, Q, Wu, JY, and Lv, R (2005). The impact of pretransplant cytomegalovirus infection on acute renal allograft rejection. Transplant Proc 37: 4203-4207.
2. Tong, CY, Bakran, A, Peiris, JS, Muir, P, and Herrington, CS (2002). The association of viral infection and chronic allograft nephropathy with graft dysfunction after renal transplantation. Transplantation 74: 576-578.
3. Grossi, PA, Costa, AN, Fehily, D, Blumberg, EA, Kuehnert, MJ, Fishman, JA, et al. (2012). Infections and organ transplantation: new challenges for prevention and treatment--a colloquium. Transplantation 93: S4-S39.
4. Harvala, H, Stewart, C, Muller, K, Burns, S, Marson, L, MacGilchrist, A, et al. (2013). High risk of cytomegalovirus infection following solid organ transplantation despite prophylactic therapy. J Med Virol 85: 893-898.
5. Kumar, D, Chernenko, S, Moussa, G, Cobos, I, Manuel, O, Preiksaitis, J, et al. (2009). Cell-mediated immunity to predict cytomegalovirus disease in high-risk solid organ transplant recipients. Am J Transplant 9: 1214-1222.
6. Schachtner, T, Stein, M, and Reinke, P (2017). CMV-Specific T Cell Monitoring Offers Superior Risk Stratification of CMV-Seronegative Kidney Transplant Recipients of a CMV-Seropositive Donor. Transplantation 101: e315-e325.
7. Hill, GR, Tey, SK, Beagley, L, Crough, T, Morton, JA, Clouston, AD, et al. (2010). Successful immunotherapy of HCMV disease using virus-specific T cells expanded from an allogeneic stem cell transplant recipient. Am J Transplant 10: 173-179.
8. Clancy, LE, Blyth, E, Simms, RM, Micklethwaite, KP, Ma, CK, Burgess, JS, et al. (2013). Cytomegalovirus-specific cytotoxic T lymphocytes can be efficiently expanded from granulocyte colony-stimulating factor-mobilized hemopoietic progenitor cell products ex vivo and safely transferred to stem cell transplantation recipients to facilitate immune reconstitution. Biol Blood Marrow Transplant 19: 725-734.
9. Stemberger, C, Graef, P, Odendahl, M, Albrecht, J, Dossinger, G, Anderl, F, et al. (2014). Lowest numbers of primary CD8(+) T cells can reconstitute protective immunity upon adoptive immunotherapy. Blood 124: 628-637.
10. Stuehler, C, Stussi, G, Halter, J, Nowakowska, J, Schibli, A, Battegay, M, et al. (2015). Combination therapy for multidrug-resistant cytomegalovirus disease. Transpl Infect Dis 17: 751-755.
11. Neuenhahn, M, Albrecht, J, Odendahl, M, Schlott, F, Dossinger, G, Schiemann, M, et al. (2017). Transfer of minimally manipulated CMV-specific T cells from stem cell or thirdparty donors to treat CMV infection after allo-HSCT. Leukemia.
12. Riddell, SR, Watanabe, KS, Goodrich, JM, Li, CR, Agha, ME, and Greenberg, PD (1992). Restoration of viral immunity in immunodeficient humans by the adoptive transfer of T cell clones. Science 257: 238-241.
13. Brestrich, G, Zwinger, S, Roemhild, A, Noutsias, M, Rohde, M, Keeren, K, et al. (2009). Generation of HCMV-specific T-cell lines from seropositive solid-organ-transplant recipients for adoptive T-cell therapy. J Immunother 32: 932-940.
14. Amini, L, Vollmer, T, Wendering, DJ, Jurisch, A, Landwehr-Kenzel, S, Otto, NM, et al. (2019). Comprehensive Characterization of a Next-Generation Antiviral T-Cell Product and Feasibility for Application in Immunosuppressed Transplant Patients. Frontiers in Immunology 10**.**
15. Macesic, N, Langsford, D, Nicholls, K, Hughes, P, Gottlieb, DJ, Clancy, L, et al. (2015). Adoptive T cell immunotherapy for treatment of ganciclovir-resistant cytomegalovirus disease in a renal transplant recipient. Am J Transplant 15: 827-832.
16. Holmes-Liew, CL, Holmes, M, Beagley, L, Hopkins, P, Chambers, D, Smith, C, et al. (2015). Adoptive T-cell immunotherapy for ganciclovir-resistant CMV disease after lung transplantation. Clin Transl Immunology 4: e35.
17. Smith, C, Beagley, L, Rehan, S, Neller, MA, Crooks, P, Solomon, M, et al. (2018). Autologous adoptive T-cell therapy for recurrent or drug-resistant cytomegalovirus complications in solid organ transplant patients: A single-arm open-label phase I clinical trial. Clin Infect Dis.
18. Einsele, H, Roosnek, E, Rufer, N, Sinzger, C, Riegler, S, Löffler, J, et al. (2002). Infusion of cytomegalovirus (CMV)-specific T cells for the treatment of CMV infection not responding to antiviral chemotherapy. Blood 99: 3916-3922.
19. Brestrich, G, Zwinger, S, Fischer, A, Schmuck, M, Rohmhild, A, Hammer, MH, et al. (2009). Adoptive T-cell therapy of a lung transplanted patient with severe CMV disease and resistance to antiviral therapy. Am J Transplant 9: 1679-1684.
20. Savoldo, B, Goss, JA, Hammer, MM, Zhang, L, Lopez, T, Gee, AP, et al. (2006). Treatment of solid organ transplant recipients with autologous Epstein Barr virus-specific cytotoxic T lymphocytes (CTLs). Blood 108: 2942-2949.
21. Tsuda, K, Yamanaka, K, Kitagawa, H, Akeda, T, Naka, M, Niwa, K, et al. (2012). Calcineurin inhibitors suppress cytokine production from memory T cells and differentiation of naive T cells into cytokine-producing mature T cells. PLoS One 7: e31465.
22. Brinkmann, V, and Kristofic, C (1995). Regulation by corticosteroids of Th1 and Th2 cytokine production in human CD4+ effector T cells generated from CD45RO- and CD45RO+ subsets. J Immunol 155: 3322-3328.
23. Herold, MJ, McPherson, KG, and Reichardt, HM (2006). Glucocorticoids in T cell apoptosis and function. Cellular and molecular life sciences : CMLS 63: 60-72.
24. He, X, Smeets, RL, Koenen, HJ, Vink, PM, Wagenaars, J, Boots, AM, et al. (2011). Mycophenolic acid-mediated suppression of human CD4+ T cells: more than mere guanine nucleotide deprivation. Am J Transplant 11: 439-449.
25. Allison, AC, and Eugui, EM (2000). Mycophenolate mofetil and its mechanisms of action. Immunopharmacology 47: 85-118.
26. Brewin, J, Mancao, C, Straathof, K, Karlsson, H, Samarasinghe, S, Amrolia, PJ, et al. (2009). Generation of EBV-specific cytotoxic T cells that are resistant to calcineurin inhibitors for the treatment of posttransplantation lymphoproliferative disease. Blood 114: 4792-4803.
27. Ricciardelli, I, Brewin, J, Lugthart, G, Albon, SJ, Pule, M, and Amrolia, PJ (2013). Rapid generation of EBV-specific cytotoxic T lymphocytes resistant to calcineurin inhibitors for adoptive immunotherapy. Am J Transplant 13: 3244-3252.
28. De Angelis, B, Dotti, G, Quintarelli, C, Huye, LE, Zhang, L, Zhang, M, et al. (2009). Generation of Epstein-Barr virus-specific cytotoxic T lymphocytes resistant to the immunosuppressive drug tacrolimus (FK506). Blood 114: 4784-4791.
29. Xu, X, Su, B, Barndt, RJ, Chen, H, Xin, H, Yan, G, et al. (2002). FKBP12 is the only FK506 binding protein mediating T-cell inhibition by the immunosuppressant FK506. Transplantation 73: 1835-1838.
30. Gundry, MC, Brunetti, L, Lin, A, Mayle, AE, Kitano, A, Wagner, D, et al. (2016). Highly Efficient Genome Editing of Murine and Human Hematopoietic Progenitor Cells by CRISPR/Cas9. Cell Rep 17: 1453-1461.
31. Amini, L, Wagner, DL, Rossler, U, Zarrinrad, G, Wagner, LF, Vollmer, T, et al. (2020). CRISPR-Cas9-Edited Tacrolimus-Resistant Antiviral T Cells for Advanced Adoptive Immunotherapy in Transplant Recipients. Mol Ther.
32. Hammoud, B, Schmueck, M, Fischer, AM, Fuehrer, H, Park, SJ, Akyuez, L, et al. (2013). HCMV-specific T-cell therapy: do not forget supply of help. J Immunother 36: 93-101.
33. Cradick, TJ, Qiu, P, Lee, CM, Fine, EJ, and Bao, G (2014). COSMID: A Web-based Tool for Identifying and Validating CRISPR/Cas Off-target Sites. Molecular Therapy - Nucleic Acids 3: e214.
34. Casper, J, Zweig, AS, Villarreal, C, Tyner, C, Speir, ML, Rosenbloom, KR, et al. (2018). The UCSC Genome Browser database: 2018 update. Nucleic Acids Research 46: D762-D769.
35. Hendel, A, Bak, RO, Clark, JT, Kennedy, AB, Ryan, DE, Roy, S, et al. (2015). Chemically modified guide RNAs enhance CRISPR-Cas genome editing in human primary cells. Nature Biotechnology 33: 985.
36. Kannanganat, S, Ibegbu, C, Chennareddi, L, Robinson, HL, and Amara, RR (2007). Multiple-cytokine-producing antiviral CD4 T cells are functionally superior to singlecytokine-producing cells. J Virol 81: 8468-8476.
37. Hermans, IF, Silk, JD, Yang, J, Palmowski, MJ, Gileadi, U, McCarthy, C, et al. (2004). The VITAL assay: a versatile fluorometric technique for assessing CTL- and NKT-mediated cytotoxicity against multiple targets in vitro and in vivo. J Immunol Methods 285: 25-40.
38. Sallusto, F, Geginat, J, and Lanzavecchia, A (2004). Central memory and effector memory T cell subsets: function, generation, and maintenance. Annu Rev Immunol 22: 745-763.
39. Arce, S, Nawar, HF, Muehlinghaus, G, Russell, MW, and Connell, TD (2007). In vitro induction of immunoglobulin A (IgA)- and IgM-secreting plasma blasts by cholera toxin depends on T-cell help and is mediated by CD154 up-regulation and inhibition of gamma interferon synthesis. Infect Immun 75: 1413-1423.
40. Frentsch, M, Stark, R, Matzmohr, N, Meier, S, Durlanik, S, Schulz, AR, et al. (2013). CD40L expression permits CD8+ T cells to execute immunologic helper functions. Blood 122: 405-412.
41. Ashokkumar, C, Green, M, Soltys, K, Michaels, M, Mazariegos, G, Reyes-Mugica, M, et al. (2020). CD154-expressing CMV-specific T cells associate with freedom from DNAemia and may be protective in seronegative recipients after liver or intestine transplantation. Pediatr Transplant 24: e13601.
42. Wagner, DL, Amini, L, Wendering, DJ, Burkhardt, LM, Akyuz, L, Reinke, P, et al. (2018). High prevalence of Streptococcus pyogenes Cas9-reactive T cells within the adult human population. Nat Med.
43. Charlesworth, CT, Deshpande, PS, Dever, DP, Camarena, J, Lemgart, VT, Cromer, MK, et al. (2019). Identification of preexisting adaptive immunity to Cas9 proteins in humans. Nat Med 25: 249-254.
44. Kaeuferle, T, Deisenberger, L, Jablonowski, L, Stief, TA, Blaeschke, F, Willier, S, et al. (2020). CRISPR-Cas9-Mediated Glucocorticoid Resistance in Virus-Specific T Cells for Adoptive T Cell Therapy Posttransplantation. Mol Ther.
45. Strauss, G, Osen, W, and Debatin, KM (2002). Induction of apoptosis and modulation of activation and effector function in T cells by immunosuppressive drugs. Clin Exp Immunol 128: 255-266.
46. Savoldo, B, Goss, J, Liu, Z, Huls, MH, Doster, S, Gee, AP, et al. (2001). Generation of autologous Epstein-Barr virus-specific cytotoxic T cells for adoptive immunotherapy in solid organ transplant recipients. Transplantation 72: 1078-1086.
47. Zhang, J, Scordi, I, Smyth, MJ, and Lichtenheld, MG (1999). Interleukin 2 receptor signaling regulates the perforin gene through signal transducer and activator of transcription (Stat)5 activation of two enhancers. J Exp Med 190: 1297-1308.
48. Alves, NL, Hooibrink, B, Arosa, FA, and van Lier, RA (2003). IL-15 induces antigenindependent expansion and differentiation of human naive CD8+ T cells in vitro. Blood 102: 2541-2546.
49. Kataoka, T (2000). Involvement of FK506-sensitive and insensitive granule exocytosis pathways in perforin-dependent target cell lysis mediated by a CD8+ CTL clone. Immunology Letters 72: 49-52.
50. Yang, J, Murphy, TL, Ouyang, W, and Murphy, KM (1999). Induction of interferon-y production in Th1 CD4+ T cells: evidence for two distinct pathways for promoter activation. European Journal of Immunology 29: 548-555.
51. Balasubramani, A, Shibata, Y, Crawford, GE, Baldwin, AS, Hatton, RD, and Weaver, CT (2010). Modular utilization of distal cis-regulatory elements controls Ifng gene expression in T cells activated by distinct stimuli. Immunity 33: 35-47.
52. Dutta, D, Barr, VA, Akpan, I, Mittelstadt, PR, Singha, LI, Samelson, LE, et al. (2017). Recruitment of calcineurin to the TCR positively regulates T cell activation. Nat Immunol 18: 196-204.
53. Goldfeld, AE, Tsai, E, Kincaid, R, Belshaw, PJ, Schrieber, SL, Strominger, JL, et al. (1994). Calcineurin mediates human tumor necrosis factor alpha gene induction in stimulated T and B cells. J Exp Med 180: 763-768.
54. O'Keefe, SJ, Tamura, J, Kincaid, RL, Tocci, MJ, and O'Neill, EA (1992). FK-506- and CsA-sensitive activation of the interleukin-2 promoter by calcineurin. Nature 357: 692-694.
55. Snyder, LD, Chan, C, Kwon, D, Yi, JS, Martissa, JA, Copeland, CA, et al. (2016). Polyfunctional T-Cell Signatures to Predict Protection from Cytomegalovirus after Lung Transplantation. Am J Respir Crit Care Med 193: 78-85.
56. (2004). Cytomegalovirus. Am J Transplant 4 Suppl 10: 51-58.
57. Braitch, M, Harikrishnan, S, Robins, RA, Nichols, C, Fahey, AJ, Showe, L, et al. (2009). Glucocorticoids increase CD4CD25 cell percentage and Foxp3 expression in patients with multiple sclerosis. Acta Neurol Scand 119: 239-245.
58. Premaud, A, Rousseau, A, Johnson, G, Canivet, C, Gandia, P, Muscari, F, et al. (2011). Inhibition of T-cell activation and proliferation by mycophenolic acid in patients awaiting liver transplantation: PK/PD relationships. Pharmacol Res 63: 432-438.
59. Maeda, N, Maruhashi, T, Sugiura, D, Shimizu, K, Okazaki, IM, and Okazaki, T (2019). Glucocorticoids potentiate the inhibitory capacity of programmed cell death 1 by upregulating its expression on T cells. J Biol Chem 294: 19896-19906.
60. Xia, M, Gasser, J, and Feige, U (1999). Dexamethasone enhances CTLA-4 expression during T cell activation. Cell Mol Life Sci 55: 1649-1656.
61. Gibson, HM, Hedgcock, CJ, Aufiero, BM, Wilson, AJ, Hafner, MS, Tsokos, GC, et al. (2007). Induction of the CTLA-4 gene in human lymphocytes is dependent on NFAT binding the proximal promoter. J Immunol 179: 3831-3840.
62. Riddell, SR, Elliott, M, Lewinsohn, DA, Gilbert, MJ, Wilson, L, Manley, SA, et al. (1996). T-cell mediated rejection of gene-modified HIV-specific cytotoxic T lymphocytes in HIV-infected patients. Nature Medicine 2: 216.
63. Fu, Y, Foden, JA, Khayter, C, Maeder, ML, Reyon, D, Joung, JK, et al. (2013). Highfrequency off-target mutagenesis induced by CRISPR-Cas nucleases in human cells. Nature Biotechnology 31: 822.
64. Di Benedetto, S, Derhovanessian, E, Steinhagen-Thiessen, E, Goldeck, D, Muller, L, and Pawelec, G (2015). Impact of age, sex and CMV-infection on peripheral T cell phenotypes: results from the Berlin BASE-II Study. Biogerontology 16: 631-643.
65. Schmueck-Henneresse, M, Omer, B, Shum, T, Tashiro, H, Mamonkin, M, Lapteva, N, et al. (2017). Comprehensive Approach for Identifying the T Cell Subset Origin of CD3 and CD28 Antibody-Activated Chimeric Antigen Receptor-Modified T Cells. J Immunol 199: 348-362.
66. Li, D, Qiu, Z, Shao, Y, Chen, Y, Guan, Y, Liu, M, et al. (2013). Heritable gene targeting in the mouse and rat using a CRISPR-Cas system. Nat Biotechnol 31: 681-683.
67. Moosmann, A, Khan, N, Cobbold, M, Zentz, C, Delecluse, HJ, Hollweck, G, et al. (2002). B cells immortalized by a mini-Epstein-Barr virus encoding a foreign antigen efficiently reactivate specific cytotoxic T cells. Blood 100: 1755-1764.
68. Hsiau, T, Conant, D, Maures, T, Waite, K, Yang, J, Kelso, R, et al. (2019). Inference of CRISPR Edits from Sanger Trace Data. BioRxiv.
69. V'Kovski, P, Kratzel, A, Steiner, S, Stalder, H, and Thiel, V (2021). Coronavirus biology and replication: implications for SARS-CoV-2. Nat Rev Microbiol 19: 155-170.
70. Siddiqi, HK, and Mehra, MR (2020). COVID-19 illness in native and immunosuppressed states: A clinical-therapeutic staging proposal. J Heart Lung Transplant 39: 405-407.
71. Xu, Z, Shi, L, Wang, Y, Zhang, J, Huang, L, Zhang, C, et al. (2020). Pathological findings of COVID-19 associated with acute respiratory distress syndrome. The Lancet Respiratory Medicine 8: 420-422.
72. Wang, F, Hou, H, Luo, Y, Tang, G, Wu, S, Huang, M, et al. (2020). The laboratory tests and host immunity of COVID-19 patients with different severity of illness. JCI Insight 5**.**
73. Diao, B, Wang, C, Tan, Y, Chen, X, Liu, Y, Ning, L, et al. (2020). Reduction and Functional Exhaustion of T Cells in Patients With Coronavirus Disease 2019 (COVID-19). Front Immunol 11: 827.
74. Liao, M, Liu, Y, Yuan, J, Wen, Y, Xu, G, Zhao, J, et al. (2020). Single-cell landscape of bronchoalveolar immune cells in patients with COVID-19. Nat Med 26: 842-844.
75. de Candia, P, Prattichizzo, F, Garavelli, S, and Matarese, G (2021). T Cells: Warriors of SARS-CoV-2 Infection. Trends Immunol 42: 18-30.
76. Group, RC, Horby, P, Lim, WS, Emberson, JR, Mafham, M, Bell, JL, et al. (2021). Dexamethasone in Hospitalized Patients with Covid-19. N EnglJ Med 384: 693-704.
77. Pearson, MM, Limaye, AP, and Biggins, SW (2021). Tacrolimus: Unlikely Harmful and Perhaps Helpful in Liver Transplant Recipients with COVID-19. Gastroenterology 160: 1012-1013.
78. Hayashi, K, Ito, Y, Yamane, R, Yoshizaki, M, Matsushita, K, Kajikawa, G, et al. (2021). The case of a liver-transplant recipient with severe acute respiratory syndrome coronavirus 2 infection who had a favorable outcome. Clin J Gastroenterol 14: 842-845.
79. García-Juárez, I, Campos-Murguia, A, Tovar-Méndez, VH, Gabutti, A, and Ruiz, I (2020). Unexpected better outcome in a liver transplant recipient with COVID-19: a beneficial effect of tacrolimus? Revista de Gastroenterología de México (English Edition) 85: 437-442.
80. Belli, LS, Fondevila, C, Cortesi, PA, Conti, S, Karam, V, Adam, R, et al. (2020). Protective role of tacrolimus, deleterious role of age and comorbidities in liver transplant recipients with Covid-19: results from the ELITA/ELTR multi-center European study. Gastroenterology*.*
81. AlGhamdi, M, Mushtaq, F, Awn, N, and Shalhoub, S (2015). MERS CoV infection in two renal transplant recipients: case report. Am J Transplant 15: 1101-1104.
82. Sanchez-Pernaute, O, Romero-Bueno, FI, and Selva-O'Callaghan, A (2020). Why Choose Cyclosporin A as First-line Therapy in COVID-19 Pneumonia. Reumatol Clin (Engl Ed).
83. Keller, MD, Harris, KM, Jensen-Wachspress, MA, Kankate, V, Lang, H, Lazarski, CA, et al. (2020). SARS-CoV-2 specific T-cells Are Rapidly Expanded for Therapeutic Use and Target Conserved Regions of Membrane Protein. Blood.
84. Leung, W, Soh, TG, Linn, YC, Low, JG, Loh, J, Chan, M, et al. (2020). Rapid production of clinical-grade SARS-CoV-2 specific T cells. Adv Cell Gene Ther: e101.
85. Haque, T, Wilkie, GM, Jones, MM, Higgins, CD, Urquhart, G, Wingate, P, et al. (2007). Allogeneic cytotoxic T-cell therapy for EBV-positive posttransplantation lymphoproliferative disease: results of a phase 2 multicenter clinical trial. Blood 110: 1123-1131.
86. Cruz, CR, Hanley, PJ, Liu, H, Torrano, V, Lin, YF, Arce, JA, et al. (2010). Adverse events following infusion of T cells for adoptive immunotherapy: a 10-year experience. Cytotherapy 12: 743-749.
87. Hegde, M, Joseph, SK, Pashankar, F, DeRenzo, C, Sanber, K, Navai, S, et al. (2020). Tumor response and endogenous immune reactivity after administration of HER2 CAR T cells in a child with metastatic rhabdomyosarcoma. Nat Commun 11: 3549.
88. Boyarsky, BJ, Werbel, WA, Avery, RK, Tobian, AAR, Massie, AB, Segev, DL, et al. (2021). Antibody Response to 2-Dose SARS-CoV-2 mRNA Vaccine Series in Solid Organ Transplant Recipients. JAMA 325: 2204-2206.
89. Ali, NM, Alnazari, N, Mehta, SA, Boyarsky, B, Avery, RK, Segev, DL, et al. (2021). Development of COVID-19 Infection in Transplant Recipients After SARS-CoV-2 Vaccination. Transplantation.
90. Gong, F, Dai, Y, Zheng, T, Cheng, L, Zhao, D, Wang, H, et al. (2020). Peripheral CD4+ T cell subsets and antibody response in COVID-19 convalescent individuals. J Clin Invest 130: 6588-6599.
91. Le Bert, N, Tan, AT, Kunasegaran, K, Tham, CYL, Hafezi, M, Chia, A, et al. (2020). SARS-CoV-2-specific T cell immunity in cases of COVID-19 and SARS, and uninfected controls. Nature 584: 457-462.
92. Grifoni, A, Weiskopf, D, Ramirez, SI, Mateus, J, Dan, JM, Moderbacher, CR, et al. (2020). Targets of T Cell Responses to SARS-CoV-2 Coronavirus in Humans with COVID-19 Disease and Unexposed Individuals. Cell 181: 1489-1501 e1415.
93. Weiskopf, D, Schmitz, KS, Raadsen, MP, Grifoni, A, Okba, NMA, Endeman, H, et al. (2020). Phenotype and kinetics of SARS-CoV-2-specific T cells in COVID-19 patients with acute respiratory distress syndrome. Sci Immunol 5**.**
94. Neidleman, J, Luo, X, Frouard, J, Xie, G, Gill, G, Stein, ES, et al. (2020). SARS-CoV-2-Specific T Cells Exhibit Phenotypic Features of Helper Function, Lack of Terminal Differentiation, and High Proliferation Potential. Cell Rep Med 1: 100081.
95. Vakulskas, CA, Dever, DP, Rettig, GR, Turk, R, Jacobi, AM, Collingwood, MA, et al. (2018). A high-fidelity Cas9 mutant delivered as a ribonucleoprotein complex enables efficient gene editing in human hematopoietic stem and progenitor cells. Nat Med 24: 1216-1224.
96. Lun, AT, McCarthy, DJ, and Marioni, JC (2016). A step-by-step workflow for low-level analysis of single-cell RNA-seq data with Bioconductor. F1000Res 5: 2122.
97. Borcherding, N, Bormann, NL, and Kraus, G (2020). scRepertoire: An R-based toolkit for single-cell immune receptor analysis. F1000Res 9: 47.
98. Pores-Fernando, AT, Gaur, S, Doyon, MY, and Zweifach, A (2009). Calcineurindependent lytic granule exocytosis in NK-92 natural killer cells. Cell Immunol 254: 105-109.
99. Morteau, O, Blundell, S, Chakera, A, Bennett, S, Christou, CM, Mason, PD, et al. (2010). Renal transplant immunosuppression impairs natural killer cell function in vitro and in vivo. PLoS One 5: e13294.
100. WO2021078645

## Claims

1. A guide RNA targeting a sequence of SEQ ID NO: 1 for CRISPR/Cas mediated gene editing.

2. The guide RNA of claim 1, wherein the guide RNA is a single guide RNA (sgRNA), preferably, a synthetic sgRNA.

3. The guide RNA of any of claims 1 or 2, wherein the guide RNA comprises a spacer sequence having at least 80% sequence identity to SEQ ID NO: 14.

4. The guide RNA of any of claims 1-3, wherein the guide RNA is a sgRNA having a nucleic acid sequence having at least 70% sequence identity to SEQ ID NO: 17.

5. A method for preparing a human immunophilin knockout T cell, NK cell or NKT cell, comprising
a) stimulating T cells, NK cells or NKT cells obtained from a subject,
b) isolating stimulated T cells, NK cells or NKT cells based on expression of a marker to obtain a composition of selected T cells, NK cells or NKT cells,
c) gene editing the cells of said composition to knock out the gene encoding the immunophilin by introducing a ribonucleoprotein complex comprising a CRISPR associated protein (Cas) and a guide RNA targeting the gene encoding the immunophilin into the cells of said composition, and
d) optionally, selecting immunophilin knockout cells by culturing the cells in the presence of an immunosuppressant agent capable of interacting with the immunophilin,
wherein the immunophilin is FKBP12 and the guide RNA is a guide RNA of any of claims 1-4, wherein, preferably, the cells are T cells.

6. The method of claim 5, wherein, additionally, the immunophilin is cyclophilin A and the guide RNA targets the sequence of SEQ ID NO: 4, 5, 6 and/or 7.

7. The method of any of claims 5 or 6, wherein the human immunophilin knockout cell is a T cell specific for a virus, another pathogen or a cancer antigen, wherein, in step a), the T cells are stimulated with a virus-derived antigen, another pathogen-derived antigen or a cancer antigen, and in step b), the marker is an activation marker, optionally, IFNγ-secretion.

8. The method of any of claims 5-7, wherein the human immunophilin knockout cell is a regulatory T cell, wherein preferably, in step b), the marker is a regulatory T cell marker selected from the group comprising CD25.

9. A human immunophilin knockout cell that is a T cell, NK cell or NKT cell, wherein the cell is obtainable by carrying out the method of any of claims 5-8.

10. The human immunophilin knockout cell of claim 9, wherein the cell further comprises a knockout of the glucocorticoid receptor gene.

11. A population of cells comprising cells of any of claim 9 or 10, wherein the population is a polyclonal population of cells,
wherein the population comprises cells with InDel mutations in the genes encoding the immunophilin.

12. A pharmaceutical composition comprising the cell of any of claims 9 or 10 or the cell population of claim 11, or
a kit comprising said pharmaceutical composition and an immunosuppressant agent capable of interacting with the immunophilin, wherein the kit optionally further comprises an immunosuppressant agent capable of inhibiting an immune response of the cells.

13. The pharmaceutical composition or kit of claim 12, wherein the T cell(s) is a T cell specific for a virus, another pathogen or a cancer antigen, or an NK cell, for use in treatment or prevention of an infection with the virus or the other pathogen or for use in treatment of the cancer,
wherein, preferably, the treated subject is immunosuppressed with an immunosuppressant agent capable of interacting with the immunophilin.

14. The pharmaceutical composition or kit of any of claims 12 or 13 for use in prevention and/or treatment of COVID-19, optionally, in immunosuppressed patients treated with an immunosuppressant agent capable of interacting with the immunophilin.

15. The pharmaceutical composition or kit of any of claims 12-14, wherein the cell is a regulatory T cell, for use in balancing an unwanted immune response in a patient having a condition selected from the group comprising autoimmunity, autoinflammation, allergy, a transplantation and an immunopathology caused by bystander activation.

## Patentansprüche

1. Eine guide RNA, die eine Sequenz mit SEQ ID NO: 1 als Ziel für CRISPR/Casvermittelte Geneditierung festlegt.

2. Die guide RNA nach Anspruch 1, wobei die guide RNA eine single guide RNA (sgRNA) ist, vorzugsweise eine synthetische sgRNA.

3. Die guide RNA nach einem der Ansprüche 1 oder 2, wobei die guide RNA eine Spacer-Sequenz umfasst, die eine Sequenzidentität von mindestens 80 % zu SEQ ID NO: 14 hat.

4. Die guide RNA nach einem der Ansprüche 1 bis 3, wobei die guide RNA eine sgRNA ist, deren Nukleinsäuresequenz eine Sequenzidentität von mindestens 70 % zu SEQ ID NO: 17 hat.

5. Ein Verfahren zur Herstellung einer menschlichen Immunophilin-Knockout-T-Zelle, NK-Zelle oder NKT-Zelle, umfassend
a) Stimulierung von T-Zellen, NK-Zellen oder NKT-Zellen, die von einem Subjekt gewonnen wurden,
b) Isolierung stimulierter T-Zellen, NK-Zellen oder NKT-Zellen basierend auf Expression eines Markers, um eine Zusammensetzung ausgewählter T-Zellen, NK-Zellen oder NKT-Zellen zu gewinnen,
c) Geneditierung der Zellen der genannten Zusammensetzung, um das für das Immunophilin kodierende Gen auszuschalten, durch Einbringen eines Ribonukleoprotein-Komplexes, der ein CRISPR-assoziiertes Protein (Cas) und eine auf das auf das Immunophilin kodierende Gen abzielende guide RNA umfasst, in die Zellen der genannten Zusammensetzung, und
d) optional, Selektion von Immunophilin-Knockout-Zellen durch Kultivierung der Zellen in Gegenwart eines Immunsuppressivums, das mit dem Immunophilin interagieren kann,
wobei das Immunophilin FKBP12 ist und die guide RNA eine guide RNA nach einem der Ansprüche 1 bis 4 ist, wobei die Zellen vorzugsweise T-Zellen sind.

6. Das Verfahren nach Anspruch 5, wobei das Immunophilin zusätzlich Cyclophilin A ist und die guide RNA die Sequenz von SEQ ID NO: 4, 5, 6 und/oder 7 als Ziel festlegt.

7. Das Verfahren nach einem der Ansprüche 5 oder 6, wobei die menschliche Immunophilin-Knockout-Zelle eine T-Zelle spezifisch gegen ein Virus, ein anderes Pathogen oder ein Krebsantigen ist, wobei in Schritt a) die T-Zellen mit einem von einem Virus stammenden Antigen, einem von einem anderen Pathogen stammenden Antigen oder einem Krebsantigen stimuliert werden und in Schritt b) der Marker ein Aktivierungsmarker ist, optional, IFN-γ-Sekretion.

8. Das Verfahren nach einem der Ansprüche 5 bis 7, wobei die menschliche Immunophilin-Knockout-Zelle eine regulatorische T-Zelle ist, wobei vorzugsweise in Schritt b) der Marker ein Marker für regulatorische T-Zellen ist, ausgewählt aus der Gruppe, die CD25 umfasst.

9. Eine menschliche Immunophilin-Knockout-Zelle, die eine T-Zelle, eine NK-Zelle oder eine NKT-Zelle ist, wobei die Zelle durch Durchführung des Verfahrens nach einem der Ansprüche 5 bis 8 erhältlich ist.

10. Die menschliche Immunophilin-Knockout-Zelle nach Anspruch 9, wobei die Zelle ferner ein Knockout des Glukokortikoidrezeptor-Gens umfasst.

11. Eine Zellpopulation, die Zellen nach einem der Ansprüche 9 oder 10 umfasst, wobei die Population eine polyklonale Zellpopulation ist,
wobei die Population Zellen mit InDel-Mutationen in den Genen umfasst, die für das Immunophilin kodieren

12. Eine pharmazeutische Zusammensetzung, die die Zelle nach einem der Ansprüche 9 oder 10 oder die Zellpopulation nach Anspruch 11 umfasst, oder
ein Kit, das die genannte pharmazeutische Zusammensetzung und ein immunsuppressives Agens umfasst, das mit dem Immunophilin interagieren kann, wobei das Kit optional ferner ein immunsuppressives Agens umfasst, das eine Immunantwort der Zellen hemmen kann.

13. Die pharmazeutische Zusammensetzung oder das Kit nach Anspruch 12, wobei die T-Zelle(n) (eine) für ein Virus, ein anderes Pathogen oder ein Krebsantigen spezifische T-Zelle ist/sind, oder eine NK-Zelle, zur Verwendung bei Behandlung oder Vorbeugung einer Infektion mit dem Virus oder dem anderen Pathogen oder zur Verwendung bei der Behandlung des Krebses,
wobei vorzugsweise das behandelte Subjekt mit einem immunsuppressiven Agens immunsupprimiert ist, das mit dem Immunophilin interagieren kann.

14. Die pharmazeutische Zusammensetzung oder das Kit nach einem der Ansprüche 12 oder 13 zur Verwendung bei Vorbeugung und/oder Behandlung von COVID-19, optional bei immunsupprimierten Patienten, die mit einem immunsuppressiven Agens behandelt werden, das mit dem Immunophilin interagieren kann.

15. Die pharmazeutische Zusammensetzung oder das Kit nach einem der Ansprüche 12 bis 14, wobei die Zelle eine regulatorische T-Zelle ist, zur Verwendung beim Ausgleich einer unerwünschten Immunantwort bei einem Patienten, der eine Erkrankung hat, die aus der Gruppe ausgewählt ist, die Autoimmunität, Autoinflammation, Allergie, eine Transplantation und eine durch Bystander-Aktivierung verursachte Immunpathologie umfasst.

## Revendications

1. ARN guide ciblant une séquence de SEQ ID NO : 1 pour une édition de gène médiée par CRISPR/Cas.

2. ARN guide selon la revendication 1, dans lequel l'ARN guide est un ARN guide unique (sgRNA), de préférence un sgRNA synthétique.

3. ARN guide selon l'une quelconque des revendications 1 à 2, dans lequel l'ARN guide comprend une séquence d'espaceur ayant au moins 80 % d'identité de séquence vis-à-vis de la SEQ ID NO : 14.

4. ARN guide selon l'une quelconque des revendications 1 à 3, dans lequel l'ARN guide est un sgRNA ayant une séquence d'acide nucléique ayant au moins 70 % d'identité de séquence vis-à-vis de la SEQ ID NO : 17.

5. Procédé de préparation d'un lymphocyte T, d'un lymphocyte NK ou d'un lymphocyte NKT invalidés pour l'immunophiline humaine, comprenant les étapes consistant à
a) stimuler des lymphocytes T, des lymphocytes NK ou des lymphocytes NKT obtenus auprès d'un sujet,
b) isoler des lymphocytes T, des lymphocytes NK ou des lymphocytes NKT stimulés sur la base de l'expression d'un marqueur pour obtenir une composition de lymphocytes T, de lymphocytes NK ou de lymphocytes NKT sélectionnés,
c) éditer les gènes des cellules de ladite composition pour invalider le gène codant pour l'immunophiline en introduisant un complexe de ribonucléoprotéine comprenant une protéine associée à CRISPR (Cas) et un ARN guide ciblant le gène codant pour l'immunophiline dans les cellules de ladite composition, et
d) éventuellement, sélectionner des cellules invalidées pour l'immunophiline en mettant en culture les cellules en présence d'un agent immunosuppresseur apte à interagir avec l'immunophiline,
dans lequel l'immunophiline est FKBP12 et l'ARN guide est un ARN guide selon l'une quelconque des revendications 1 à 4, dans lequel, de préférence, les cellules sont des lymphocytes T.

6. Procédé selon la revendication 5, dans lequel, de plus, l'immunophiline est la cyclophiline A et l'ARN guide cible la séquence de SEQ ID NO : 4, 5, 6 et/ou 7.

7. Procédé selon l'une quelconque des revendications 5 ou 6, dans lequel la cellule invalidée pour l'immunophiline humaine est un lymphocyte T spécifique d'un virus, d'un autre pathogène ou d'un antigène cancéreux, dans lequel, dans l'étape a), les lymphocytes T sont stimulés par un antigène dérivé d'un virus, un antigène dérivé d'un autre pathogène ou un antigène cancéreux, et dans l'étape b), le marqueur est un marqueur d'activation, éventuellement, la sécrétion d'IFNγ.

8. Procédé selon l'une quelconque des revendications 5 à 7, dans lequel la cellule invalidée pour l'immunophiline humaine est un lymphocyte T régulateur, dans lequel de préférence, dans l'étape b), le marqueur est un marqueur de lymphocyte T régulateur choisi dans le groupe comprenant CD25.

9. Cellule invalidée pour l'immunophiline humaine qui est un lymphocyte T, un lymphocyte NK ou un lymphocyte NKT, dans laquelle la cellule peut être obtenue en mettant en œuvre le procédé selon l'une quelconque des revendications 5 à 8.

10. Cellule invalidée pour l'immunophiline humaine selon la revendication 9, dans laquelle la cellule comprend en outre une invalidation du gène du récepteur des glucocorticoïdes.

11. Population de cellules comprenant des cellules selon l'une quelconque des revendications 9 ou 10, dans laquelle la population est une cellule population de cellules polyclonale,
dans laquelle la population comprend des cellules avec des mutations InDel dans les gènes codant pour l'immunophiline.

12. Composition pharmaceutique comprenant la cellule selon l'une quelconque des revendications 9 ou 10 ou la population de cellules selon la revendication 11, ou
kit comprenant ladite composition pharmaceutique et un agent immunosuppresseur apte à interagir avec l'immunophiline, dans lequel le kit comprend en outre éventuellement un agent immunosuppresseur apte à inhiber une réponse immunitaire des cellules.

13. Composition pharmaceutique ou kit selon la revendication 12, dans lequel le ou les lymphocytes T sont un lymphocyte T spécifique d'un virus, d'un autre pathogène ou d'un antigène cancéreux, ou un lymphocyte NK, pour une utilisation dans le traitement prophylactique ou thérapeutique d'une infection par le virus ou l'autre pathogène ou pour une utilisation dans le traitement du cancer,
dans lequel, de préférence, le sujet traité est immunosupprimé par un agent immunosuppresseur apte à interagir avec l'immunophiline.

14. Composition pharmaceutique ou kit selon l'une quelconque des revendications 12 ou 13 pour une utilisation dans le traitement prophylactique et/ou thérapeutique du COVID-19, éventuellement, chez des patients immunosupprimés traités par un agent immunosuppresseur apte à interagir avec l'immunophiline.

15. Composition pharmaceutique ou kit selon l'une quelconque des revendications 12 à 14, dans laquelle la cellule est un lymphocyte T régulateur, pour une utilisation dans l'équilibrage d'une réponse immunitaire non souhaitée chez un patient ayant une affection choisie dans le groupe comprenant l'auto-immunité, l'auto-inflammation, une allergie, une greffe et une immunopathologie activée par effet bystander.
